(19) Europäisches Patentamt
European Patent Office
Office européen des brevets

(11) EP 0 929 311 B1

(12) **EUROPEAN PATENT SPECIFICATION**

(45) Date of publication and mention of the grant of the patent:
**23.11.2005 Bulletin 2005/47**

(21) Application number: **97943323.2**

(22) Date of filing: **12.09.1997**

(51) Int Cl.[7]: **A61K 38/00**, C07H 21/00

(86) International application number:
**PCT/US1997/016369**

(87) International publication number:
**WO 1998/010778 (19.03.1998 Gazette 1998/11)**

(54) **INHIBITION OF APOPTOSIS USING INTERLEUKIN-1 beta-CONVERTING ENZYME (ICE)/CED-3 FAMILY INHIBITORS**

HEMMUNG DER APOPTOSE UNTER VERWENDUNG VON INHIBITOREN DER INTERLEUKIN-1 BETA CONVERTING ENZYM (ICE)/CED-3 FAMILIE

INHIBITION DE L'APOPTOSE AU MOYEN D'INHIBITEURS DE LA FAMILLE CED-3/ICE (ENZYME DE CONVERSION DE L'INTERLEUKINE-1 beta

(84) Designated Contracting States:
**AT BE CH DE DK ES FI FR GB GR IE IT LI LU MC NL PT SE**

(30) Priority: **12.09.1996 US 26011 P**
**20.09.1996 US 710621**
**16.12.1996 US 767175**

(43) Date of publication of application:
**21.07.1999 Bulletin 1999/29**

(73) Proprietor: **Idun Pharmaceuticals, Inc.**
**La Jolla, CA 92037 (US)**

(72) Inventors:
- **FRITZ, Lawrence, C.**
  **Rancho Santa Fe, CA 92067 (US)**
- **TOMASELLI, Kevin, J.**
  **San Diego, CA 92103 (US)**
- **KARANEWSKY, Donald S.**
  **Escondido, CA 92029 (US)**
- **LINTON, Steven D.**
  **San Diego, CA 92131 (US)**
- **BAI, Xu.**
  **Carlsbad, CA 92009 (US)**

(74) Representative:
**Gowshall, Jonathan Vallance et al**
**FORRESTER & BOEHMERT**
**Pettenkoferstrasse 20-22**
**80336 München (DE)**

(56) References cited:
**EP-A- 0 595 610** **WO-A-95/05192**
**US-A- 5 514 694**

- **SCIENCE, February 1994, Vol. 263, GAGLIARDINI V. et al., "Prevention of Vertebrate Neuronal Death by the crmA Gene", pages 826-828, XP002013931.**
- **CELL, November 1993, Vol. 75, MIURA M. et al., "Induction of Apoptosis in Fibroblasts by IL-1 Beta-Converting Enzyme, a Mammalian Homolog of the C. Elegans Cell Death Gene ced-3", pages 653-660, XP000676423.**
- **NATURE, July 1995, Vol. 376, NICHOLSON D.W. et al., "Identification and Inhibition of the ICE/CED-3 Protease Necessary for Mammalian Apoptosis", pages 37-43, XP000574812.**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### Field of the Invention

The present invention relates generally to programmed cell death and specifically to methods for expansion of hematopoietic cells, for prolonging viability of an organ for transplantation, and maintaining viability of cell lines used in bioproduction using interleukin-1β-converting enzyme (ICE)/CED-3 family inhibitors.

### Background of the Invention

The present invention relates generally to programmed cell death and specifically to methods for expansion of hematopoietic cells, for prolonging viability of an organ for transplantation, and maintaining viability of cell lines used in bioproduction using interleukin-1β-converting enzyme (ICE)/CED-3 family inhibitors.

Necrosis and apoptosis are two basic processes by which cells may die. In necrosis cell death usually is a result of cell injury. The cells generally swell and lyse, and the cell contents ultimately spill into the extracellular space. By contrast, apoptosis is a mode of cell death in which single cells are deleted in the midst of living tissues. Apoptosis accounts for most of the programmed cell death in tissue remodeling and for the cell loss that accompanies atrophy of adult tissues following withdrawal of endocrine and other growth stimuli. In addition, apoptosis is believed to be responsible for the physiologic death of cells in the course of normal tissue turnover (i.e., tissue homeostasis) (Kerr, J. F.,*et al,* 1972. *Br. J. Cancer* 26:239-257; Wyllie, A. H., *et al.* 1980. *Int. Rev. Cytol.* 68:251-306).

The effector mechanisms of apoptosis are not completely understood, but ultimately, certain nuclear changes occur that appear to be caused by the activation of endogenous nucleases that cleave chromatin between nucleosomes and reduce the content of intact DNA in apoptotic cells. A number of regulators of apoptosis have been identified. Some of these are already familiar as protooncogenes and oncosuppressor genes, including c-myc, bcl-2, p53, and ras. The protooncogene products and oncosuppressor proteins are believed to control cellular susceptibility to apoptosis (Isaacs, J. T. 1994. *Curr. Opin. Oncol.* 6:82-89). C-myc can determine whether cells continuously proliferate or enter apoptosis, depending on the availability of critical growth factors (Bisonnette, R. P., *et al.* 1994. In *Apoptosis II. The Molecular Basis of Apoptosis in Disease.* Cold Spring Harbor Laboratory Press). In cultured cells, proliferation is usually observed in the presence of c-myc and growth factors, whereas apoptosis is seen when c-myc is present but growth factors are absent. Certain other oncogenes (e.g., bcl-2) rescue cells from susceptibility to apoptosis. Specifically, members of the bcl-2 gene family can act to inhibit programmed cell death (e.g., bcl-2, bcl-xL, ced-9) or promote cell death (*e.g.,* bax, bak, bcl-xS). Additionally, members of the ICE/CED-3 family can promote cell death (*e.g.,* ICE, CPP32, Ich-1, CED3).

Interleukin 1 ("IL-1") is a major pro-inflammatory and immunoregulatory protein that stimulates fibroblast differentiation and proliferation, the production of prostaglandins, collagenase and phospholipase by synovial cells and chondrocytes, basophil and eosinophil degranulation and neutrophil activation. Oppenheim, J.H. *et al.,* Immunology Today, 7:45-56 (1986). As such, it is involved in the pathogenesis of chronic and acute inflammatory and autoimmune diseases. IL-1 is predominantly produced by peripheral blood monocytes as part of the inflammatory response. Mosely, B.S. *et al.,* Proc. Nat. Acad. Sci., 84:4572-4576 (1987); Lonnemann, *G. et al.,* Eur. J. Immunol., 19:1531-1536 (1989).

Mammalian IL-1β is synthesized as a precursor polypeptide of about 31.5 kDa (Limjuco, *et al., Proc. Natl. Acad. Sci. USA,* 83:3972, 1986). Precursor IL-1β is unable to bind to IL-1 receptors and is biologically inactive (Mosley, *et al., J. Biol. Chem.,* 262:2941, 1987). Biological activity appears dependent upon proteolytic processing which results in the conversion of the precursor 31.5 kDa form to the mature 17.5 kDa form.

Proteolytic maturation of human precursor IL-1β to mature, 17 kDa IL-1β results from cleavage between $Asp^{116}$ and $Ala^{117}$. An endoproteinase, termed Interleukin-1β Converting Enzyme (ICE), has been identified in human monocytes that is capable of cleaving the IL-1β precursor at $Asp^{116}$- $Ala^{117}$, as well as at the site $Asp^{27}$ -$Gly^{28}$, and generating mature IL-1β with the appropriate amino terminus at $Ala^{117}$. The Asp at position 116 has been found to be essential for cleavage, since substitution of Ala (Kostura, *et al., Proc. Natl. Acad. Sci.,* 86:5227, 1989) or other amino acids (Howard, *et al., J. Immunol.,* 147:2964, 1991) for Asp inhibits this cleavage event.

The substrate specificity of human ICE has been defined with the use of peptides that span the cleavage site of the enzyme. Two features of peptide substrates are essential for catalytic recognition by the enzyme. First, there is a strong preference for aspartic acid adjacent to the cleavage site, in that any substitution of this residue in the IL-1β precursor and peptide substrates leads to a substantial reduction in the rate of catalysis (Kostura, *et al., Proc. Natl. Acad. Sci.,* 86:5227, 1989; Sleath, *et al., J. Biol. Chem.,* 265:14526, 1990; Howard, *et al., J. Immunol.,* 147:2964, 1991). There is an equally stringent requirement for four amino acids to the left of the cleavage site, whereas methylamine is sufficient to the right. The minimal substrate for the enzyme, AC-Tyr-Val-Ala-Asp-NH-$CH_3$, is a particularly good peptide substrate with a relative Vmax/Km similar to that of the IL-1β precursor itself (Thomberry, *et al., Nature* 356:768, 1992).

ICE is a cysteinyl proteinase by the following criteria: (1) the diazomethylketone AC-Tyr-Val-Ala-Asp-$COCHN_2$

is a potent, competitive, irreversible inhibitor of the enzyme, (2) inactivation of the enzyme by iodoacetate is competitive with substrate, and (3) the catalytically active Cys reacts selectively with [$^{14}C$] iodoacetate more than 10 times faster than do other cysteines or dithiothreitol (Thornberry, *et al., Nature,* 356:768, 1992).

ICE is related structurally and functionally to the CED-3 protease that functions as a cell death effector in the roundworm *C. elegans* (Yuan, *et al., Cell,* 75:641, 1993). ICE and CED-3 form part of a larger family of proteases (the ICE/CED-3 family) that includes CPP32, ICH-1, Mch-2, $ICE_{rel}$II, $ICE_{rel}$III, Mch-3, Mch4 and Mch-5. All of these enzymes are cysteine proteases that share significant homology at their active sites. They also share the specificity for substrate cleavage at asp-x bonds. Additionally, each of the ICE/CED-3 family members is synthesized as a pro-enzyme that is then proteolytically activated to form an active enzyme.

Thus, disease states in which inhibitors of the ICE/ced-3 family of cysteine proteases may be useful as therapeutic agents include: infectious diseases, such as meningitis and salpingitis; septic shock, respiratory diseases; inflammatory conditions, such as arthritis, cholangitis, colitis, encephalitis, endocerolitis, hepatitis, pancreatitis and reperfusion injury, ischemic diseases such as the myocardial infarction, stroke and ischemic kidney disease; immune-based diseases, such as hypersensitivity; auto-immune diseases, such as multiple sclerosis; bone diseases; and certain neurodegenerative diseases.

In various cell culture systems, it has been shown that inhibition of ICE/CED-3 family members can effectively inhibit apoptosis. For example, the compound acetyl-DEVD-aldehyde inhibited anti-Fas induced apoptosis in a T-lymphocyte cell line (Schlegel, *et al., J. Biol. Chem.,* 271:1841, 1996; Enari, *et al., Nature,* 380:723, 1996). Similarly, acetyl-YVAD-aldehyde and acetyl-YVAD-chloromethylketone blocked the death of motoneurons in *vitro* and *in vivo* (Milligan, *et al., Neuron,* 15:385, 1995). In addition, the ICE/CED-3 family inhibitor Boc-D-(benzyl) chloromethylketone as well as crmA prevented the cell death of mammary epithelial cells that occurs in the absence of extracellular matrix (Boudreau, *et al., Science,* 267:891, 1995).

It is known that control of apoptosis may have utility in treating disease. Specifically, inhibitors of the ICE/CED-3 family may have therapeutic effects. For example, it has been suggested that inhibition of ICE may be useful in the treatment of inflammatory disorders (Dolle, *et al., J. Med. Chem.,* 37:563, 1994; Thornberry, *et al., Biochemistry,* 33:3934, 1994). It is also known that inhibitors of ICE/CED-3 family members may have utility in treating degenerative diseases such as neurodegenerative diseases (e.g., Alzheimer's disease, Parkinson's disease, amyotrophic lateral sclerosis, Huntington's disease), ischemic disease of heart or central nervous system (i.e., myocardial infarction and stroke), and traumatic brain injury, as well as in alopecia, AIDS and toxin induced liver disease (Nicholson, *Nature Biotechnology* 14:297, 1996).

Peptide and peptidyl inhibitors of ICE have been described. However, such inhibitors have been typically characterized by undesirable pharmacologic properties, such as poor oral absorption, poor stability and rapid metabolism. Plattner, J.J. and D.W. Norbeck, in Drug Discovery Technologies, C.R Clark and W.H. Moos, Eds. (Ellis Horwood, Chichester, England, 1990), pp. 92-126. These undesirable properties have hampered their development into effective drugs. The methods of this invention include either the use of conformationally constrained dipeptide mimetics or a N-substituted indolyl peptide replacement. These mimetics exhibit improved properties relative to their peptidic counterparts, for example, such as improved absorption and metabolic stability resulting in enhanced bioavailability.

## SUMMARY

The present invention is directed to preventing the programmed death of cells by inhibiting the activity of proteases of the interleukin-1β-converting enzyme (ICE)/CED-3 family. The current invention provides new methods for using ICE/CED-3 inhibitors. The present invention provides a method of using ICE/CED-3 inhibitors to expand hematopoietic cell subpopulations, enhance survival of cells, including granulocytes, *in vitro,* for use in cell transfusions, enhance survival of various organs, and enhance bioproduction from cells *in vitro.*

In a first embodiment, the invention provides an in vitro a method for expanding hematopoietic cell populations or enhancing their survival, including contacting the cells with an effective amount of a reagent which suppresses the activity of at least one member of the ICE/CED-3 family thereby inhibiting the programmed cell death of immature precursors and/or mature cells and expanding and/or enhancing the survival of the cell population. Cell populations included in the method of the invention include granulocytes, monocytes, erythrocytes, lymphocytes and platelets.

In another embodiment, the invention provides an in vitro a method for prolonging organ viability prior to and/or following transplantation, comprising contacting the cells of an organ with an inhibiting effective amount of a reagent which suppresses one or more ICE/CED-3 family members' activity, thereby prolonging the viability of the organ as compared to an untreated organ. An organ for transplantation can be treated with such a reagent either *in vivo, ex vivo,* or both. The organ may be either an intact organ, or isolated cells derived from an organ (*e.g.,* isolated pancreatic islet cells, isolated dopaminergic neurons, blood or hematopoietic cells).

In yet another embodiment, the invention provides a method for increasing bioproduction *in vitro* comprising contacting bioproducing host cells with a reagent which suppresses the activity of at least one member of the ICE/CED-3 family, thereby increasing survival of such cells *in vitro.*

Exemplary compounds useful as ICE/CED-3 inhibitors are also included herein. Such compounds and methods of synthesis are described in their entirety in co-pending U.S. Patent Applications 08/710,621, filed 9/20/96 and 08/767,175, filed 12/16/96 and their respective continuations-in-part.

## BRIEF DESCRIPTION OF THE DRAWINGS

Figure 1 sets forth the activity of compounds in Formula 3 in inhibiting the activity of ICE, CPP32, MCH2, MCH3 and MCH5 enzymes.

Figure 2 sets forth the activity of Example 106 in inhibiting the activity of ICE, CPP32, MCH2 and MCH5.

Figure 3 shows results dervied from FACS analysis demonstrating the effect of ICE/CED-3 inhibitors on neutrophil survival as measured by DNA content (% hypodiploid).

## DETAILED DESCRIPTION OF THE INVENTION

The present invention provides methods for the inhibition of programmed cell death, or apoptosis, by inhibition of members of the ICE/CED-3 family. This would include not only inhibitors of ICE/CED-3 enzymatic activity, but also any method which specifically prevents the expression of ICE/CED-3 family encoding genes. Thus, antisense RNA or DNA comprised of nucleotide sequences complementary to ICE/CED-3 family member genes and capable of inhibiting the transcription or translation of the relevant proteins, expression of dominant negative forms of the ICE/CED-3 proteases (e.g. mutants engineered to replace the active site cysteine with another amino acid, like serine or alanine), or antibodies which bind to ICE/CED-3 family polypeptides, are within the scope of the invention, as are small molecule inhibitors, including peptides.

The compounds of Formula 3, below, are also useful in the methods of the invention:

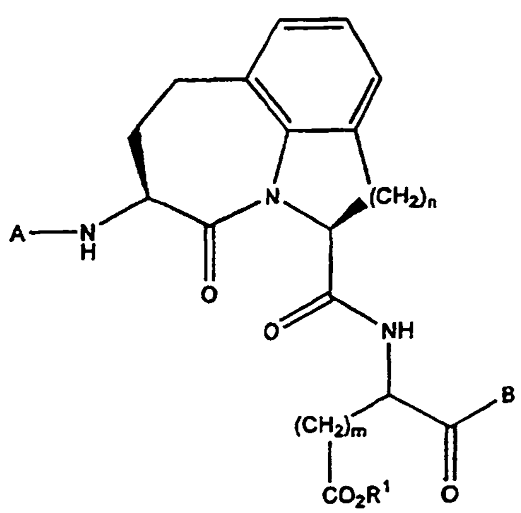

FORMULA 3

wherein:

n is 1 or 2;

m is 1 or 2;

A is $R^2CO$-, $R^3$-O-CO-, or $R^4SO_2$-;

a group of the formula:

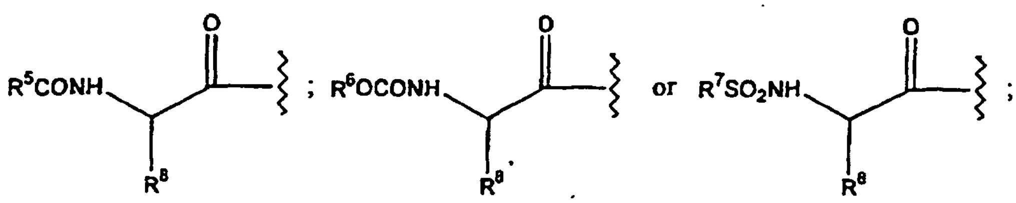

further wherein:

$R^1$ is a hydrogen atom, alkyl or phenylalkyl;

$R^2$ is alkyl, cycloalkyl, (cycloalkyl)alkyl, phenyl, phenylalkyl, substituted phenyl, (substituted phenyl)alkyl, heteroaryl, or (heteroaryl)alkyl;

$R^3$ is alkyl, cycloalkyl, (cycloalkyl)alkyl, phenylalkyl, or (substituted phenyl)alkyl;

$R^4$ is alkyl, cycloalkyl, (cycloalkyl)alkyl, phenyl, phenylalkyl, substituted phenyl, (substituted phenyl)alkyl, heteroaryl, or (heteroaryl)alkyl;

$R^5$ is alkyl, cycloalkyl, (cycloalkyl)alkyl, phenyl, phenylalkyl, substituted phenyl, (substituted phenyl)alkyl, heteroaryl, or (heteroaryl)alkyl;

$R^6$ is alkyl, cycloalkyl, (cycloalkyl)alkyl, phenylalkyl, or (substituted phenyl)alkyl;

$R^7$ is alkyl, cycloalkyl, (cycloalkyl)alkyl, phenyl, phenylalkyl, substituted phenyl, (substituted phenyl)alkyl, heteroaryl, or (heteroaryl)alkyl;

$R^8$ is an amino acid side chain chosen from the group consisting of natural and unnatural amino acids;

B is a hydrogen atom, a deuterium atom, alkyl, cycloalkyl, (cyclalkyl)alkyl, phenyl, phenylalkyl, (substituted)phenyl, (substituted)phenylalkyl, heteroaryl, (heteroaryl)alkyl, or halomethyl;

a group of the formula

$$-CH_2XR^9;$$

wherein $R^9$ is phenyl, phenylalkyl, substituted phenyl, (substituted phenyl)alkyl, heteroaryl, or (heteroaryl)alkyl; and X is an oxygen or a sulfur atom;

a group of the formula:

$$-CH_2\text{-O-CO-(aryl)};$$

a group of the formula:

$$-CH_2\text{-O-CO-(heteroaryl)};$$

a group of the formula:

$$-CH_2-O-PO(R^{10})R^{11}$$

wherein $R^{10}$ and $R^{11}$ are independently selected from a group consisting of alkyl, cycloalkyl, phenyl, substituted phenyl, phenylalkyl and (substituted phenyl) alkyl; and the pharmaceutically-acceptable salts thereof.

The compounds of Formula 3 may also exist as solvates and hydrates. Thus, these compounds may crystallize with, for example, waters of hydration, or one, a number of, or any fraction thereof of molecules of the mother liquor solvent. The solvates and hydrates of such compounds are included within the scope of this invention.

The compounds of Formulas 1 and 3 of this invention may be synthesized using conventional techniques. Advantageously, these compounds are conveniently synthesized from readily available starting materials.

Thus, compounds of Formula 3 can be synthesized in general by combining a tricyclic nucleus set forth below in Formula 4:

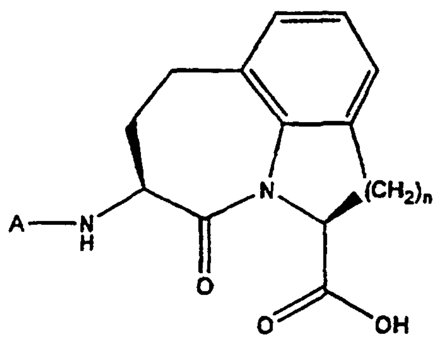

FORMULA 4

with the modified aspartic and glutamic acid residues of Formula 5a-d:

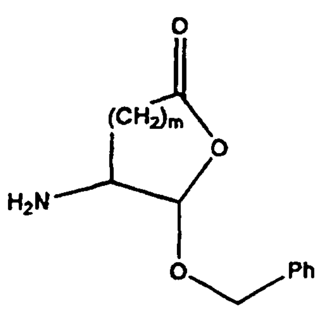

FORMULA 5a ;

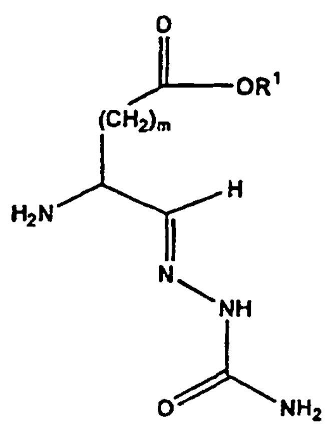

FORMULA 5b ;

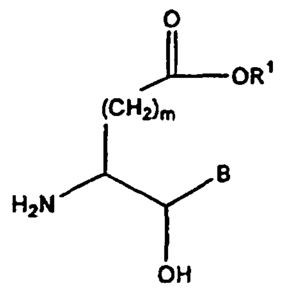

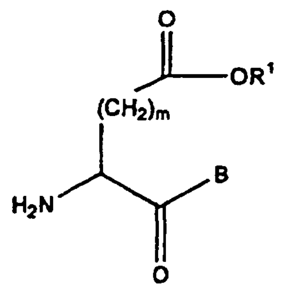

**FORMULA 5c; or** **FORMULA 5d**

in the presence of a standard peptide coupling agents such as dicyclohexylcarbodiimide(DCC)-1-bydroxybenzotriazole (HOBt), BOP reagent, pyBOP, TBTU, EEDQ, 1-ethyl(3,3'-dimethyl-1'-aminopropyl)carbodiimide(EDAC)-HOBt, and the like, as discussed in J. Jones, "Amino Acid and Peptide Synthesis," Steven G. Davis ed., Oxford University Press, Oxford, pp. 25-41 (1992). In the above formula, A is an amino protecting group. The amino protecting group is then removed and the resulting amine is combined with the substituted acyl group of Formula 6:

$$R^C\text{-CO-X}$$

**FORMULA 6**

or the sulfonyl group of Formula 7:

$$R^4SO_2\text{-X.}$$

**FORMULA 7**

In the above formulas, $R^1$ is as defined above, and $R^c$ is $R^2$, $R^3$ -O, $R^4$, or any of the side chains containing $R^8$ as defined for group A in Formula 3. Of course, such moieties would have any hydroxy, carboxy or amino groups in the protected form so as not to interfere with the coupling reaction (Formula 5a-d), the acylation reaction (Formula 4) or the sulfonation reaction (Formula 7). X in the above Formulas represents a facile leaving group for the acylation or sulfonation reactions.

In the case where the coupling reaction was carried out with the amino alcohol of Formula 5c, the alcohol moiety must be oxidized to the corresponding carbonyl compound prior to removal of the protecting groups. Preferred methods for the oxidation reaction include Swern oxidation (oxalyl chloride-dimethyl sulfoxide, methylene chloride at -78°C followed by triethylmine; and Dess-Martin oxidation (Dess-Martin periodinane, t-butanol, and methylene chloride.) The protecting groups contained in substructures of the Fomula 5a-d and A are removed by methods well known in the art.

The tricyclic nucleus of Formula 3 is synthesized by methods known in the art. For example, see D.S. Karanewsky, U.S. Patent No. 5,504,080 issued April 2, 1996; J.A. Robl *et al.*, Tetrahedron Letters, 36:1593-1596 (1995); and S. De Lombaert *et al.,* Tetrahedron Letters, 35:7513-7516 (1994)

The modified aspartic or glutamic acid for Formula 5a-d can be elaborated by methods well known in the art. See, for example, European Patent Application 519,748; PCT Patent Application No. PCT/EP92/02472; PCT Patent Application No. PCT/US91/06595; PCT Patent Application No. PCT/US91/02339; European Patent Application No. 623,592; World Patent Application No. WO 93/09135; PCT Patent Application No. PCT/US94/08868; European Patent Application No. 623,606; European Patent Application No. 618,223; European Patent Application No. 533,226; European Patent Application No. 528,487; European Patent Application No. 618,233; PCT Patent Application No. PCT/EP92/02472; World Patent Application No. WO 93/09135; PCT Patent Application No. PCT/US93/03589; and PCT Patent Application No. PCT/US93/00481.

The acyl group of Formula 6 and the corresponding $R^4SO_2$ groups are also synthesized by methods well

known in the art. See, for example, U.S. Patent No. 5,504,080, issued April 2, 1996. While this group can be elaborated once bonded to the tricyclic nucleus, it is preferable that it be intact before being attached to the nucleus.

Once the side chains of Formula 5 and Formula 6 or Formula 7 are bonded to the tricyclic nucleus of Formula 3, one skilled in the art would usually remove any amino, hydroxy, or carboxy-protecting groups to enhance the activity of the synthesized molecule.

In Formula 3 above, a group of optimal compounds occurs when n is one, more so when B is a hydrogen atom, and especially so when $R^3$ is a hydrogen atom or a t-butyl group. Of note within this group of compounds as those when A is naturally-occurring amino acid. This latter group of compounds will be referred to herein as the "4-oxobutanoic compounds".

Within this group of 4-oxobutanoic compounds is a group of optimal compounds wherein $R^1$ is a methyl group, that is, the N-methylindole compounds. One embodiment of this group of N-methylindole compounds occurs when A is an alanine, valine, leucine, phenylalanine, glycine or a proline residue. Compounds of note within each one of these groups of natural amino acid, N-methylindole compounds occur when the N-methylindole is otherwise unsubstituted, that is, wherein X, Y and $R^2$ are each a hydrogen atom, and optimally so when $R^3$ is a hydrogen atom.

Another optimal group of 4-oxobutanoic compounds consists of the N-benzylindole compounds. For example, one group of the N-benzylindole compounds occurs when A is an alanine residue. Of note within this group of alanine compounds are those in which X, Y and $R^2$ are each a hydrogen atom, and especially so where $R^3$ is a hydrogen atom.

An alternate optimal group of 4-oxobutanoic compounds occurs when the N-substituent of the indole group is a 1-butenyl group. An embodiment of this group of N-(1-butenyl)indole compounds occurs when A is a valine residue, and especially so when X, Y and $R^2$ are each a hydrogen atom. An optimal group of this latter group of compounds occurs when $R^3$ is a hydrogen atom.

Yet another group of optimal 4-oxobutanoic compounds occurs when the N-substituent of the indole ring is a 2'-acetic acid residue. An exemplary group of the N-(2'-acetic acid compounds) occurs when A is an alanine residue. An embodiment of this particular group of alanine compounds occurs when X, Y and $R^2$ are each a hydrogen atom, and especially so when $R^3$ is a hydrogen atom.

A group of the 4-oxobutanoic compounds when the indole group is substituted on the nitrogen with 3'-propionic acid residue is another example of this invention. An optimal group of such N-(propionic acid)indole compounds occurs when A is an alanine residue. Of note within this group of alanine compounds are those when X, Y and $R^2$ are each a hydrogen atom, and especially so when $R^3$ is a hydrogen atom.

Another optimal group of compounds of Formula 1 occurs wherein n is one and more so when B is a monofluoromethyl group. An embodiment of these monofluoromethyl compounds occurs when $R^3$ is a hydrogen atom or a t-butyl group, and an even more so when A is a natural amino acid. An example of these compounds wherein A is a natural amino acid occurs when A is a valine residue. This latter group of valine compounds will be referred to herein as the "4-oxo-5-(fluoropentanoic acid) compounds".

One optimal group of 4-oxo-5-(fluoropentanoic acid) compounds occurs when $R^1$ is a methyl group, in other words, the N-methylindole compounds. An exemplary group of such N-methylindole compounds occurs when $R^2$ is a methyl group and X and Y are each a hydrogen atom, and especially so when $R^3$ is a hydrogen atom. Another exemplary group of such N-methylindole compounds occurs when $R^2$ is a chloro atom and X and Y are each a hydrogen atom, and especially so when $R^3$ is a hydrogen atom. A third exemplary group of N-methylindole compounds occurs when $R^2$ is a chloro group, X is a 5-fluoro group, and Y is a hydrogen atom, and especially so when $R^3$ is a hydrogen atom.

Another optimal group of 4-oxo-5-(fluoro-pentanoic acid) compounds is composed of N-(3'-phenylprop-1-yl) indole compounds. A group of note within this latter class of compounds occurs when $R^2$, X and Y are each a hydrogen atom, and especially so when $R^3$ is a hydrogen atom.

A third optimal group of 4-oxo-5-(fluoro-pentanoic acid) compounds has an N-(carboxymethyl or protected carboxymethyl)indole moiety. An embodiment of this group occurs wherein $R^2$, X and Y are each a hydrogen atom, and especially so wherein $R^3$ is a hydrogen atom and the nitrogen atom of the indole ring is substituted with a carboxymethyl group.

## ***METHODS FOR INHIBITING APOPTOSIS***

The present invention provides in vitro methods for the inhibition of programmed cell death, or apoptosis, by inhibition of members of the ICE/CED-3 family. The invention provides new uses for not only inhibitors of ICE/CED-3 enzymatic activity, but also any method which specifically prevents the expression of ICE/CED-3 family encoding genes. Thus, antisense RNA or DNA comprised of nucleotide sequences complementary to ICE/CED-3 family member genes and capable of inhibiting the transcription or translation of the relevant proteins, expression of dominant negative forms of the ICE/CED-3 proteases (e.g. mutants engineered to replace the active site cysteine with another amino acid, like serine or alanine), or antibodies which bind to ICE/CED-3 family polypeptides, are within the scope of the invention, as are small molecule inhibitors, including peptides and especially the compounds presented herein.

In a first aspect, the invention provides a method for expanding hematopoietic and blood cell populations or prolonging survival of such populations, including contacting the cells ex vivo with an effective amount of a reagent which suppresses the activity of one or more ICE/CED-3 family members, inhibiting the programmed cell death of immature precursors and/or mature cells, thereby expanding the cell population and/or enhancing the survival of the cell population. The term "expansion" or "expanding" as used herein means increasing the number of cells of a pre-existing cell population. The term "survival" refers to maintaining viability of cells, typically *ex vivo,* however the term is meant to include *in vivo* as well. Survival may be from a few hours to several days or longer.

The method includes contacting the desired cells ex vivo with an inhibiting effective amount of a reagent which suppresses ICE/CED-3 activity. The term "contacting" as used herein means exposing the cells to the ICE/CED-3 family inhibitor(s) such that the inhibitor(s) can effectively inhibit ICE/CED-3 activity thereby inhibiting apoptosis in the cells and allowing the cells to proliferate and accumulate. The term "inhibiting effective amount" means that amount of ICE/CED-3 inhibitor that effectively blocks ICE/CED-3 enzymatic activity in intact target cells. It will be apparent that one or more ICE/CED-3 family inhibitors can be used simultaneously in the method of the invention.

Examples of such reagents are commonly known in the art, including Cbz-VaLAlaAsp-$CH_2F$, Cbz-ValAlaAsp-$CH_2OCO$(2,6-diCl-$C_6H_4$), Cbz-ValAlaAsp-$CH_2F$, methyl ester, Ac-AspValAlaAsp-$CH_2F$. Exemplary compounds are those of Formula 3 as described *supra.*

Detection of ICE/CED-3 activity is by standard methods, such as an enzymatic assay to measure the fluorescence generated by enzymatic cleavage of aminomethylcoumarin (AMC) conjugated to a relevant peptide (e.g., Ac-DEVD-amc). Such assays are standard in the art (Armstrong *et al., J. Biol. Chem.* 271:16850, 1996); Fernandes-Alnemri, *et al., Cancer Res.,* 55:6045, 1995). In addition, the inhibition of ICE activity can be measured by a bioassay for IL-1β. ICE/CED-3 activity is preferably suppressed by the ICE/CED-3 family inhibitor(s) by at least about 75%, and preferably by about 90%.

The "cells" or "cell population" include precursor cells (e.g., pluripotent stem cells) and/or differentiated, mature cells. Examples of cells expanded and/or whose survival is enhanced by the method of the invention include but are not limited to granulocytes (e.g., neutrophils), monocytes, erythrocytes, lymphocytes and platelets.

Success of hematopoiesis can also be detected by measuring hematocrit, white blood cell count, incorporation of tritiated thymidine into bone marrow DNA, spleen weight, number of burst-forming units-erythroid or number of colony-forming units (erythroid, granulocyte/macrophage and megakaryocyte forming lineages) from spleen or bone marrow for example.

Hematopoietic disorders can occur as a result of primary disease or as a consequence of therapy for another disease. For example, a serious side effect of radiation or chemotherapy is myelosuppression. Pancytopenia can be observed following bone marrow transplantation and profound leukopenia is often observed in AIDS. Patients with chronic renal failure undergoing dialysis often have anemia. AIDS patients treated with AZT, cancer patients treated with platinum, and anemic patients with rheumatoid arthritis often require transfusions because of anemia and leukopenia. Various hematopoietic growth factors, such as erythropoietin, granulocyte or granulocyte-macrophage colony stimulating factors, or cytokines such as interleukin-1 are known to stimulate hematopoiesis and have been used to treat these conditions. However, treatment times are generally on the order of weeks, and especially for the case of neutropenias, patients are at risk for opportunistic infections. These therapies are usually quite expensive, as they are recombinantly produced proteins. Thus, it would be advantageous to be able to administer a small molecule, e.g., ICE/CED-3 inhibitor, either alone or in combination with a growth factor to speed hematopoietic recovery. Various cytokines including hematopoietic growth factors, are known to both increase proliferation and inhibit apoptosis in target cells (Koury and Bondurant, *Science,* 248:378, 1990; Muta and Krantz, *J. Cell Physiol.,* 156:264, 1993). However, those agents act upstream of the apoptotic cellular machinery, affecting that machinery only indirectly. The present invention provides methods to enhance cell expansion by directly inhibiting the apoptotic machinery. The combination therapy comprising administering a hematopoietic growth factor together with the methods of the present invention is a preferable embodiment.

The method of the invention is useful for restoring normal hematopoiesis in individuals in need of reconstitution of their bone marrow. The pluripotent stem cell has the unique capacity for self-renewal and the potential for growth and differentiation along granulocytic, monocytic, erythroid, megakaryocytic, and lymphoid lineages.

As one skilled in the art will also appreciate, stem cells can be directed to differentiation as a lymphoid, myeloid, or erythroid-lineage population by incubation with the appropriate hematopoietic growth factor or combination of growth factors. Therefore, the method of the invention further includes contacting the cells with a suitable hematopoietic growth factor, in addition to the ICE/CED-3 inhibitor. As used herein, the term "a suitable hematopoietic growth factor" means one or more hematopoietic growth factors known in the art to direct a progenitor stem cell to the desired lymphoid, granulocytic, monocytic, megakaryocytic, myeloid, or erythroid-lineage cell population. Various growth factors function both by promoting proliferation of cells and by inhibiting apoptosis; both of these functions promote the accumulation of cells. Certain hematopoietic growth factors, including G-CSF, GM-CSF, and erythropoietin are examples of such growth factors that can inhibit programmed cell death of their respective target cells, as well as stimulate proliferation

of those cells. These factors have proven clinically useful in promoting the repopulation of granulocytes (e.g., neutrophils), monocytes and erythrocytes in patients. G-CSF and GM-CSF are often used in patients following chemotherapy or radiation, and erythropoietin is commonly used in patients undergoing kidney dialysis. Other representative growth factors known in the art that can be used for these purposes are IL-1, IL-6, stem cell factor, and IL-3. Other growth factors for stimulating proliferation of hematopoietic lineages will be known to those of skill in the art (see, for example, Harrison's Principles of Internal Medicine, Isselbacher, *et al.,* eds., pp 1714-1717, McGraw Hill, 1994).

Apoptosis inhibitors, such as ICE/CED-3 inhibitors, can extend survival of blood cell precursors in the bone marrow as well as extend survival of the mature blood cells themselves. Regarding extension of survival of mature blood cells, this may be particularly valuable for repopulation of granulocytes following chemotherapy and/or radiation (with or without bone marrow transplant). Mature granulocytes (specifically neutrophils) last for only 24 hours in the bloodstream after which they die by apoptosis. ICE/CED-3 inhibitors that block apoptosis and increase neutrophil survival would increase the rate at which a patient normalized his white blood cell count. Inhibition of apoptosis in precursor cells which ultimately give rise to the mature cell populations would be synergistic with effects on mature cells.

The invention provides methods to preserve the viability of neutrophils/granulocytes ex vivo for subsequent transfusion into recipients in need of additional granulocytes. The life-span of granulocytes removed from donors is limited and thus it is difficult to obtain supplies of functional granulocytes for transfusion.

The reagents of the present invention are "ICE/CED-3 inhibitors" in that they inhibit the catalytic activity of members of the ICE/CED-3 family in a reversible or an irreversible manner. The term "irreversible" as used herein means the formation of a covalent bond between the ICE/CED-3 family member and the inhibitor. It is possible to convert a reversible inhibitor to an irreversible inhibitor by incorporating an irreversible "warhead" into what would otherwise be a reversible inhibitor.

The reversibility of ICE/CED-3 inhibition is generally a function of the electronegative group in the molecule. When the electronegative group is a diazoalkyl ketone, the inhibition of ICE activity is irreversible and the compound is an irreversible inhibitor. When the electronegative group is an aldehyde, the inhibition of ICE is reversible and the inhibitor is a reversible inhibitor.

A compound of the invention preferably has an aldehyde, a diazoalkyl ketone, a haloalkyl ketone, or acyloxymethyl ketone. As used herein in reference to an electronegative group, "alkyl" refers to linear or branched chain radicals having 1-3 carbon atoms, which may be optionally substituted. Representative alkyl groups include methyl, ethyl, propyl and the like. Optionally, the electronegative group is an aldehyde, fluoromethyl ($CH_2F$) ketone, or acyloxylmethyl ketone.

The compounds of the present invention are made by techniques generally corresponding to methods known and readily apparent to those of skill in the art. *See, e.g.,* Kettner, *et al., Arch, Biochem, Biophys.,* 162:56, 1974; U.S. Pat. Nos. 4,582,821; 4,644,055; Kettner, *et al. Arch, Biochem, Biophys,* 165:739, 1974; Dakin and West, *J. Biol. Chem.,* 78:91, 1928; Rasnick, D., *Anal. Biochem.,* 149:461, 1985; Revesz, L., *Tetrahedron Lett.,* 35:9693, 1994. Exemplary indolyl dipeptide and tricyclic compounds are provided herein.

Compounds having a non-fluoro, haloalkyl ketone electronegative leaving group are preferably synthesized in accordance with the Kettner procedure. An N-blocked amino acid or peptide is reacted with N-methylmorpholine and an alkyl, non-fluoro haloformate to generate a peptide-acid anhydride. The anhydride is then reacted with a diazoalkane in an inert, aprotonic solvent to form a peptide-diazomethane ketone. The diazomethane ketone is then reacted with an anhydrous solution of HCl, HBr or HI to produce the desired N-blocked, C-terminal haloalkyl ketone peptide or amino acid.

Compounds having a fluoromethyl electronegative leaving group are preferably synthesized by the Revesz procedure. An N-blocked peptide or amino acid is reacted with t-butyl (3-amino-4-hydroxy-5-fluoro) pentanoate in the presence of a standard peptide coupling agent such as dicyclohexylcarbodiimide-hydroxy-benztriazole. The resulting product is oxidized to the corresponding ketone by either Severn or Dess-Martin oxidation. Finally, deprotection of the t-butylester with trifluoracetic acid gives the corresponding carboxylic acid.

Compounds having a fluoroalkyl ketone electronegative leaving group can be extended in the N-terminus direction by removing the N-terminal blocking group and coupling the deprotected compound with other protected amino acids. Bodanszky, *The Practice of Peptide Synthesis,* Springer-Verlag, Berlin, 1984. Alternatively, deprotected compounds are acetylated to yield compounds having an N-terminal acetyl protecting group. Stewart, *et al.,* Solid Phase Peptide Synthesis, *Pierce Chemical Co., ockford, III.,* 1984.

In another embodiment, the invention provides a method for prolonging organ viability comprising contacting the cells of an organ for transplantation with an inhibiting effective amount of a reagent which suppresses interleukin-1β-converting enzyme (ICE)/CED-3 activity, thereby prolonging the viability of the organ as compared to an untreated organ. The term "organ" is meant to include intact multi-cellular organs such as kidney, liver, or heart; cell suspensions derived from multi-cellular organs (e.g., pancreatic islet cells in dopaminergic neurons); as well as suspensions of blood cells or hematopoietic precursor cells. Preferably, an organ is treated *ex vivo* in order to preserve the organ for transplantation. The term "prolonging" means that an organ for transplantation is preserved by treatment using the

method of the invention as compared to a similar organ that has not been treated with an ICE/CED-3 inhibitor. While not wanting to be bound by a particular theory, it is believed that contacting the cells of an organ for transplantation with an ICE/CED-3 inhibitor, inhibits programmed cell death, thereby preserving the organ and prolonging viability.

The method of the invention includes treatment of the recipient prior and subsequent to transplantation with genetically altered or ICE/CED-3 inhibitor-bathed donor organs to inhibit apoptosis of donor organ cells, and optionally, an immunosuppressive agent.

The present invention includes the *ex vivo* expansion or survival of hematopoietic cells which is useful for a variety of clinical uses including gene therapy, augmentation of bone marrow transplantation, and the replacement of bone marrow transplantation. Therefore, the use of an inhibitor of the apoptosis machinery, such as an ICE/CED-3 family inhibitor, to promote the *ex vivo* expansion and/or survival of populations of granulocytes, erythrocytes, lymphocytes and or platelet constitutes a useful method. For example, one can add the ICE/CED-3 inhibitor(s) to a tissue culture of cells isolated from a subject, or by transfecting the cells with an expression vector containing an operable ICE/CED-3 inhibitor-encoding polynucleotide, e.g., antisense, to such cells.

The method of the invention has therapeutic utility. For instance, a suspension of pluripotent hematopoietic stem cells (HSC) obtained by standard methods in the art, can be treated by the method of the invention and can be used for performing hematopoietic reconstitution of a recipient. As an *ex vivo* treatment for example, enriched pluripotent stem cells derived from the recipient (autologous reconstitution) or derived from an individual other than the recipient (non-autologous reconstitution) can be expanded and used in the treatment or prevention of various diseases or disorders such as anemias, malignancies, autoimmune disorders, and various immune dysfunctions and deficiencies, as well as recipients whose hematopoietic cellular repertoire has been depleted, such as recipients treated with various chemotherapeutic or radiologic agents, or recipients with AIDS. Other therapeutic uses of the compositions of the invention are well known to those of skill in the art. Treatment of the transplanted cells can be both *ex vivo*, and following transplantation, *in vivo.*

An example of the way in which the invention may be applied to non-autologous donor organs for human recipients is as follows: beginning one week prior to the transplantation, the recipient may be dosed with cyclophosphamide to reduce the potential for evoked immunological responses. An immunosuppressive dose of cyclosporine or FK506 may be started shortly (1-3 days) before transplantation to enhance graft acceptance. Immediately prior to transplantation, the donor may be dosed with an ICE/CED-3 inhibitor, prior to organ removal. Upon removal prior to transplantation, the donor organ is flushed with a solution containing at least one ICE/CED-3 inhibitor, e.g., a peptide fluoroalkyl ketone. Following transplantation by standard surgical techniques, the patient is typically maintained on routine immunosuppression using cyclosporine or FK506, cyclophosphamide, and steroids and optionally, the ICE/CED-3 inhibitor(s). Based on clinical signs and symptoms related to immune responsiveness, various of the immunosuppressants are reduced in dosage.

An immunosuppressive agent used during transplantation is an agent such as Cyclosporine A (CsA), however other agents which cause immune suppression, such as rapamycin, desoxyspergualine, and FK506 or functional equivalents of these compounds, may also be utilized. CsA is preferably administered by injection at an immunosuppressive dose. The duration of CsA treatment may range from about 2 to about 20 days.

For enhancing organ survival prior to transplantation, the ICE/CED-3 family inhibitor may be administered by addition to the fluid used to perfuse the excised organ. If the ICE/CED-3 inhibitor is also to be administered to the donor prior to excision of the organ, the ICE/CED-3 inhibitor(s) are administered by any suitable means, including parenteral, subcutaneous, intrapulmonary, and intranasal administration. Parenteral infusions include intramuscular, intravenous, intraarterial, or intraperitoneal administration.

It is understood that any organ from any species can be transplanted. The method of the invention is useful for preserving organs to be used for same species transplants such as human recipients and other human donors (allografts and autologous grafts) or to human recipients from other species such as sheep, pigs, or non-human primates (xenografts), for example. Such tissues for transplant include, but are not limited to, heart, liver, kidney, lung, pancreas, pancreatic islets, brain tissue, cornea, bone, intestine, skin, and hematopoietic cells. The human is the preferred recipient.

In order to determine the amount of ICE/CED-3 inhibitor to administer to the donor, e.g., pig, and the amount of ICE/CED-3 inhibitor with which to treat the organ to be transplanted, a compound is administered so that its concentration in the vasculature is at least as high as that necessary for inhibition of apoptosis in a cell culture model. A variety of cell culture models are known in the art (e.g., Armstrong *et al., supra;* Schlegel, *et al., supra;* Boudreau, *et al., supra).* The donor organ, and optionally the donor, is then dosed with an ICE inhibitor in order to reduce apoptosis to as much as less than about 10% of the normal apoptosis observed in the organ from the donor. Therefore, as used herein, the term "apoptosis-inhibiting" amount refers to that amount of ICE inhibitor that inhibits ICE activity and/or apoptosis by about 75%, and preferably by about 90%.

## *METHOD FOR ENHANCING BIOPRODUCTION*

In yet another embodiment, the invention provides a method for increasing survival of cells cultured *in vitro* for utilities other than transplantation. For example, inhibition of programmed cell death is of use in enhancing bioproduction processes. For example, in production of recombinant proteins, yields may be limited by apoptotic cell death of cultured host cells. Therefore, treatment of host cells with ICE/CED-3 inhibitors, or using host cells which express antisense RNA or DNA sequences complementary to ICE/CED-3 and capable of inhibiting the transcription or translation of ICE/CED-3 family members or that express genes encoding ICE/CED-3 family inhibitors, would enhance bioproduction by allowing cells to survive longer and produce and/or secrete a desired product longer, thus resulting in a greater yield of product. The ability to prevent programmed cell death may allow cells to live independent of normally required growth factors, reducing the cost of media supplements.

In order to demonstrate utility in bioproduction, a cell line that naturally produces a useful product (e.g., a cytokine) or a cell line genetically engineered to express a useful product (e.g., COS or CHO cells stably expressing recombinant human erythropoieten, growth hormone or G-CSF) is contacted with the ICE/CED-3 inhibitor during fermentation. Effectiveness of the ICE/CED-3 inhibitor on bioproduction can be measured in several way 1) determining the percentage of apoptotic cells in the culture at different time points; 2) determining the useful lifespan of the culture with regard to production of the desired product; 3) measuring the yield of product per gram of cells or per volume of culture; 4) measuring final purity of the product.

Methods for increasing the efficiency or overall productivity of bioproduction are useful because they reduce costs of producing a natural or recombinant product. Mammalia cell fermentation is limited by decreasing cell viability with time. Cell death during fermentation has been shown to be apoptotic, thus inhibitors of apoptosis will increase cell viability during bioproduction. Growth media used for bioproduction are often serum-free supplemented with growth factors to enhance cell viability. Supplementation with ICE/CED-3 ihhibitors of the present invention is designed to replace or agument such additives.

## *PHARMACEUTICAL COMPOSITIONS*

Pharmaceutical compositions of this invention comprise any of the compounds of the present invention, and pharmaceutically acceptable salts thereof, with any pharmaceutically acceptable carrier, adjuvant or vehicle (hereinafter collectively referred to as "pharmaceutically-acceptable casriers"). Pharmaceutically acceptable carriers, adjuvants and vehicles that may be used in the pharmaceutical compositions of this invention include, but are not limited to, alumina, aluminum stearate, lecithin, serum proteins, such as human serum albumin; buffer substances such as the various phosphates, glycine, sorbic acid, potassium sorbate, partial glyceride mixtures of saturated vegetable fatty acids; water, salts or electrolytes, such as protamine sulfate, disodium hydrogen phosphate, potassium hydrogen phosphate, sodium chloride, and zinc salts; colloidal silica, magnesium trisilicate, polyvinyl pyrrolidone, cellulose-based substances, polyethylene glycol, sodium carboxymethylcellulose, polyarylates, waxes, polyethylene-polyoxypropylene-block polymers, polyethylene glycol and wool fat, and the like.

The pharmaceutical compositions of this invention may be administered orally, parenterally, by inhalation spray, topically, rectally, nasally, buccally, vaginally or by an implanted reservoir. Oral and parenteral administration are preferred. The term "parenteral" as used herein includes subcutaneous, intracutaneous, intravenous, intramuscular, intra-articular, intrasynovial, intrasternal, intrathecal, intralesional and intracranial injection or infusion techniques.

The pharmaceutical compositions may be in the form of a sterile injectable preparation, for example, as a sterile injectable aqueous or oleaginous suspension. This suspension may be formulated according to techniques known in the art using suitable dispersing or wetting agents (such as, for example, Tween 80) and suspending agents. The sterile injectable preparation may also be a sterile injectable solution or suspension in a non-toxic parenterally-acceptable diluent or solvent, for example, as a solution in 1,3-butanediol. Among the acceptable vehicles and solvents that may be employed are mannitol, water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant.

The pharmaceutical compositions of this invention may be orally administered in any orally acceptable dosage form including, but not limited to, capsules, tablets, and aqueous suspensions and solutions. In the case of tablets for oral use, carrier which are commonly used include lactose and corn starch. Lubricating agents, such as magnesium stearate, are also typically added. For oral administration in capsule form useful diluents include lactose and dried corn starch. When aqueous suspensions are administered orally, the active ingredient is combined with emulsifying and suspending agents. If desired, certain sweetening and/or flavoring and/or coloring agents may be added.

The pharmaceutical compositions of this invention may also be administered in the form of suppositories for rectal administration. These compositions can be prepared by mixing a compound of this invention with a suitable non-irritating excipient which is solid at room temperature but liquid at the rectal temperature. Such materials include, but are not limited to, cocoa butter, beeswax and polyethylene glycols.

Topical administration of the pharmaceutical compositions of this invention is especially useful when the desired treatment involves areas or organs readily accessible to topical application. For application topically to the skin, the pharmaceutical composition should be formulated with a suitable ointment containing the active components suspended or dissolved in a carrier. Carriers for topical administration of the compounds of this invention include, but are not limited to, mineral oil, liquid petroleum, white petroleum, propylene glycol, polyoxyethylene, polyoxypropylene compound, emulsifying wax and water. Alternatively, the pharmaceutical composition can be formulated with a suitable lotion or cream containing the active compound suspended or dissolved in a carrier. Suitable carriers include, but are not limited to, mineral oil, sorbitan monostearate, polysorbate 60, cetyl esters wax, cetearyl alcohol, 2-octyldodecanol, benzyl alcohol and water. The pharmaceutical compositions of this invention may also be topically applied to the lower intestinal tract by rectal suppository formulation or in a suitable enema formulation. Topically-applied transdermal patches are also included in this invention.

The pharmaceutical compositions of this invention may be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art.

The compounds of this invention may be used in combination with either conventional anti-inflammatory agents or with matrix metalloprotease inhibitors, lipoxygenase inhibitors and antagonists of cytokines other than IL-1β.

When the compounds of this invention are administered in combination therapies with other agents, they may be administered sequentially or concurrently to the patient. Alternatively, pharmaceutical compositions according to this invention may be comprised of a combination of a compound of Formula 1 or 3 or other ICE inhibitors as described herein, and another therapeutic or prophylactic agent mentioned above.

The disease states which may be treated or prevented by the instant pharmaceutical compositions include, but are not limited to, inflammatory diseases, autoimmune diseases and neurodegenerative diseases, and for inhibiting unwanted apoptosis involved in ischemic injury, such as ischemic injury to the heart (e.g., myocardial infarction), brain (e.g., stroke), and kidney (e.g., ischemic kidney disease). As a consequence of their ability to inhibit apoptosis, the present pharmaceutical compositions are also useful for the repopulation of hematopoietic cells of a patient following chemotherapy. Methods of administering an effective amount of the above-described pharmaceutical compositions to mammals, also referred to herein as patients, in need of such treatment (that is, those suffering from inflammatory diseases, autoimmune diseases, neurodegenerative diseases and for the repopulation of hematopoietic cells in cancer patients who have undergone chemotherapy) are another aspect of the instant invention. Finally, as a further consequence of their ability to inhibit apoptosis, the instant pharmaceutical compositions may be used in a method to prolong the viability of organs to be used in transplantations.

Inflammatory disease which may be treated or prevented include, for example, septic shock, septicemia, and adult respiratory distress syndrome. Target autoimmune diseases include, for example, rheumatoid, arthritis, systemic lupus erythematosus, scleroderma, chronic thyroiditis, Graves' disease, autoimmune gastritis, insulin-dependent diabetes mellitus, autoimmune hemolytic anemia, autoimmune neutropenia, thrombocytopenia, chronic active hepatitis, myasthenia gravis and multiple sclerosis. Target neurodegenerative described in this invention may also be used to promote wound healing. Target diseases associated with harmful, apoptosis, in other words, those associated with ischemic injury, includes myocardial infarction, stroke, and ischemic kidney disease.

The term "effective amount" refers to dosage levels of the order of from about 0.05 milligrams to about 140 milligrams per kilogram of body weight per day for use in the treatment of the above-indicated conditions (typically about 2.5 milligrams to about 7 grams per patient per day). For example, inflammation may be effectively treated by the administration of from about 0.01 to 50 milligrams of the compound per kilogram of body weight per day (about 0.5 milligrams to about 3.5 grams per patient per day).

The amount of the compounds of Formula 1, 3, or other ICE/ced-3 inhibitors that may be combined with the carrier materials to produce a single dosage form will vary depending upon the host treated and the particular mode of administration. For example, a formulation intended for the oral administration of humans may contain from 0.5 milligrams to 5 grams of a compound of Formula 1 combined with an appropriate and convenient amount of a pharmaceutically-acceptable carrier which may vary from about 5 to about 95 percent of the total composition. Dosage unit forms will generally contain between from about 1 milligram to about 500 milligrams of an active compound of Formula 1, 3, or other ICE/ced-3 inhibitors.

It will be understood, however, that the specific "effective amount" for any particular patient will depend upon a variety of factors including the activity of the specific compound employed, the age, body weight, general health, sex, diet, time of administration, route of administration, rate of excretion, drug combination and the severity of the particular

disease undergoing prevention or therapy.

Although this invention focuses on the use of the compounds disclosed herein for inhibiting ICE/CED-3 proteases and for preventing apoptosis, the compounds of this invention can also be used as inhibitory agents for other cysteine proteases.

The compounds of this invention are also useful as commercial reagents which effectively bind to the ICE/ced-3 family of cysteine protease or other cysteine proteases. As commercial reagents, the compounds of this invention, and their derivatives, may be used to block proteolysis of a target peptide or may be derivatized to bind to a stable resin as a tethered substrate for affinity chromatography applications. These and other uses which characterize commercial cystine protease inhibitors will be evident to those of ordinary skill in the art.

The following examples are intended to illustrate, the invention. While they are typical of those that might be used, other procedures known to those skilled in the art may alternatively be used.

In the following Examples, proton NMR spectra were obtained at 300 MHZ; chemical shifts are quoted downfield from internal tetramethylsilane.

## EXAMPLE 1

### Assays for Inhibition of ICE/ced-3 Protease Family Activity

### A. Determination of $IC_{50}$ values

Fluorescence enzyme assays detecting the activity of the compounds of Formula 1 utilizing the recombinant ICE and cpp32 enzymes were performed essentially according to Thornberry *et al.* (Nature, 356:768:774 (1992)) and Nicholson *et al.* (Nature, 376:37-43 (1995)) respectively, (herein incorporated by reference) in 96 well microtiter plates. The substrate for these assays was Acetyl-Tyr-Val-Ala-Asp-amino-4-methylcoumarin (AMC) for the ICE assay and Acetyl-Asp-Glu-Val-Asp-amino-4-methylcoumarin for the cpp32 and Mch5 assay. Enzyme reactions were run in ICE buffer (25 mM HEPES, I mM EDTA, 0.1% CHAPS, 10% sucrose, pH 7.5) containing 2 mM DTT at room temperature in duplicate. The assays were performed by mixing the following components:

50 µl of either ICE, Mch2, Mch5 or cpp32 (18.8, 38,
8.1 and 0.153 nM concentrations, respectively) or Mch3 (1 unit) enzyme in ICE buffer containing either 8.0 (ICE, Mch2, Mch3, cpp32) or 20 (Mch5) mM DTT;
50 µl of either the compound of Formula 1 or
ICE buffer (control); and
100 µl of 20µM substrate.

The enzyme and the compound of Formula 1 to be assayed were preincubated in the microtitre plate wells for 30 minutes at room temperature prior to the addition of substrate to initiate the reaction. Fluorescent AMC product formation was monitored for one hour at room temperature by measuring the fluorescence emission at 460 nm using an excitation wavelength of 360 nm. The fluorescence change in duplicate (control) wells were averaged and the mean values were plotted as a function of inhibitor concentration to determine the inhibitor concentration producing 50% inhibition ($IC_{50}$). The results are set forth in Tables 1 and 4 (Figures 1 and 4).

The reference compound for this assay was Cbz-ValAlaAsp-H and the values are denoted in Tables 1 and 4 as "Reference".

### B. Determination of the dissociation constant $K_i$ and irreversible rate constant $k_3$ for irreversible inhibitors

For the irreversible inhibition of a ICE/ced-3 Family Protease enzyme with a competitive irreversible inhibitor; using the model represented by the following formulas:

$$E + I \overset{K_i}{\rightleftharpoons} EI \overset{k_3}{\dashrightarrow} E\text{-}I$$

$$E + S \overset{K_s}{\rightleftharpoons} ES \overset{k_s}{\dashrightarrow} E\text{+}P\ .$$

The product formation at time t may be expressed as:

$$[P]_t = [E]^T \left(\frac{[S]K_i}{[I]K_s}\right)\left(\frac{k_s}{k_3}\right)\left[1 - e^{-k_3 t/(1+\frac{K_i}{[I]}(1+\frac{[S]}{K_s}))}\right]$$

**Equation 1**

where E, I, EI, and E-I denote the active enzyme, inhibitor, non-covalent enzyme-inhibitor complex and covalent enzyme-inhibitor adduct, respectively. The $K_i$ value is the overall dissociation constant of reversible binding steps, and $k_3$ is the irreversible rate constant. The [S] and $K_s$ values are the substrate concentration and the dissociation constant of the substrate bound to the enzyme, respectively.

The above equations were used to determine the $K_i$ and $k_3$ values of a given inhibitor bound to a ICE/ced-3 family protease. Thus, a continuous assay was run for sixty minutes at various concentrations of the inhibitor and the substrate. The assay was formulated essentially the same as described above for generating the data in Table 1, except that the reaction was initiated by adding the enzyme to the substrate-inhibitor mixture. The Ki and $k_3$ values were obtained by simulating the product AMC formation as a function of time according to Equation I. The results of this second assay are set forth below in Tables 2, 3 and 5 (Figures 2, 3 and 5).

The reference compound for this assay was Cbz-ValAlaAsp-$CH_2F$ and the values are denoted in Tables 2, 3 and 5 as "Reference".

## EXAMPLE 76

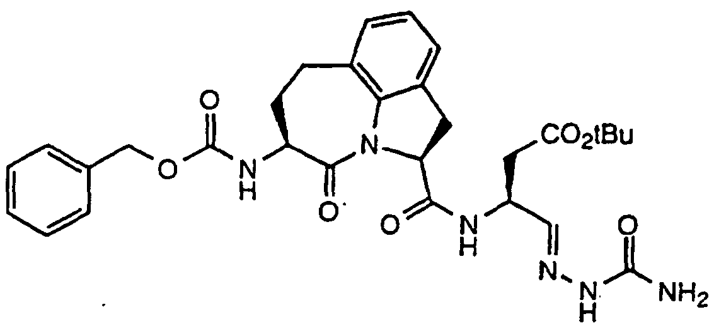

(2 S-cis)-[5-Benzyloxycarbonylamino-1,2,4,5,6,7-Hexahydro-4-Oxoazepino[3,2,1-hi]indole-2-Carbonyl)amino]-4-Oxo-butanoic Acid tert-Butyl Ester Semicarbazone

1. Preparation of (2S-cis)-5-Benzyloxycarbonylamino-1,2,4,5,6,7-Hexahydro-4-Oxoazepino[3,2,1-hi]indole-2-Carboxilic Acid, Ethyl Ester

To a solution of (2S-cis)-5-amino-1,2,4,5,6,7-hexahydro-4-oxoazepino[3,2,1-hi]indole-2-carboxylic acid, ethyl ester (0.437 g, 1.73 mmol, prepared as described in Tetrahedron Letters 36, pp. 1593-1596 (1995) and U.S. Patent 5,504,080 (April 2, 1996)) in methylene chloride (4 mL) stirring at 0°C was added benzyl chloroformate (0.370 mL, 2.6 mmol) and triethylamine (0.724 mL, 5.2 mmol) and the resulting mixture was stirred under nitrogen for 45 minutes. The reaction was quenched with water then partitioned between ethyl acetate and 5% aqueous potassium bisulfate solution. The aqueous layer was back-extracted two times with ethyl acetate, then the combined organic layers were washed with saturated sodium chloride solution, dried over sodium sulfate and evaporated to dryness. Purification of the crude product by flash chromatography on silica gel (S/P brand silica gel 60Å, 230-400 mesh ASTM) eluting with ethyl acetate-hexane (2:1) gave 0.558 g (68%) of crude product. Trituration with ethyl acetate-hexane (1:4) gave 0.480 g of the title compound as white solid; m.p.: 139-140°C. TLC (ethyl acetate-hexane, 2:1): Rf=0.6; $^1$H-NMR (300 MHz, $CDCl_3$): δ 7.35-7.30 (m, 5H), 7.02-6.94 (m, 3H), 6.17 (d, J=5.4 Hz, 1H), 4.15 (q, J=7.1 Hz, 2H), 3.46 (dd, J=11.0, 16.7

Hz, 1H), 3.29 (m, 1H), 3.10 (d, J=116.5, 2H), 2.35 (m, 1H), 2.16 (m, 1H), 1.23 (t, J=7.2 Hz, 3H).

2. Preparation of (2S-cis)-5-Benzyloxycarbonylamino-1,2,4,5,6,7-Hexahydro-4-Oxoazepino[3,2,1-hi]indole-2-Carboxylic Acid

To a solution of (2S-cis)-5-benzyloxycarbonylamino-1,2,4,5,6,7-hexahydro-4-oxoazepino[3,2,1-hi]indole-2-carboxylic acid, ethyl ester, (0.428g, 1.05 mmol) in 1,4-dioxane (7.5 mL) and water (2.5 mL) was added 1M aqueous lithium hydroxide (1.6 mL, 1.6 mmol) and the resulting mixture was stirred at room temperature under nitrogen for 30 minutes. The reaction mixture was acidified to pH 3 with a 5% aqueous potassium bisulfate sodium chloride solution. The aqueous layer was back-extracted two times with ethyl acetate, and the combined organic layers were dried over sodium sulfate and evaporated to dryness to yield 0.395 g (99%) of title compound as a fine white solid; m.p.: 188-189°C. TLC (methylene chloride-methanol-acetic acid, 9:1:1): Rf=0.55; $^1$H-NMR (300 MHz, $CDCl_3$) δ 7.34-7.26 (m, 5H), 7.07-6.97 (m, 3H), 6.08 (d, J=5.7 Hz, 1H), 5.25 (dd, J=3.2, 9.8 Hz, 1H), 5.10 (s, 2H), 4.30 (m, 1H), 3.36 (m, 1H), 3.26 (m, 2H), 3.06 (d, J=12.0 Hz, 1H), 2.36 (m, 1H), 2.09 (m, 1H).

3 Preparation of N-(Benzyloxycarbonyl)-L-(N'-Methyl-N'-Methoxy)aspartamide β-(tert-Butyl Ester)

To a solution of N-(benzyloxycarbonyl)-L-aspartic acid-β-(tert-butyl)ester (14.65 g, 45.3 mmol, Bachem) in $CH_2Cl_2$ (150 mL) at 0°C (ice bath) under a nitrogen atmosphere was added 1-hydroxybenzotriazole hydrate (7.29 g, 47.6 mmol, Aldrich) followed by 1-ethyl-3-(3',3'-dimethyl-1'-aminopropyl)carbodiimide hydrochloride (9.55 g, 49.8 mmol, Sigma). After stirring at 0°C for 15 min., N,O-dimethylhydroxylamine hydrochloride (5.10 g, 52.3 mmol, Aldrich) and N-methylmorpholine (5.8 mL, 53 mmol, Aldrich) were added. The mixture was allowed to warm to room temperature over 3 hours then stirred at room temperature for 16 hours. The solution was concentrated under vacuum and the residue partitioned between ethyl acetate-5% $KHSO_4$ (200 mL each). The organic phase was washed in turn with 5% $KHSO_4$, saturated sodium bicarbonate and saturated sodium chloride solutions; dried over anhydrous sodium sulfate and evaporated to an oil. The oil was crystallized from hexane to give the title product (16.10 g, 97% yield) as a fluffy white crystalline solid. TLC (ethyl acetate), single spot (UV and PMA): Rf=0.37.

A similar procedure to the one above, starting with 29.3 g of N-(benzyloxycarbonyl)-L-aspartic acid-β-(tert-butyl)ester (2-fold scale up) gave 31.18 g (94% yield) of the title product.

4. Preparation of N-(Benzyloxycarbonyl)-L-Aspartic Acid Semicarbazone β-(tert-Butyl)Ester

To a solution of N-(benzyloxycarbonyl)-L-(N'-methyl-N'-methoxy)aspartamide β-(tert-butyl ester) (15.50 g, 42.3 mmol) in anhydrous ether (400 mL) at 0°C (ice bath) under a nitrogen atmosphere was added dropwise to a 1.0 M solution of $LiAlH_4$ in ether (22.0 mL, 22.0 mmol, Aldrich) at such a rate as to keep the reaction solution temperature between 0-5°C (addition time 15-20 min). After the addition of the lithium aluminum hydride reagent was complete, the mixture was stirred at 0-5°C for 1 hr, then quenched by the dropwise addition of 0.3 N $KHSO_4$ solution (100 mL). The resultant mixture was transferred to a separatory funnel adding sufficient 5% $KHSO_4$ solution (75 mL) to dissolve the solids. The organic phase was separated and the combined aqueous washes back-extracted with ether (100 mL). The combined ether extracts were washed with saturated NaCl solution, dried over anhydrous sodium sulfate and concentrated in vacuo with minimal heating. TLC (ethyl acetate): streaky spot (UV and PMA) Rf=0.48. TLC (methanol/methylene chloride, 1:9) major spot (UV and PMA): Rf=0.75.

The crude aldehyde was immediately taken up in aqueous ethanol (45 mL water/105 mL alcohol), placed in an ice bath and treated with sodium acetate (3.82 g, 46.6 mmol) and semicarbazide hydrochloride (5.20 g, 46.6 mmol, Aldrich). The mixture was stirred at 0°C (ice bath) under a nitrogen atmosphere for 3 hrs, allowed to warm to room temperature, and stirred overnight (16 hrs). Most of the ethanol was removed under vacuum and the residue partitioned between ethyl acetate and water (100 mL each). The organic phase was washed sequentially with 5% $KHSO_4$, saturated sodium bicarbonate and saturated sodium chloride solutions; dried over anhydrous sodium sulfate and evaporated to dryness. The crude product of this reaction was combined with that of two similar procedures starting with 15.40 g and 4.625 g of N-(benzyloxycarbonyl)-L-(N'-methyl-N'-methoxy)aspartamide b-(tert-butyl ester) (total: 35.525 g, 97 mmol) and these combined products were purified by flash chromotagraphy on silica gel eluting with acetone/methylene chloride (3:7) then methanol-acetone-methylene chloride (0.5:3:7) to give pure title product (27.73 g, 78.5%) as a colorless foam. TLC (MeOH-$CH_2Cl_2$, 1:9): single spot (UV and PMA), Rf=0.51.

5 Preparation of L-Aspartic Acid Semicarbazone β-(tert-Butyl) Ester. p-Toluenesulfonate Salt

To a solution of N-(benzyloxycarbonyl)-L-aspartic acid semicarbazone β-(tert-butyl)ester (13.84 g, 38.0 mmol) in absolute ethanol (250 mL) was added 10% Pd/C (1.50 g, Aldrich) and the resulting mixture stirred under an atmosphere of hydrogen (balloon) until TLC (methanol/methylene chloride, 1:9) indicated complete consumption of the starting material (60 min). Note: It is important to follow this reaction closely since the product can be over-reduced. The mixture was filtered though Celite and evaporated to an oil. The oil was chased with methylene chloride (2 x 75mL) then with methylene chloride/toluene (1:1, 75 mL) to give the crude amine as a white crystalline solid. TLC (EtOAc-pyridine-AcOH-$H_2O$; 60:20:5:10) single spot (UV and PMA) Rf=0.24. Note: In this TLC system, any over-reduced product will show up immediately below the desired product, Rf=0.18 (PMA only).

The crude amine was taken up in $CH_3CN$ (60 mL) and treated with a solution of p-toluenesulfonic acid monohydrate (7.22 g, 38.0 mmol) in acetonitrile (60 mL). The crystalline precipitate was collected, washed with acetonitrile and ether, and air-dried to give the title compound (13.95 g, 92% yield) as a white, crystalline solid.

The optical purity of this material was checked by conversion to the corresponding Mosher amide [1.05 equiv (*R*)-(-)-α-methoxy-α-(trifluoromethyl)phenylacetyl chloride, 2.1 equivalents of i-$Pr_2$NEt in $CH_2Cl_2$, room temperature, 30 min]. The desired product has a doublet at 7.13 ppm (1H, d, J=2.4 Hz, CH=N) while the corresponding signal for its diastereomer is at 7.07 ppm. The optical purity of the title compound obtained from the above procedure is typically > 95:5.

6. (2S-cis)-[5-Benzyloxycarbonylamino-1,2,4,5,6,7-Hexahydro-4-Oxoazepino[3,2,1-hi]indole-2-Carbonyl)amino]-4-Oxo-butanoic Acid tert-Butyl Ester Semicarbazone

To a solution of (2S-cis)-5-benzyloxycarbonylamino-1,2,4,5,6,7-hexahydro-4-oxoazepino[3,2,1-hi]indole-2-carboxylic acid (0.375 g, 0.989 mmol) in methylene chloride (7 mL) stirring at 0°C under nitrogen was added 1-hydroxybenzotriazole hydrate (0.182 g, 1.19 mmol) and 1-ethyl-3-(3',3'-dimethyl-1'-aminopropyl)carbodiimide hydrochloride (0.284 g, 1.48 mmol). After 15 minutes L-aspartic acid semicarbazone b-(tert-butyl) ester, p-toluenesulfonate salt (0.386 g, 0.989 mmol) and N-methylmorpholine (0.163 mL, 1.48 mmol) were added and the resultant reaction mixture allowed to come to room temperature within 1 hour. After stirring overnight, the reaction mixture was diluted with ethyl acetate and washed successively with 5% potassium bisulfate and saturated sodium chloride solutions; dried over sodium sulfate and evaporated to dryness. Purification of the crude product by flash chromatography on silica gel (S/P brand silica gel 60Å, 230-400 mesh ASTM) eluting with 2% methanol-methylene chloride gave 0.463 g (79%) of the title compound as a white foam. TLC (methylene chloride-methanol, 9:1): Rf=0.5. $^1$H-NMR (300 MHz, $CDCl_3$): δ 8.42 (s, 1H), 7.82 (d, J=8.1 Hz, 1H), 7.32 (m, 5H), 7.07 (m, 3H), 5.94 (d, J=6.3 Hz, 1H), 5.26 (d, J=9 Hz, 1H), 5.10 (s, 2H), 4.82 (m, 1H), 4.35 (m, 1H), 3.56 (d, J=18 Hz, 1H), 3.27 (m, 2H), 3.07 (m, 1H), 2.64 (dd, J=4.7, 15.8 Hz, 1H), 2.44 (dd, J=6.6, 15.9 Hz, 2H), 2.22 (m, 1H), 1.30 (s, 9H). Mass spectrum: m/z 593 (M+H).

## EXAMPLE 77

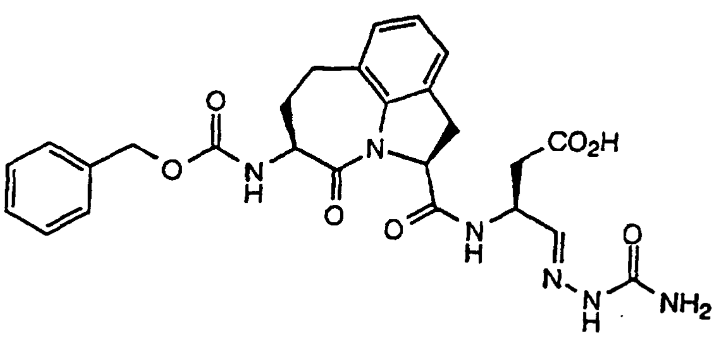

(2-cis)-[5-Benzyloxycarbonylamino-1,2,4,5,6,7-hexadydro-4-oxoazepino[3,2,1-hi]indole-2-carbonyl)-amino]-4-oxo-butanoic acid semicarbazone

To a solution of (2S-cis)-[5-benzyloxycarbonylamino-1,2,4,5,6,7-bexahydro-4-oxoazepino[3,2,1-hi]indole-2-carbonyl)amino]-4-oxo-butanoic acid tert-butyl ester semicarbazone (0.214 g, 0.362 mmol) in methylene chloride (1.5 mL) was added anisole (0.5 mL, 4.34 mmol) followed by trifluoroacetic acid (0.75 mL). After stirring at room temperature under nitrogen for 2 hours the reaction mixture was diluted with methylene chloride and evaporated, then chased twice with methylene chloride to give the title compound (0.195 g). TLC (methylene chloride-methanol, 95:5),

Rf=0.2. $^1$H-NMR (300 MHz, $CDCl_3$) δ 9.77 (bs, 1H), 8.32 (d, J=12 Hz, 1H), 8.12 (d, J-7.8 Hz, 1H), 7.31-7.27 (m, 5H) 7.13-7.04 (m, 3H), 6.64 (m, 1H) 5.32 (d, J=9.9 Hz, 1H), 5.12 (s, 2H), 4.86 (m, 1H), 4.41 (m, 1H), 3.56 (d, J=15 Hz, 1H), 3.25 (m, 2H), 3.10 (m, 2H), 2.64 (m, 2H), 2.28 (m, 2H).

## EXAMPLE 78

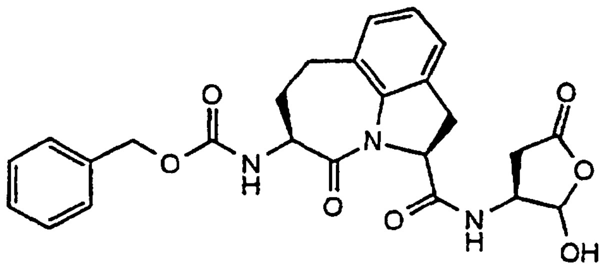

(2S-cis)-5-[Benzyloxycarbonylamino-1,2,4,5,6,7-hexahydro-4-oxoazepino[3,2,1-hi]indole-2-carbonyl)-amino]-4-oxo-butanoic acid

(2-cis)-[5-Benzyloxycarbonylamino-1,2,4,5,6,7-hexadydro-4-oxoazepino[3,2,1-hi]indole-2-carbonyl)-amino]-4-oxo-butanoic acid semicarbazone (0.195 g, 0.36 mmol) was treated with a 3:1:1 solution of methanol-acetic acid-37% formaldehyde (2 mL) and the resulting mixture stirred under nitrogen for 1.5 hours. The reaction mixture was diluted with water, methanol removed by evaporation, then the remaining mixture lyophilized. Purification of the crude product by flash chromatography on reverse phase gel (MCI gel, CHP-20P, 75-150 micron)eluting with a 10%-80% methanol-water gradient gave 0.073 g, (42%) of the title compound as a white solid after lyophilization; m.p. 101-104°C. TLC (methylene chloride-methanol-acetic acid, 97:2.5:0.5) Rf=0.45. $^1$H-NMR (300 MHz, $CDCl_3$) δ 7.45 (m, 1H), 7.30 (s, 5H), 7.07 (d, J=3.3 Hz, 1H), 7.00 (d, J=4.8 Hz, 2H), 6.12 (m, 1H), 5.17 (d, J=9.6 Hz, 1H), 5.07 (s, 2H), 4.49 (m, 1H), 4.28 (m, 1H), 3.46 (d, J=9.9 Hz, 1H), 3.30-3.12 (m, 2H), 3.04-2.99 (m, 1H), 2.83-2.76 (m, 1H), 2.46-2.33 (m, 2H), 2.03 (bs, 1H). Mass spectrum: m/z 480 (M+H).

## EXAMPLE 79

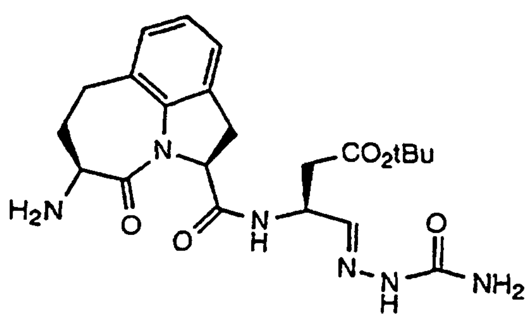

(2S-cis)-[5-Amino-1,2,3,4,5,6,7-hexahydro-4-oxoazepino[3,2,1-hi]indole-2-carbonyl])-amino]-4-oxo-butanoic acid tert-buly] ester semicarbazone

10% Palladium on carbon (0.180 g) was added to a solution of (2S-cis)-[5-benzyloxycarbonylamino-1,2,3,4,5,6,7-hexahydro-4-oxoazepino[3,2,1-hi]indole-2-carbonyl)-amino]-4-oxo-butanoic acid tert-butyl ester semicarbazone (0.308 g, 0.520 mmol) in methanol (27 mL) and the resulting mixture was hydrogenated using a balloon of hydrogen (1 atm, R.T.) for 18 hours. The mixture was filtered through Celite, evaporated to dryness, then chased two

times with toluene to give the title compound as an off-white solid (0.215 g). TLC (methylene chloride-methanol, 9:1) Rf=0.15. $^1$H-NMR (300 MHz, $CDCl_3$) δ 8.53 (s, 1H), 7.89 (d, J=7.6 Hz, 1H), 7.13 (m, 3H), 5.21 (dd, J=2.3, 10.14 Hz, 1H), 4.82 (m, 1H), 3.52 (m, 1H), 3.24 (dd, J=10.3, 16.3 Hz, 1H), 3.03 (m, 2H), 2.62 & 2.42 (AB, dd, J=4.2, 7.1, 15.7 Hz, 2H), 2.19 (m, 1H), 1.32 (s, 9H).

## EXAMPLE 80

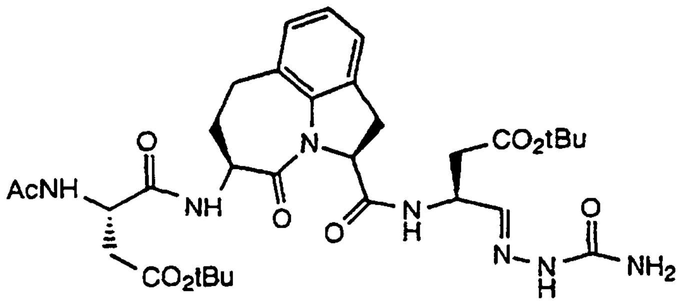

(2S-cis)-[5-(N-Acetyl-(S)-aspartyl-b-tert-butyl ester)amino-1,2,3,4,5,6,7-hexahydro-4-oxoazepino[3,2,1-hi]indole-2-carbonyl)-amino]-4-oxo-butanoic acid tert-butyl ester semicarbazone

To a solution of N-acetyl aspartic acid, β-tert-butyl ester (0.120 g, 0.517 mmol) in methylene chloride (1.5 mL) stirring at 0°C under nitrogen was added 1-hydroxybenzotriazole hydrate (0.086 g, 0.564 mmol) and 1-(3-dimethylaminopropyl)-3-ethylcarbodiimide hydrochloride (0.135 g, 0.705 mmol). After 15 minutes, a solution of (25-cis)-[5-amino-1,2,3,4,5,6,7-hexahydro-4-oxoazepino[3,2,1-hi]indole-2-carbonyl)-amino]-4-oxo-butanoic acid tert-butyl ester semicarbazone (0.213 g, 0.47 mmol) in methylene chloride (2 mL) was added and the reaction was allowed to come to room temperature over 1 hour. After stirring overnight, the reaction mixture was diluted with ethyl acetate and washed successively with 5% potassium bisulfate and saturated sodium chloride solutions; dried (sodium sulfate) and evaporated to dryness. Purification of the crude product by flash chromatography on silica gel (S/P brand silica gel 60Å, 230-400 mesh ASTM) eluting with 5% then 10% methanol-methylene chloride gave 0.126 g (41%) of the title compound as a white solid. TLC (methylene chloride-methanol, 9:1) Rf=0.4. $^1$H-NMR (300 MHz, $CDCl_3$) δ 9.63 (s, 1H), 8.32 (d, J=7.8 Hz, 1H), 7.82 (d, J=.6.6 Hz, 1H), 7.53 (d, J=4.8 Hz, 1H), 7.09 (m, 1H), 7.00 (m, 2H), 5.18 (d, J= 8.1 Hz, 1H), 4.86 (m, 1H), 4.39 (m, 1H), 3.01 (m, 1H), 2.92 (dd, J= 4.2, 14.7 Hz, 1H) 2.68 (d, J=12.3 Hz, 1H), 2.52 (m, 2H), 2.51 (m, 2H), 2.03 (s,3H), 1.39 (s,.9H), 1.24 (s, 9H).

## EXAMPLE 81

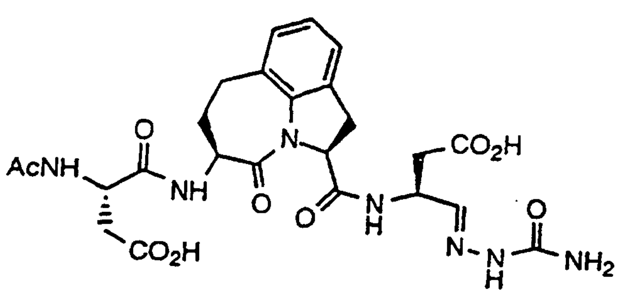

(2S-cis)-[5-(N-Acetyl-(S)-aspartyl)amino-1,2,3,4,5,6,7-hexahydro-4-oxoazepino[3,2,1-hi]indole-2-carbonyl)-amino]-4-oxo-butanoic acid semicarbazone

To a solution of (2S-cis)-[5-(N-acetyl-(S)-aspartyl-b-tert-butyl ester)amino-1,2,3,4,5,6,7-hexahydro-4-oxoazepino[3,2,1-hi]indole-2-carbonyl)-amino]-4-oxo-butanoic acid tert-butyl ester semicarbazone (0.117 g, 0.178 mmol) in methylene chloride (1 mL) was added anisole (0.5 mL) followed by trifluoroacetic acid (1 mL). After stirring at room temperature under nitrogen for 2 hours, the reaction mixture was diluted with methylene chloride and evaporated, then chased twice with methylene chloride to give the title compound (0.099 g). TLC (methylene chloride-methanol-acetic acid, 13:6:1 ) Rf=0.2. Mass spectrum: m/z 560 (M+H).

## EXAMPLE 82

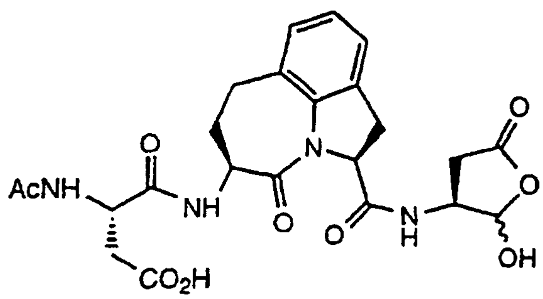

(2S-cis)-[5-(N-Acetyl-(S)-aspartyl)amino-1,2,3,4,5,6,7-hexahydro-4-oxoazepino[3,2,1-hi]indole-2-carbonyl)-amino]-4-oxo-butanoic acid

(2S-cis)-[5-(N-Acetyl-(S)-aspartyl)amino-1,2,3,4,5,6,7-hexahydro-4-oxoazepino[3,2,1-hi]indole-2-carbonyl)-amino]-4-oxo-butanoic acid semicarbazone (0.097 g, 0.177 mmol). was treated with a 3:1:1 solution of methanol-acetic acid-37% formaldehyde (2 mL) and the resulting mixture stirred under nitrogen for 1.5 hours. The reaction mixture was then diluted with water, methanol removed by evaporation, then the remaining mixture lyophilized. Purification of the crude product by flash chromatography on reverse phase gel (MCI gel, CHP-20P, 75-150 micron) eluting with a 10%-80% methanol-water gradient gave 0.050g (56%) of the title compound as a white solid after lyophilization; m.p. 160-175°C (dec). TLC (methylene chloride-methanol-acetic acid, 13:6:1) Rf=0.3. Mass spectrum: m/z 503 (M+H).

## EXAMPLE 83

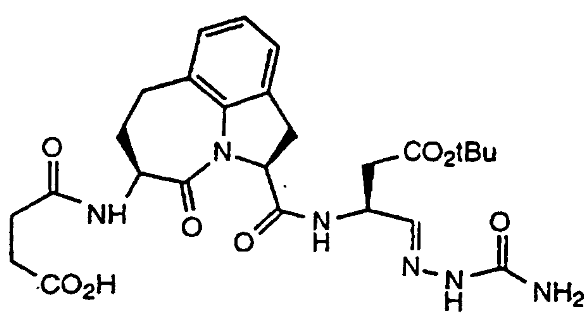

(2S-cis)-[5-Succinylamino-1,2,3,4,5,6,7-hexahydro-4-oxoazepino[3,2,1-hi]indole-2-carbonyl)-amino]-4-oxo-butanoic acid tert-butyl ester semicarbazone

To a solution of (2S-cis)-[5-amino-1,2,3,4,5,6,7-hexahydro-4-oxoazepino[3,2,1-hi]indole-2-carbonyl)-amino]-

4-oxo-butanoic acid tert-butyl ester semicarbazone (0.197 g, 0.435 mmol) in methylene chloride (6 mL) stirring at 0°C under nitrogen was added succinic anhydride (0.057 g, 0.566 mmol), followed by pyridine (0.052 mL, 0.653 mmol). After stirring at room temperature under nitrogen for 3 hours, the reaction mixture was diluted with ethyl acetate and washed successively with 5% potassium bisulfate and saturated sodium chloride solutions; dried (sodium sulfate) and evaporated to dryness. Purification of the crude product by flash chromatography on silica gel (S/P brand silica gel 60Å, 230-400 mesh ASTM) eluting with 10% methanol-methylene chloride then 80:19:1 methylene chloride-methanol-acetic acid gave 0.216 g (88%) of the title compound as a white solid. TLC (methylene chloride-methanol-acetic acid, 8:1:1) Rf=0.5. Mass spectrum: m/z 557 (M-H).

## EXAMPLE 84

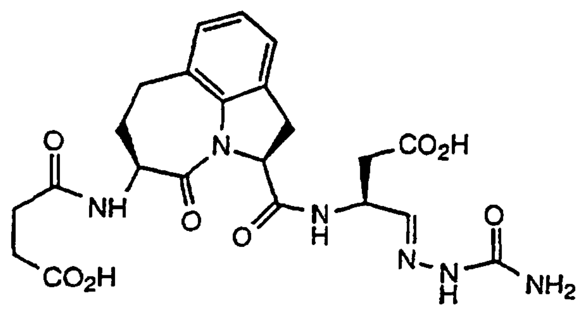

(2S-cis)-[5-Succinylamino-1,2,3,4,5,6,7-hexahydro-4-oxoazepino[3,2,1-hi]indole-2-carbonyl)-amino]-4-oxo-butanoic acid semicarbazone

To a solution of (2S-cis)-[5-succinylamino-1,2,3,4,5,6,7-hexahydro-4-exoazepino[3,2,1-hi]indole-2-carbonyl)-amiuno]-4-oxo-butanoic acid tert-butyl ester semicarbazone (0.191g, 0.342 mmol) in methylene chloride (1 mL) was added anisole (0.5 mL) followed by trifluoroacetic acid (1 mL). After stirring at room temperature under nitrogen for 2 hours, the reaction mixture was diluted with methylene chloride and evaporated, then chased twice with methylene chloride to give the title compound (0.210 g). TLC (methylene chloride-methanol-acetic acid, 8:1:1) Rf=0.4. Mass spectrum: m/z 503 (M+H).

## EXAMPLE 85

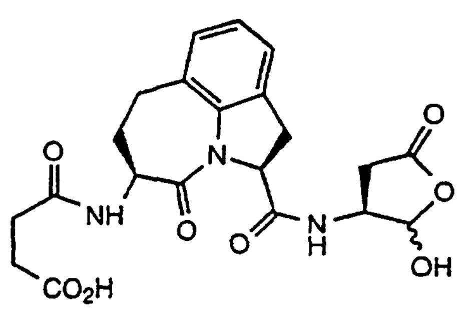

(2S-cis)-[5-Succinylamino-1,2,3,4,5,6,7-hexahydro-4-oxoazepino[3,2,1-hi]indole-2-carbonyl)-amino]-4-oxo-butanoic acid

(2S-cis)-[5-Succinylamino-1,2,3,4,5,6,7-hexahydro-4-oxoazepino[3,2,1-hi]indole-2-carbonyl)-amino]-4-oxo-butanoic acid semicarbazone (0.208 g, ca. 0.342 mmol), was treated with a 3:1:1 solution of methanol-acetic acid-37% formaldehyde (3 mL), and the resulting mixture stirred under nitrogen for 1.5 hours. The reaction mixture was then diluted with water, methanol removed by evaporation, then the remaining mixture lyophilized. Purification of the crude product by flash chromatography on reverse phase gel (MCI gel, CHP-20P, 75-150 micron) eluting with a 10%-80% methanol-water gradient gave 0.064 g (42%) of the title compound as a white solid after lyophilization; m.p. 145-160°C (dec). TLC (methylene chloride-methanol-acetic acid, 8:1:1) Rf=0.45. Mass spectrum: m/z 446 (M+H).

## EXAMPLE 86

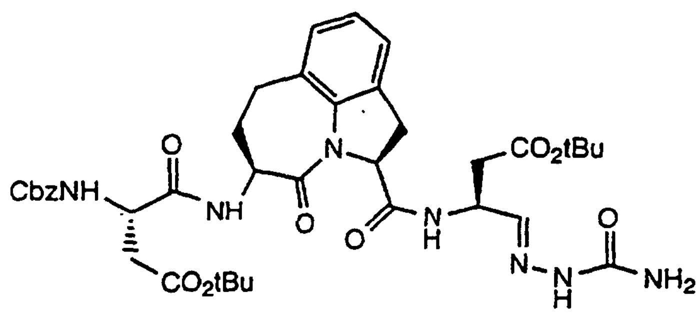

(2S-cis)-[5-(-N-Benzyloxycarbonyl-(S)-aspartyl-b-tert-butyl ester)amino-1,2,3,4,5,6,7-hexahydro-4-oxoazepino [3,2,1-hi]indole-2-carbonyl)-aminp]-4-oxo-butanoic acid tert-butyl ester semicarbazone

To a solution of N-benzyloxycarbonyl-(S)-aspartyl-β-tert-butyl ester (0.169 g, 0.521 mmol) in methylene chloride (1.5 mL) stirring at 0°C under nitrogen was added 1-hydroxybenzotriazole hydrate (0.087 g, 0.569 mmol) and 1-(3-dimethylaminopropyl)-3-ethylcarbodiimide hydrochloride (0.136 g, 0.711 mmol). After 15 minutes, a solution of (2S-cis)-[5-amino-1,2,3,4,5,6,7-hexahydro-4-oxoazepino[3,2,1-hi]indole-2-carbonyl)-amino]-4-oxo-butanoic acid tert-butyl ester semicarbazone (0.217 g, 0.474 mmol) in methylene chloride (2 mL) was added and the reaction was allowed to come to room temperature within 1 hour. After stirring overnight, the reaction mixture was diluted with ethyl acetate and washed successively with 5% potassium bisulfate and saturated sodium chloride solutions; dried (sodium sulfate) and evaporated to dryness. Purification of the crude product by flash chromatography on silica gel (S/P brand silica gel 60Å, 230-400 mesh ASTM) eluting with 2% then 5% methanol-methylene chloride gave 0.244 g (67%) of the title compound as an off-white solid. TLC (methylene chloride-methanol, 9:1) Rf=0.55. $^1$H-NMR (300 MHz, $CDCl_3$) δ 9.13 (s, 1H), 7.85 (d, J= 6 Hz, 1H), 7.56 (d, J= 5.7 Hz, 1H) 7.23 (m, 5H), 7.08 (m, 1H), 7.00 (m, 2H), 5.13 (m, 3H) 4.77 (m, 1H), 4.62 (m, 1H), 4.43 (m, 1H), 3.60 (d, J= 16 Hz, 1H), 3.22 (m, 2H), 2.98 (m, 1H), 2.83 (d, J= 15.3 Hz, 1H), 2.65 & 2.36 (AB, dd, J= 4.2, 7.7, 16.9 Hz, 2H), 2.42 (m,1H), 2.10 (m, 1H), 1.35 (s, 9H), 1.24 (s, 9H).

## EXAMPLE 87

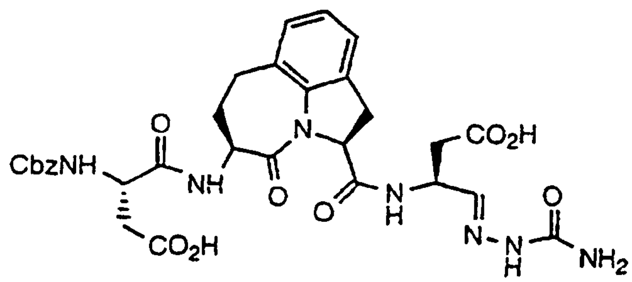

(2S-cis)-[5-(N-Benzyloxycarbonyl-(S)-aspartyl)amino-1,2,3,4,5,6,7-hexahydro-4-oxoazepino[3,2,1-hi]indole-2-carbonyl)-amino]-4-oxo-butanoic acid semicarbazone

To a solution of (2S-cis)-[5-(N-Benzyloxycarbonyl-(S)-aspartyl-b-tert-butyl ester)amino-1,2,3,4,5,6,7-hexahydro-4-oxoazepino[3,2,1-hi]indole-2-carbonyl)-amino]-4-oxobutanoic acid tert-butyl ester semicarbazone (0.217 g, 0.289 mmol) in methylene chloride (1 mL) was added anisole (0.5 mL) followed by trifluoroacetic acid (1 mL). After stirring at room temperature under nitrogen for 3 hours, the reaction mixture was diluted with methylene chloride and evaporated, then chased twice with methylene chloride to give the title compound (0.193 g). TLC (methylene chloride-methanol, 9:1) Rf=0.35. Mass spectrum: m/z 652 (M+H).

## EXAMPLE 88

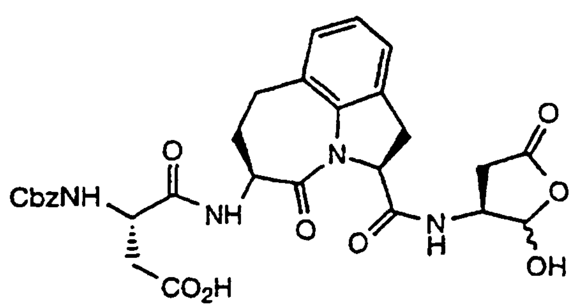

(2S-cis)-[5-(N-Benzyloxycarbonyl-(S)-aspartyl)amino-1,2,3,4,5,6,7-hexahydro-4-oxoazepino[3,2,1-hi]indole-2-carbonyl)-amino]-4-oxo-butanoic acid

(2S-cis)-[5-(N-Benzyloxycarbonyl-(S)-aspartyl)amino-1,2,3,4,5,6,7-hexahydro-4-oxoazepino[3,2,1-hi]indole-2-carbonyl)-amino)-4-oxo-butanoic acid semicarbazone (0.191 g, 0.29 mmol), was treated with a 3:1:1 solution of methanol-acetic acid-37% formaldehyde (2 mL) and the resulting mixture stirred under nitrogen for 2 hours. The reaction mixture was then diluted with water, methanol removed by evaporation, then the remaining mixture lyophilized. Purification of the crude product by flash chromatography on reverse phase gel (MCI gel, CHP-20P, 75-150 micron) eluting with a 10%-80% methanol-water gradient gave 0.111 g. (64%) of the title compound as a white solid after lyophilization; m.p. 140-144°C (dec.). TLC (methylene chloride-methanol, 9:1) Rf=0.4. Mass spectrum: m/z 593 (M-H).

## EXAMPLE 89

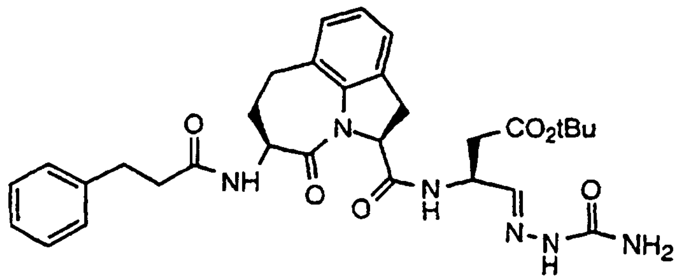

(2S-cis)-[5-Dihydrocinnamylamino-1,2,3,4,5,6,7-hexahydro-4-oxoazepino[3,2,1-hi]indole-2-carbonyl)-amino]-4-oxo-butanoic acid tert-butyl ester semicarbazone

To a solution of dihydrocinnamic acid (0.169 g, 0.521 mmol) in methylene chloride (1.5 mL) stirring at 0°C under nitrogen was added 1-hydroxybenzotriazole hydrate (0.088 g, 0.576 mmol) and 1-(3-dimethylaminopropyl)-3-ethylcarbodiimide hydrochloride (0.127 g, 0.665 mmol). After 15 minutes, a solution of (2S-cis)-[5-amino-1,2,3,4,5,6,7-hexahydro-4-oxoazepino[3,2,1-hi]indole-2-carbonyl)-amino]-4-oxo-butanoic acid tert-butyl ester semicarbazone (0.203 g, 0.443 mmol) in methylene chloride (2 mL), was added and the reaction was allowed to come to room temperature within 1 hour. After stirring overnight, the reaction mixture was diluted with ethyl acetate and washed successively with 5% potassium bisulfate and saturated sodium chloride solutions; dried (sodium sulfate) and evaporated to dryness. Purification of the crude product by flash chromatography on silica gel (S/P brand silica gel 60A, 230-400 mesh ASTM) eluting with 2 then 5% methanol-methylene chloride gave 0.208 g (79%) of the title compound as an off-white solid. TLC (methylene chloride-methanol, 9:1) Rf=0.7. $^1$H-NMR (300 MHz, $CDCl_3$) δ 8.82 (s, 1H), 7.72 (d, J= 8.1 Hz, 1H), 7.19 (m, 5H), 7.06 (m, 1H), 7.01 (m, 2H), 6.76 (d, J=6.3, 1H), 5.23 (d, J= 8.4 Hz, 1H0, 4.84 (m, 1H), 4.50 (m, 1H), 3.48 (m, 1H), 3.26 (m, 2H), 3.05 (m, 1H), 2.94 (m, 2H), 2.53 (m,4H), 2.28 (m, 1H), 2.06 (m, 1H), 1.29 (s, 9H). Mass spectrum: m/z 591 (M+H).

## EXAMPLE 90

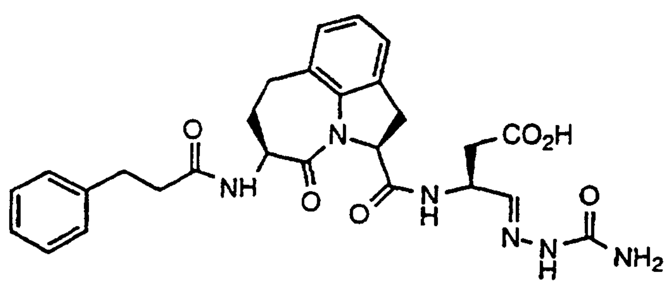

(2S-cis)-[5-Dihydrocinnamylamino-1,2,3,4,5,6,7-hexahydro-4-oxoazepino[3,2,1-hi]indole-2-carbonyl)-amino]-4-oxo-butanoic acid semicarbazone

To a solution of (2S-cis)-[5-dihydrocinnamylamino-1,2,3,4,5,6,7-hexahydro-4-oxoazepino[3,2,1-hi]indole-2-carbonyl)-amino]-4-oxo-butanoic acid tert-butyl ester semicarbazone (0.189 g, 0.320 mmol) in methylene chloride (1 mL) was added anisole (0.5 mL) followed by trifluoroacetic acid (1 mL). After stirring at room temperature under nitrogen for 3 hours, the reaction mixture was diluted with methylene chloride and evaporated, then chased twice with methylene chloride to give the title compound (0.183 g). TLC(methylene chloride-methanol, 9:1) Rf=0.25. Mass spectrum: m/z 535 (M+H).

## EXAMPLE 91

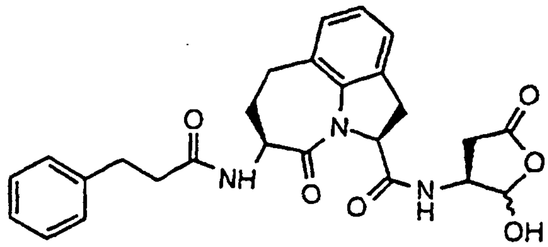

(2S-cis)-[5-Dihydrocinnamylamino-1,2,3,4,5,6,7-hexahydro-4-oxoazepino[3,2,1-hi]indole-2-carbonyl)-amino]-4-oxo-butanoic acid

(2S-cis)-[5-Dihydrocinnamylamino-1,2,3,4,5,6,7-hexahydro-4-oxoazepino[3,2,1-hi]indole-2-carbonyl)-amino]-4-oxo-butanoic acid semicarbazone (0.181 g, ca. 0.320 mmol), was treated with a 3:1:1 solution of methanol-acetic acid-37% formaldehyde (2 mL) and the resulting mixture stirred under nitrogen for 4 hours. The reaction mixture was then diluted with water, methanol removed by evaporation, then the remaining mixture lyophilized. Purification of the crude product by flash chromatography on reverse phase gel (MCI gel, CHP-20P, 75-150 micron) eluting with a 10%-80% methanol-water gradient gave 0.075 g (47%) of the title compound as a white solid after lyophilization; m.p. 78-81°C. TLC (methylene chloride-methanol, 9:1) Rf=0.45. $^1$H-NMR (300 MHz, DMSO d6): δ 8.58 (m, 1H), 8.30 (d, J= 7.5 Hz, 1H), 7.24 (m, 5H), 7.08 (m, 2H), 6.99 (m, 1H), 5.04 (d, J=9.3 Hz, 1H), 4.39 (m, 1H), 4.19 (m, 1H), 3.46 (m, 1H), 3.05 (m, 2H), 2.93 (d, J= 16.8 Hz, 2H), 2.83 (m, 2H), 2.00 (d, J= 5.1 Hz, 2H). Mass spectrum: m/z 478 (M+H).

## EXAMPLE 92

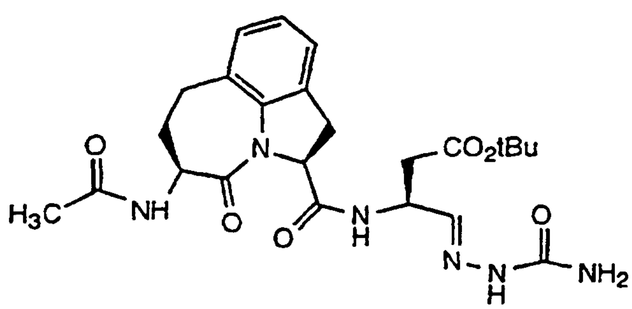

(2S-cis)-[5-Acetylamino-1,2,3,4,5,6,7-hexahydro-4-oxoazepino[3,2,1-hi]indole-2-carbonyl)-amino]-4-oxo-butanoic acid tert-butyl ester semicarbazone

To a solution of (2S-cis)-[5-amino-1,2,3,4,5,6,7-hexahydro-4-oxoazepino[3,2,1-hi]indole-2-carbonyl)-amino]-4-oxo-butanoic acid tert-butyl ester semicarbazone (0.222 g, 0.490 mmol) in pyridine (3 mL) at room temperature under nitrogen was added acetic anhydride (0.07 mL, 0.735 mmol). After stirring overnight, the reaction mixture was diluted with methylene chloride and evaporated to give a foam. This was taken up in ethyl acetate and washed successively with 5% potassium bisulfate and saturated sodium chloride solutions; dried (sodium sulfate) and evaporated to dryness to give 0.130 g (53%) of the title compound as an off-white solid. TLC (methylene chloride-methanol, 9:1) Rf=0.55. $^1$H-NMR (300 MHz, $CDCl_3$): δ 8.75 (s, 1H), 7.75 (d, J= 8.4 Hz, 1H), 7.08 (m, 1H), 7.01 (m, 2H), 6.87 (d, J=6.3 Hz, 1H), 5.25 (d, J= 8.1 Hz, 1H), 4.84 (m, 1H), 4.52 (m, 1H), 3.50 (m, 1H), 3.28 (m, 2H), 3.02 (m, 1H), 2.55 & 2.46 (AB, dd, J= 4.2, 7.1, 15.7 Hz, 2H), 2.36 (m, 1H), 2.18 (m, 1H), 2.02 (s, 3H), 1.31 (s, 9H).

## EXAMPLE 93

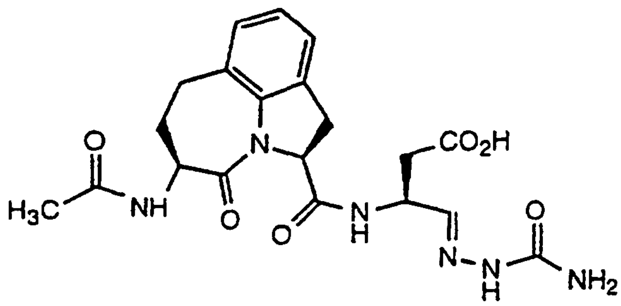

(2S-cis)-[5-Acetylamino-1,2,3,4,5,6,7-hexahydro-4-oxoazepino[3,2,1-hi]indole-2-carbonyl)-amino]-4-oxo-butanoic acid semicarbazone

To a solution of (2S-cis)-[5-acetylamino-1,2,3,4,5,6,7-hexahydro-4-oxoazepino[3,2,1-hi]indole-2-carbonyl)-amino]-4-oxo-butanoic acid tert-butyl ester semicarbazone (0.112 g, 0.224 mmol) in methylene chloride (1 mL) was added anisole (0.5 mL) followed by trifluoroacetic acid (1 mL). After stirring at room temperature under nitrogen for 2.5 hours, the reaction mixture was diluted with methylene chloride and evaporated, then chased twice with methylene chloride to give the title compound (0.117 g). TLC (methylene chloride-methanol, 9:1) Rf=0.15. Mass spectrum: m/z 445 (M+H).

## EXAMPLE 94

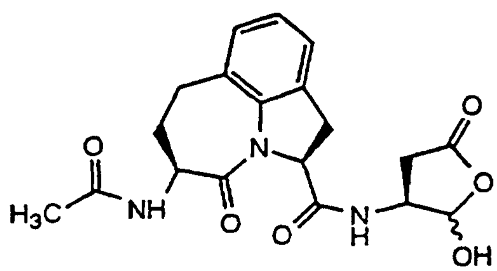

(2S-cis)-[5-Acetylamino-1,2,3,4,5,6,7-hexahydro-4-oxoazepino[3,2,1-hi]indole-2-carbonyl)-amino]-4-oxo-butanoic acid

(25-cis)-[5-Acetylamino-1,2,3,4,5,6,7-hexahydro-4-oxoazepino[3,2,1-hi]indole-2-carbonyl)-amino]-4-oxo-butanoic acid semicarbazone (0.115 g, ca. 0.224 mmol) was treated with a 3:1:1 solution of methanol-acetic acid-37% formaldehyde (2 mL) and the resulting mixture stirred under nitrogen for 5 hours. The reaction mixture was diluted with water, methanol removed by evaporation, then the remaining mixture lyophilized. Purification of the crude product by flash chromatography on reverse phase gel (MCI gel, CHP-20P, 75-150 micron) eluting with a 10%-80% methanol-water gradient gave 0.044 g (51 %) of the title compound as a white solid after lyophilization; m.p. 210-215°C (dec). TLC (methylene chloride-methanol-acetic acid, 44:5:1) Rf=0.45. Mass spectrum: m/z 388 (M+H).

## EXAMPLE 95

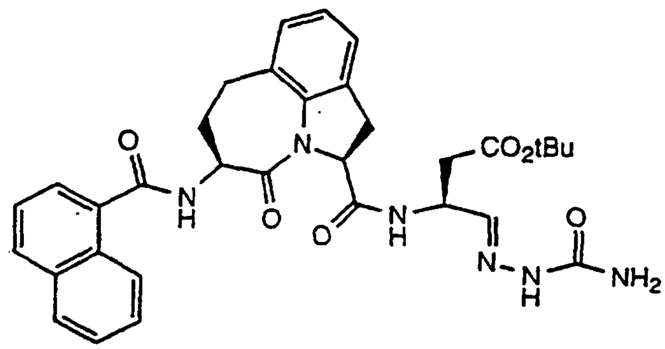

(2S-cis)-[5-(1-Naphthoyl)amino-1,2,3,4,5,6,7-hexahydro-4-oxoazepino[3,2,1-hi]indole-2-carbonyl)-amino]-4-oxo-butanoic acid tert-butyl ester semicarbazone

To a solution of 1-naphthoic acid (0.072 g, 0.417 mmol) in methylene chloride (1.5 mL) stirring at 0°C under nitrogen was added 1-hydroxybenzotriazole hydrate (0.077 g, 0.501 mmol.) and 1-(3-dimethylaminopropyl)-3-ethylcarbodiimide hydrochloride (0.120 g, 0.626 mmol). After 15 minutes, a solution of (2S-cis)-[5-amino-1,2,3,4,5,6,7-hexahydro-4-oxoazepino[3,2,1-hi]indole-2-carbonyl)-amino]-4-oxo-butanoic acid tert-butyl ester semicarbazone (0.189 g, 0.147 mmol) in methylene chloride (2 mL), was added and the reaction was allowed to come to room temperature within 1 hour. After stirring a total of 5 hours, the reaction mixture was diluted with ethyl acetate and washed successively with 5% potassium bisulfate and saturated sodium chloride solutions, dried (sodium sulfate) and evaporated to dryness. Purification of the crude product by flash chromatography on silica gel (S/P brand silica gel 60Å, 230-400 mesh ASTM) eluting with 5% methanol-methylene chloride gave 0.168 g (66%) of the title compound as an off-white solid; m.p. 103-105°C (dec.). TLC (methylene chloride-methanol, 9:1) Rf=0.6. Mass spectrum: m/z 613 (M+H). $^1$H-NMR (300 MHz, $CDCl_3$) δ 9.09 (bs, 1H), 8.38 (d, J=8.4 Hz, 1H), 7.82-7.93 (m, 3H), 7.70 (d, J=6.3 Hz, 1H), 7.45-7.58 (m, 3H), 7.37 (d, J=6.6 Hz, 1H), 7.06-7.15 (m, 4H), 5.30 (d, J= 8.4 Hz, 1H), 4.80-4.85 (m, 2H), 3.57 (d, J= 3.6 Hz, 1H), 3.30-3.45 (m, 2H), 3.16 (m, 1H), 2.59-2.65 (m, 2H), 2.27-2.49 (m, 2H), 1.29 (s, 9H).

## EXAMPLE 96

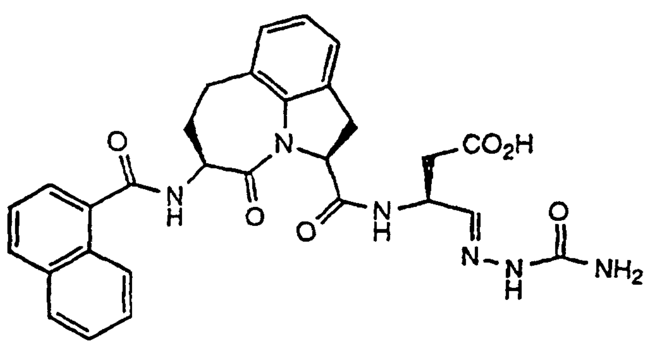

(2S-cis)-[5-(1-Naphthoyl)amino-1,2,3,4,5,6,7-hexahydro-4-oxoazepino[3,2,1-hi]indole-2-carbonyl)-amino]-4-oxo-butanoic acid semicarbazone

To a solution of (2S-cis)-[5-(1-naphthoyl)amino-1,2,3,4,5,6,7-hexahydro-4-oxoazepino[3,2,1-hi]indole-2-carbonyl)-amino]-4-oxo-butanoic acid tert-butyl ester semicarbazone (0.106 g, 0.173 mmol) in methylene chloride (1 mL) was added anisole (0.5 mL) followed by trifluoroacetic acid (1 mL). After stirring at room temperature under nitrogen for 3 hours, the reaction mixture was diluted with methylene chloride and evaporated, then chased twice with methylene

chloride to give the title compound (0.110 g). TLC (methylene chloride-methanol, 9:1) Rf=0.3.

## EXAMPLE 97

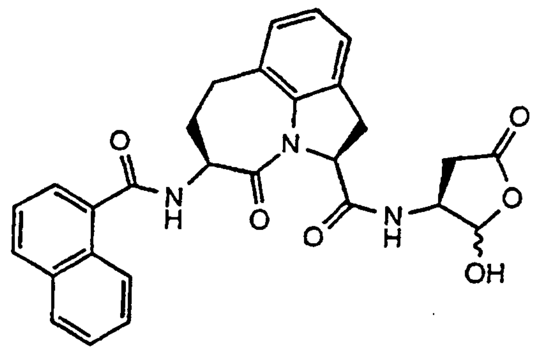

(2S-cis)-[5-(1-Naphthoyl)-amino-1,2,3,4,5,6,7-hexahydro-4-oxoazepino[3,2,1-hi]indole-2-carbonyl)-amino]-4-oxo-butanoic acid

(2S-cis)-[5-(1-Naphthoyl)amino-1,2,3,4,5,6,7-hexahydro-4-oxoazepino[3,2,1-hi]indole-2-carbonyl)-amino]-4-oxo-butanoic acid semicarbazone (0.110 g, ca. 0.173 mmol) was treated with a 3:1:1 solution of methanol-acetic acid-37% formaldehyde (3 mL) and the resulting mixture stirred under nitrogen for 5 hours. The reaction mixture was then diluted with water, methanol removed by evaporation, then the remaining mixture lyophilized. Purification of the crude product by flash chromatography on silica gel (S/P brand silica gel 60Å, 230-400 mesh ASTM) eluting with a 5%-20% methanol-methylene chloride gradient gave 0.076g (86%) of the title compound as a white solid; m.p. 202-203°C (dec). TLC(methylene chloride-methanol-acetic acid, 20:1:1) Rf=0.3. Mass spectrum: m/z 498 (M-H). $^1$H-NMR (300 MHz, $CDCl_3$) δ 9.38 (bs, 1H), 8.94 (m, 1H), 8.56 (m, 1H), 8.36 (m, 1H), 7.94-8.02 (m, 2H), 7.68 (d, J=6.9 Hz, 1H), 7.51-7.59 (m, 3H), 7.07-7.13 (m, 2H), 6.97 (m, 1H), 5.20 (d, J= 10.5, 1Hz), 4.67 (m, 1H), 4.15 (m, 1H), 3.49 (m, 1H), 2.95-3.23 (m, 2H), 2.53 (m, 1H), 2.22-2.34 (m, 2H).

## EXAMPLE 98

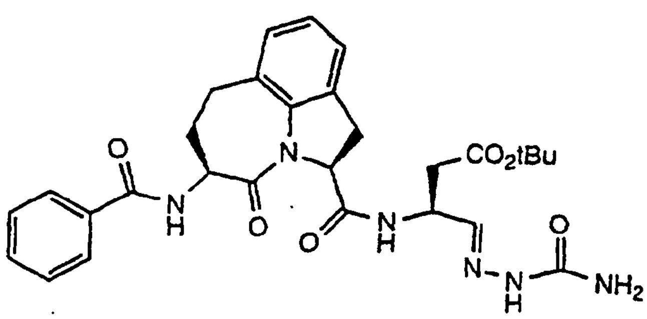

(2S-cis)-[5-Benzoylamino-1,2,3,4,5,6,7-hexahydro-4-oxoazepino[3,2,1-hi]indole-2-carbonyl)-amino]-4-oxo-butanoic acid tert-butyl ester semicarbazone

To a solution of (2S-cis)-[5-amino-1,2,3,4,5,6,7-hexahydro-4-oxoazepino[3,2,1-hi]indole-2-carbonyl)-amino]-4-oxo-butanoic acid tert-butyl ester semicarbazone (0.121 g, 0.264 mmol) in methylene chloride (2.5 mL) stirring at 0°C under nitrogen was added triethylamine (0.055 mL, 0.396 mmol), followed by benzoyl chloride (0.037 mL, 0.317

mmol). After stirring at room temperature under nitrogen for 1 hour, the reaction mixture was diluted with ethyl acetate and washed successively with 5% potassium bisulfate, saturated sodium bicarbonate and saturated sodium chloride solutions; dried (sodium sulfate) and evaporated to dryness. Purification of the crude product by flash chromatography on silica gel (S/P brand silica gel 60Å, 230-400 mesh ASTM) eluting with 10% hexane-ethyl acetate, 100% ethyl acetate, then 10% methanol-ethyl acetate gave 0.073 g (49%) of the title compound as a off-white solid. TLC (methylene chloride-methanol, 9:1) Rf=0.7. Mass spectrum: m/z 563 (M+H). $^1$H-NMR (300 MHz, $CDCl_3$): δ 8.61 (bs, 1H), 7.83-7.86 (m, 2H), 7.47-7.53 (m,3H), 7.06-7.12 (m, 3H), 5.30 (dd, J= 2.2, 7.8 Hz, 1H), 4.89 (m, 1H), 4.72 (m, 1H), 3.60 (d, J= 16.5 Hz, 1H), 3.36 (m, H), 3.19 (m, 1H), 2.69 (dd, J= 4.4, 11.7 Hz, 1H), 2.52 (m, 1H), 2.29 (m, 1H), 1.34 (s, 9H).

## EXAMPLE 99

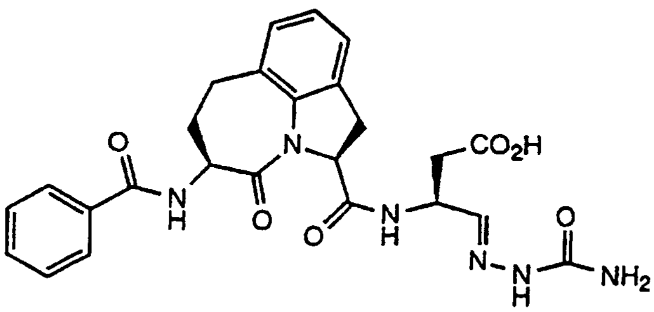

(2S-cis)-[5-Benzoylamino-1,2,3,4,5,6,7-hexahydro-4-oxoazepino[3,2,1-hi]indole-2-carbonyl)-amino]-4-oxo-butanoic acid semicarbazone

To a solution of (2S-cis)-[5-benzoylamino-1,2,3,4,5,6,7-hexahydro-4-oxoazepino[3,2,1-hi]indole-2-carbonyl)-amino]-4-oxo-butanoic acid tert-butyl ester semicarbazone (0.064 g, 0.114 mmol) in methylene chloride (1 mL) was added anisole (0.5 mL) followed by trifluoroacetic acid (1 mL). After stirring at room temperature under nitrogen for 2.5 hours, the reaction mixture was diluted with ethyl acetate and evaporated to give the title compound (0.070 g). TLC (methylene chloride-methanol, 4:1) Rf=0.4. Mass spectrum: m/z 507 (M+H).

## EXAMPLE 100

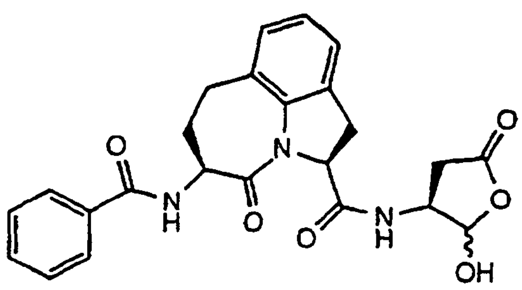

(2S-cis)-[5-Benzoylamino-1,2,3,4,5,6,7-hexahydro-4-oxoazepino[3,2,1-hi]indole-2-carbonyl)-amino]-4-oxo-butanoic acid

(2S-cis)-[5-Benzoylamino-1,2,3,4,5,6,7-hexahydro-4-oxoazepino[3,2,1 -bi]indole-2-carbonyl)-amino]-4-oxo-butanoic acid semicarbazone (0.070 g, ca. 0.114 mmol) was treated with a 3:1:1 solution of methanol-acetic acid-37% formaldehyde (3 mL), and the resulting mixture stirred under nitrogen for 3.5 hours. The reaction mixture was then

diluted with water, methanol removed by evaporation, then the remaining mixture lyophilized. Purification of the crude product by flash chromatography on silica gel (S/P brand silica gel 60Å, 230-400 mesh ASTM) eluting with 10 and 20% methanol-methylene chloride gave 0.042 g (82%) of the title compound as a white solid; m.p. 204-205°C (dec). TLC (methylene chloride-methanol, 4:1) Rf=0.4. Mass spectrum: m/z 448 (M-H). $^1$H-NMR (300 MHz, DMSO-$d_6$) δ 8.84 (m, 1H), 8.53 (m, 1H), 7.91-7.95 (m, 2H), 7.46-7.58 (m, 3H), 7.11 (m, 2H), 6.99 (t, J=7.3 Hz, 1H), 5.14 (d, 10.2 Hz, 1H), 4.62 (m, 1H), 4.23 (m, 1H), 3.48 (m, 1H), 3.12-3.18 (m, 2H), 2.99 (m, 1H), 2.58 (m, 1H), 2.12-2.46 (m, 3H).

## EXAMPLE 101

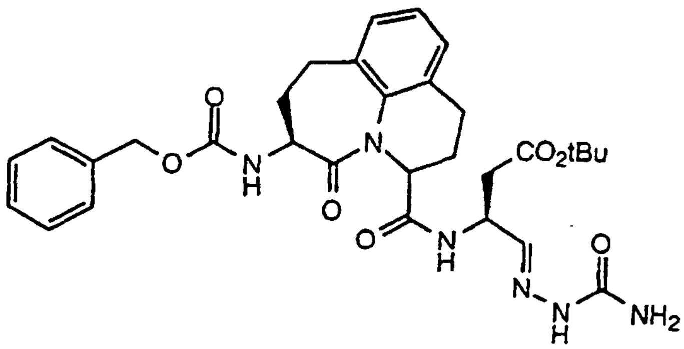

(3R, S-cis)-6-Benzyloxycarbonylamino-5-oxo-2,3,4,5,6,7,8-hexahydro-1H-azepino[3,2,1-hi]quinoline-3-carbonyl)-amino]-4-oxo-butanoic acid tert-butyl ester semicarbazone

1. Preparation of (3R,S-cis)-6-Benzyloxycarbonylamino-5-oxo-2,3,4,5,6,7,8-hexahydro-1H-azepino[3,2,1-hi] quinoline-3-carboxylic acid. methyl ester

To a solution of (3R,S-cis)-6-Amino-5-oxo-2,3,4,5,6,7,8-hexahydro-1H-azepino[3,2,1-hi]quinoline-3-carboxylic acid, methyl ester (0.570 g, 2.1 mmol, prepared as described in Tetrahderon Letters 36, pp. 1593-1596 (1995) and U.S. Patent 5,504,080 (April 2, 1996)) in methylene chloride (6 mL) stirring at 0°C was added benzyl chloroformate (0.6 mL, 4.2 mmol) and triethylamine (1.2 mL, 8.4 mmol) and the resulting mixture was stirred under nitrogen for 30 minutes. The reaction was quenched with water then partitioned between ethyl acetate and 5% aqueous potassium bisulfate solution. The aqueous layer was back extracted two times with ethyl acetate, then the combined organic layers were washed with saturated sodium chloride solution, dried (sodium sulfate) and evaporated to dryness. Purification of the crude product by flash chromatography on silica gel (S/P brand silica gel 60Å, 230-400 mesh ASTM) eluting with ethyl acetate-hexane (2:1) gave 0.643 g (76%) of of the title compound as a white foam. TLC (methylene chloride-methanol, 95:5) Rf=0.8. $^1$H-NMR (300 MHz, $CDCl_3$) δ 7.36-7.25 (m, 5H), 7.13-7.02 (m, 3H), 5.67 (d, J=7.8 Hz, 1H), 5.02 (t, J=9.15, 18.3 Hz, 2H), 4.34 ( m, 1H), 3.70 (s, 3H), 3.16 (m, 1H), 2.69-2.56 (m, 5H), 2.06 (m, 1H). Mass spectrum: m/z 408 (M+H).

2 Preparation of (3R,S-cisy-6-Benzyloxycarbonylamino-5-oxo-2,3,4,5,6,7,8-hexahydro-1H-azepino[3,2,1-hi]quinoline-3-carboxylic acid

To a solution of (3R,S-cis)-6-Benzyloxycarbonylamino-5-oxo-2,3,4,5,6,7,8-hexahydro-1H-azepino[3,2,1-hi] quinoline-3-carboxylic acid, methyl ester (0.622g, 1.53 mmol) in 1,4-dioxane (10.5 mL) and water (3.5 mL) was added 1M aqueous lithium hydroxide (2.3 mL, 2.3 mmol) and the resulting mixture was stirred at room temperature under nitrogen for 1 hour. The reaction mixture was acidified to ca. pH 2 with a 5% aqueous potassium bisulfate solution, then partitioned between ethyl acetate and saturated sodium chloride solution. The aqueous layer was back extracted two times with ethyl acetate, and the combined organic layers were dried (sodium sulfate) and evaporated to yield 0.670 g of the title compound. TLC (methylene chloride-methanol-acetic acid, 32:1:1) Rf=0.35. $^1$H-NMR (300 MHz, $CDCl_3$): δ 7.38-7.28 (m, 5H), 7.13-7.04 (m, 3H), 5.72 (d, J=8.1 Hz, 1H), 5.03 (s, 2H), 4.35 (m, 1H), 3.77-3.67(m, 5H), 3.10 (m, 1H), 2.72-2.52 (m, 5H), 2.07 (m, 1H), 1.70 (m, 1H).

3. (3R,S-cis)-6-Benzyloxycarbonylamino-5-oxo-2,3,4,5,6,7,8-hexahydro-1H-azepino[3,2,1-hi]quinoline-3-carbonyl)-amino]-4-oxo-butanoic acid tert-butyl ester semicarbazone

To a solution of (3R,S-cis)-6-benzyloxycarbonylamino-5-oxo-2,3,4,5,6,7,8-hexahydro-1H-azepino[3,2,1-hi] quinoline-3-carboxylic acid (0.604 g, 1.5 mmol) in methylene chloride (12 mL) stirring at 0°C under nitrogen was added 1-hydroxybenzotriazole hydrate (0.282 g, 1.8 mmol) and 1-(3-dimethylaminopropyl)-3-ethylcarbodiimide hydrochloride (0.442 g, 3 mmol). After 15 minutes, L-aspartic acid semicarbazone β-tert-butyl ester, p-toluenesulfonate salt (0.60 g, 1.5 mmol) and N-methylmorpholine (0.25 mL, 3 mmol) were added and the mixture allowed to come to room temperature within 1 hour. After stirring an additional hour, the reaction mixture was diluted with ethyl acetate and washed successively with 5% potassium bisulfate and saturated sodium chloride solutions; dried (sodium sulfate) and evaporated to dryness. Purification of the crude product by flash chromatography on silica gel (S/P brand silica gel 60Å, 230-400 mesh ASTM) eluting with 10% methanol-methylene chloride gave 0.523 g (56%) of the title compound as a white foam. TLC (methylene chloride-methanol, 9:1) Rf=0.65. $^1$H-NMR (300 MHz, $CDCl_3$): δ 9.89 (m, 1H), 7.72 (m, 1H), 7.92 (d, J=9 Hz, 1H), 7.65 (d, J=8.1 Hz, 1H), 7.32-7.28 (m, 5H), 7.12 (s, 1H), 7.07 (d, J=5.7 Hz, 2H), 6.03 (d, J=7.5 Hz, 1H), 5.84 (d, J=8.1 Hz, 1H), 5.03 (s, 2H), 5.01 (m, 1H) 4.80 (m, 1H), 4.31 (m, 1H), 2.98 (m, 1H), 2.75-2.41 (m, 7H), 2.12 (m, 1H), 1.77 (m, 1H), 1.39 (s, 9H).

## EXAMPLE 102

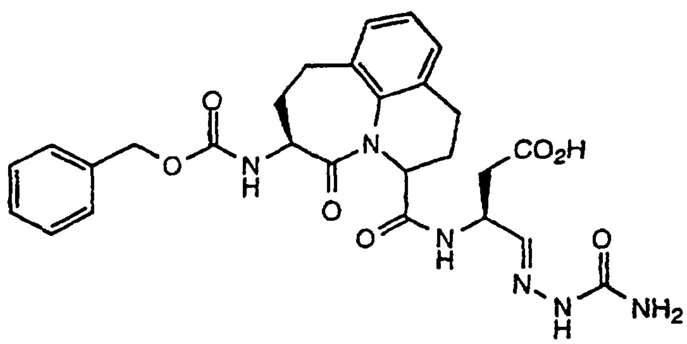

(3R,S-cis)-6-Benzyloxycarbonylamino-5-oxo-2,3,4,5,6,7,8-hexahydro-1H-azepino[3,2,1-hi]quinoline-3-carbonyl)-amino]-4-oxo-butanoic acid semicarbazone

To a solution of (3R,S-cis)-6-Benzyloxycarbonylamino-5-oxo-2,3,4,5,6,7,8-hexahydro-1H-azepino[3,2,1-hi] quinoline-3-carbonyl)-amino]-4-oxo-butanoic acid tert-butyl ester semicarbazone (0.200 g, 0.33 mmol) in methylene chloride (1 mL) was added anisole (0.5 mL, 4.62 mmol) followed by trifluoroacetic acid (1 mL,. After stirring at room temperature under nitrogen for 1.5 hours the reaction mixture was diluted with methylene chloride and evaporated, then azeotroped twice with methylene chloride to give the title compound (0.248 g). TLC (methylene chloride-methanol-acetic acid, 8:1:1) Rf=0.2. Mass spectrum: m/z 549 [M-H]$^-$.

## EXAMPLE 103

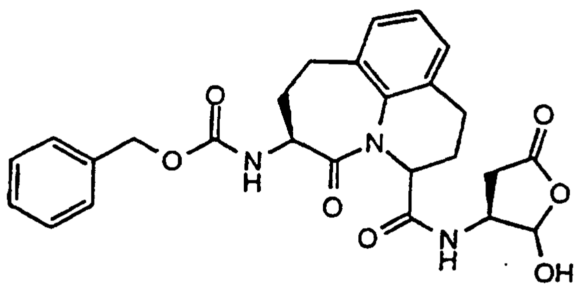

(3R.S-cis)-6-Benzyloxycarbonylamino-5-oxo-2,3,4,5,6,7,8-hexahydro-1H-azepino[3,2,1-hi]quinoline-3-carbonyl)-amino]-4-oxo-butanoic acid

(3R,S-cis)-6-Benzyloxycarbonylamino-5-oxo-2,3,4,5,6,7,8-hexahydro-1H-azepino[3,2,1-hi]quinoline-3-carbonyl)-amino]-4-oxo-butanoic acid semicarbazone (0.245 g, ca 0.33 mmol), was treated with a 3:1:1 solution of methanol-acetic acid-37% formaldehyde (3 mL) and the resulting mixture stirred under nitrogen for 1.5 hours. The reaction mixture was diluted with water, methanol removed by evaporation, then the remaining mixture lyophilized. Purification of the crude product by flash chromatography on reverse phase gel (MCI gel, CHP-20P, 75-150 micron) eluting with a 10%-80% methanol-water gradient gave 0.090 g (60%) of the title compound as a white solid after lyophilization; m. p. 120-123°C (dec). TLC (methylene chloride-methanol-acetic acid, 32:1:1) Rf=0.45. $^1$H-NMR (300 MHz, DMSO d6): δ 8.67 (m, 1H), 7.79 (m, 1H), 7.57 (m, 1H), 7.37-7.27 (m, 5H), 7.17-7.08 (m, 3H), 5.44 (m, 1H), 4.95 (s, 2H), 4.70 (m, 1H), 4.07 (m, 1H), 3.92 (m, 1H), 3.16 (m, 1H), 2.98 (m, 1H), 2.75-2.41 (m, 7H), 2.25 (m, 1H), 2.11 (m, 1H), 1.29 (m, 1H). Mass spectrum: m/z 492 $[M-H]^-$.

## EXAMPLE 104

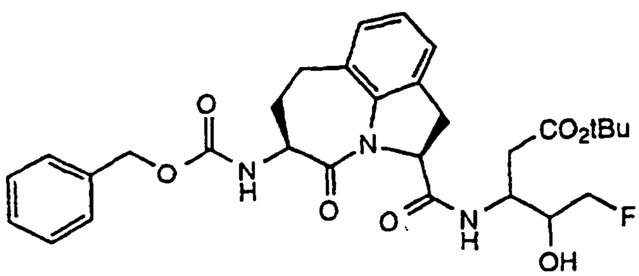

3{(2S-cis)-[5-Benzyloxycarbonylamino-1,2,3,4,5,6,7-hexahydro-4-oxoazepino[3,2,1-hi]indole-2-carbonyl)-amino]}-5-fluoro-4-hydroxy-pentanoic acid tert-butyl ester

To a solution of (2S-cis)-5-benzyloxycarbonylamino- 1,2,3,4,5,6,7-hexahydro-4-oxoazepino[3,2,1-hi]indole-2-carboxylic acid (0.373 g, 0.98 mmol) in methylene chloride (3 mL) stirring at 0°C under nitrogen was added 1-hydroxybenzotriazole hydrate (0.151 g, 0.98 mmol) and 1-(3-dimethylaminopropyl)-3-ethylcarbodiimide hydrochloride (0.283 g, 1.47 mmol). After 15 minutes, 3-amino-4-hydroxy-5-fluoropentanoic acid, tert-butyl ester (0.204 g, 0.98 mmol, prepared as described in Tetrahedron Letters 35, pp. 9693-9696 (1994)) was added and the mixture allowed to come to room temperature within 1 hour. After stirring overnight, the reaction mixture was diluted with ethyl acetate and washed successively with 5% potassium bisulfate and saturated sodium chloride solutions; dried (sodium sulfate) and evaporated to dryness. Purification of the crude product by flash chromatography on silica gel (S/P brand silica gel 60Å, 230-400 mesh ASTM) eluting with 2% methanol-methylene chloride gave 0.383 g (68%) of the title compound as a white foam. TLC (methylene chloride-methanol, 9:1 ) Rf=0.6. $^1$H-NMR (300 MHz, $CDCl_3$): δ 7.45-7.31 (m, 5H),

7.08-7.01 (m, 3H), 6.10 (m, 1H), 5.26 (m, 1H), 5.12 (s, 2H), 4.52 (m, 1H), 4.38-4.30 (m, 2H), 4.21-4.19 (m, 2H), 4.03-3.95 (m, 2H), 3.43-3.20 (m, 4H), 3.13 (m, 2H), 2.62-2.50 (m, 2H), 2.42 (m, 1H), 1.42 (s, 4H), 1.32 (s, 5H). Mass spectrum: m/z 570 (M+H).

## EXAMPLE 105

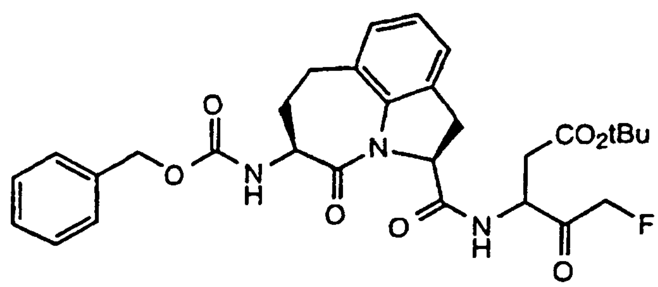

3{(2S-cis)- [5-Benzyloxycarbonylamino-1,2,3,4,5,6,7-hexahydro-4-oxoazepino[3,2,1-hi]indole-2-carbonyl)-amino]}-5-fluoro-4-oxo-pentanoic acid tert-butyl ester

To a solution of 3 {(2S-cis)-[5-benzyloxycarbonylamino-1,2,3,4,5,6,7-hexahydro-4-oxoazepino[3,2,1-hi]indole-2-carbonyl)-amino]}-5-fluoro-4-hydroxy-pentanoic acid tert-butyl ester (0.114 g, 0.20 mmol) in methyl sulfoxide (1.3 mL) was added Dess-Martin periodinane (0.228 g). After stirring at room temperature under nitrogen for 2 hours an additional portion of Dess-Martin periodinane (0.135 g) was added followed 2.5 hours later by a third portion (0.10 g). The reaction mixture was diluted with ethyl acetate and washed twice with water and saturated sodium chloride solution; dried (sodium sulfate) and evaporated to dryness. Purification of the crude product by flash chromatography on silica gel (S/P brand silica gel 60Å, 230-400 mesh ASTM) eluting 1/1 ethyl acetate-hexanes gave 0.076 g (67%) of the title compound as a white foam. TLC (ethyl acetate-hexanes, 1:1) Rf=0.6. $^1$H-NMR (300 MHz, $CDCl_3$): δ 7.58 (d, J = 8.4 Hz, 1H), 7.34-7.30 (m, 5H), 7.07-6.99 (m, 3H), 6.06 (m, 1H), 5.23 (d, J = 12.3 Hz, 1H), 5.12 (s, 2H), 4.53 (d, J = 13.2 Hz, 1H), 4.77 (d, J = 9.9 Hz, 2H), 4.32 (m, 1H), 3.44 (dd, J = 5, 8.4 Hz, 1H), 3.32-3.21 (m, 2H), 3.06 (m, 1H), 2.9 (m, 1H), 2.62 (m, 1H), 2.41 (m, 1H), 2.17 (m, 1H), 1.39 (s, 4H), 1.29 (s, 5H).

## EXAMPLE 106

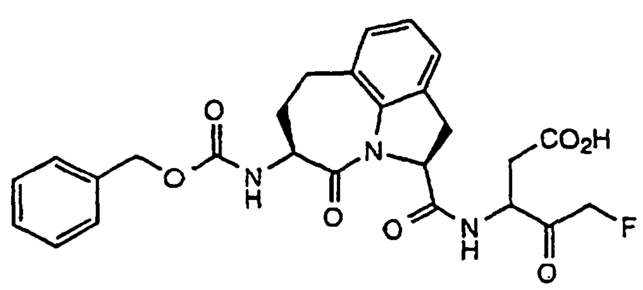

3{(2S-cis)- [5-Benzyloxycarbonylamino-1,2,3,4,5,6,-hexahydro-4-oxoazepino[3,2,1-hi]indole-2-carbonyl)-amino]}-5-fluoro-4-oxo-pentanoic acid

To a solution of 3 {(2S-cis)-[5-benzyloxycarbonylamino-1,2,3,4,5,6,7-hexahydro-4-oxoazepino[3,2,1-hi]indole-2-carbonyl)-amino]}-5-fluoro-4-oxo-pentanoic acid tert-butyl ester (0.063 g, 0.111 mmol) in methylene chloride (1.0 mL) was added anisole (0.5 mL), followed by trifluoroacetic acid (1.0 mL). After stirring at room temperature under nitrogen for 2 hours the reaction mixture was diluted with methylene chloride and evaporated, then chased twice with methylene chloride. The crude residue was triturated with ethyl ether to give 0.030 g of the titled product as a white solid; m.p. 106-107°C (dec). TLC (methylene chloride-methanol-acetic acid, 32:1:1) Rf=0.3. $^1$H-NMR (300 MHz,

$CDCl_3$): δ 7.61 (m, 1H), 7.32 (s, 5H), 7. 1 (d, J = 4 Hz, 1H), 7.03 (d, J = 4 Hz, 2H), 6.17 (m, 1H), 5.22 (m, 1H), 5.10 (s, 2H), 4.75-4.70 (m, 2H), 4.32 (m, 1H), 3.5 (m, 1H), 3.31-3.15 (m, 2H), 3.03 (m, 1H), 2.93 (m, 1H), 2.69 (m, 1H), 2.36 (m, 1H), 2.12 (m, 1H). Mass spectrum: m/z 512 (M+H).

## EXAMPLE 107

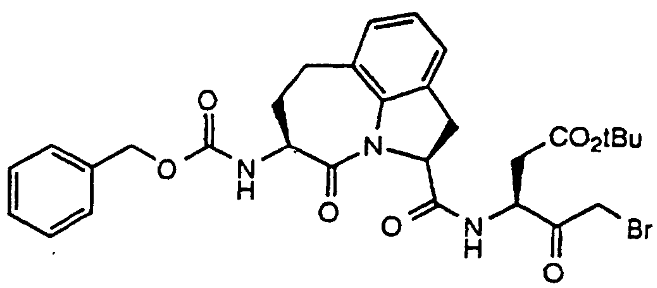

3-[(2S-cis)-[5-Benzyloxycarbonylamino-1,2,3,4,5,6,7-hexahydro-4-oxoazepino[3,2,1-hi]indole-2-carbonyl]amino]-5-bromo-4-oxo-pentanoic acid, tert-butyl ester

To a solution of (2S-cis)-5-benzyloxycarbonylamino-1,2,3,4,5,6,7-hexahydro-4-oxoazepino[3,2,1-hi]indole-2-carboxylic acid (0.302 g, 0.797 mmol) in methylene chloride (5.5 mL) stirring at 0°C under nitrogen was added 1-hydroxybenzotriazole hydrate (0.146 g, 0.96 mmol) and 1-(3-dimethylaminopropyl)-3-ethylcarbodiimide hydrochloride (0.230 g, 1.2 mmol). After 15 minutes, aspartic acid, α-methyl, β-tert-butyl diester hydrochloride (0.191 g, 0.797 mmol) was added followed by N-methylmorpholine (0.13 mL, 1.2 mmol) and the mixture allowed to come to room temperature within 1 hour. After stirring overnight, the reaction mixture was diluted with ethyl acetate and washed successively with 5% potassium bisulfate and saturated sodium chloride solutions; dried (sodium sulfate) and evaporated to dryness. Purification of the crude product by flash chromatography on silica gel (S/P brand silica gel 60Å, 230-400 mesh ASTM) eluting with ethyl acetate-hexane (1:1) gave 0.350 g (78%) of N-[(2S-cis)-[5-benzyloxy- carbonylamino-1,2,3,4,5,6,7-hexahydro-4-oxoazepino[3,2,1-hi]indole-2-carbonyl]aspartic acid, α-methyl, β-tert-butyl diester as a white solid. TLC (methylene chloride-methanol, 9:1 ) $R_f$=0.8. m.p. 147-148°C(dec.).$^1$H-NMR(300 MHz, $CDCl_3$): δ 7.48 (d, J=7.5 Hz, 1H), 7.34-7.29 (m, 5H), 7.07 (m, 1H), 7.03-6.96 (m, 2H), 6.15 (d, J=5.7 Hz, 1H), 5.28 (d, J=7.8 Hz 1H), 5.11 (s, 2H), 4.72 (m, 1H), 4.32 (m, 1H), 3.74 (s, 3H), 3.49 (d, J=16.5 Hz, 1H), 3.31-3.20 (m, 2H), 3.05 (m, 1H), 2.72 (ABX, dd, J=4.65, 15, 64.5 Hz, 2H), 2.43 (m, 1H), 2.15 (m, 1H), 1.30 (s, 9H).

To a solution of the above product (0.330 g, 0.585 mmol) in 1,4-dioxane (4.5 mL) and water (1.5 mL) was added 1M aqueous lithium hydroxide (0.7 mL, 0.702 mmol) and the resulting mixture was stirred at room temperature under nitrogen for 30 minutes. The reaction mixture was acidified to pH 3 with a 0.1N HCl solution, then partitioned between ethyl acetate and saturated sodium chloride solution. The aqueous layer was back extracted two times with ethyl acetate, and the combined organic layers were dried (sodium sulfate) and evaporated to yield 0.275 g (85%) of N-[(2S-cis)-[5-benzyloxycarbonylamino-1,2,3,4,5,6,7-hexahydro-4-oxoazepino[3,2,1-hi]indole-2-carbonyl]]aspartic acid, β-tert-butyl ester as a white foam. TLC(methylene chloride-methanol, 9:1): $R_f$=0.25. $^1$H-NMR(300 MHz, $CDCl_3$): d 7.57 (d, J=7.8 Hz, 1H), d7.35-7.29 (m, 5H), 7.08 (m, 1H), 7.03-6.98 (m, 2H), 6.24 (d, J=6 Hz, 1H), 5.28 (d, J=5.1 Hz 1H), 5.11 (s, 2H), 4.73 (m, 1H), 4.35 (m, 1H), 3.48 (d, J=16.8 Hz, 1H), 3.36-3.20 (m, 2H), 3.07 (m, 1H), 2.76 (ABX, dd, J=4.8, 18, 66 Hz, 2H), 2.40 (m, 1H), 2.19 (m, 1H), 1.33 (s, 9H).

To a solution of the above product (0.262 g, 0.475 mmol) in tetrahydrofuran (3.0 mL) stirring at -10°C under nitrogen was added N-methylmorpholine (0.114 mL, 1.05 mmol) followed by dropwise addition of isobutyl chloroformate (0.107 mL, 0.81 mmol). After 40 minutes the reaction mixture was filtered, the salts washed with dry THF, and the filtrate cooled to 0°C. This was treated with a freshly prepared ethereal solution of diazomethane (excess). After stirring the mixture at 0°C for 30 minutes, a mixture of hydrobromic acid (48% wt. aq. solution)/acetic acid (1.3 mL, 1/1) was added dropwise. After stirring for another 10 minutes, the reaction mixture was diluted with ethyl acetate, then washed successively with saturated sodium bicarbonate and saturated sodium chloride solutions; dried (sodium sulfate) and evaporated to dryness. Purification of the crude product by flash chromatography on silica gel (S/P brand silica gel 60Å, 230-400 mesh ASTM) eluting with ethyl acetate-hexane (1:1) gave 0.200 g (67%) of the title compound as a white foam. TLC(ethyl acetate-hexane, 1:1): $R_f$=0.7. $^1$H-NMR (300 MHz, $CDCl_3$): δ 7.71 (d, J=9 Hz, 1H), d7.35-7.30 (m, 5H), 7.09 (m, 1H), 7.04-7.02 (m, 2H), 6.1 (d, J=5.4 Hz, 1H), 5.28 (d, J=7.2 Hz 1H), 5.12 (s, 2H), 4.89 (dd, J=4.5, 15 Hz 1H),

4.35 (m, 1H), 4.16 (s, 2H), 3.50-3.21(m, 3H), 3.06 (m, 1H), 2.76 (ABX, dd, J=4.65, 18, 103 Hz, 2H), 2.37 (m, 1H), 2.15 (m, 1H), 1.27 (s, 9H). Mass spectrum: m/z 626/628 (M-H).

## EXAMPLE 108

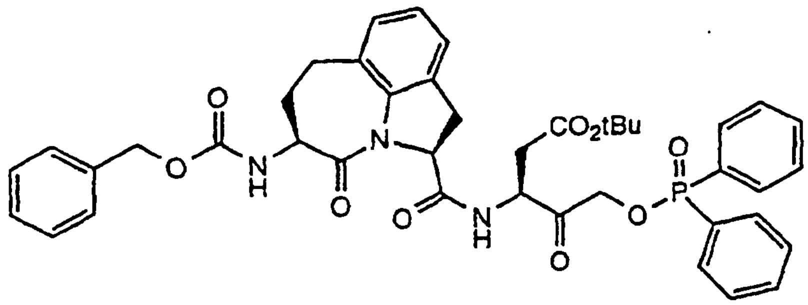

3-[(2S-cis)-[5-Benzyloxycarbonylamino-1,2,3,4,5,6,7-hexahydro-4-oxoazepino[3,2,1-hi]indole-2-carbonyl]amino]-5-(diphenylphosphinyl)oxy-4-oxo-pentanoic acid, tert-butyl ester

To a solution of 3-[(2S-cis)-[5-benzyloxycarbonylamino -1,2,3,4,5,5,7-hexahydro-4-oxoazepino[3,2,1-hi]indole-2-carbonyl]amino]-5-bromo-4-oxo-pentanoic acid, tert-butyl ester (0.069 g, 0.110 mmol) in N,N-dimethylformamide (1.0 mL) was added potassium fluoride (0.029 g, 0.495 mmol), followed diphenylphosphinic acid (0.029 g, 0.139 mmol). After stirring at room temperature under nitrogen for 48 hours, the reaction mixture was diluted with ethyl acetate, then washed successively with a dilute sodium bicarbonate solution then water; dried (sodium sulfate) and evaporated to dryness. Purification of the crude product by flash chromatography on silica gel (S/P brand silica gel 60Å, 230-400 mesh ASTM) eluting with ethyl acetate-hexane (1:1) gave 0.048 g (59%) of the title compound as a clear oil. TLC(ethyl acetate-hexane, 2: 1): $R_f$=0.3. $^1$H-NMR(300 MHz, $CDCl_3$): δ 7.89-7.80 (m, 4H), 7.52-7.30 (m, 11H), 7.06 (m, 1H), 7.01-6.96 (m, 2H), 6.45 (m, 1H), 5.21 (m, 1H), 5.13 (s, 2H), 4.96 (dd, J=8.3, 18 Hz, 1H), 4.78-4.70 (m, 2H), 4.35 (m, 1H), 3.35-3.23 (m, 3H), 3.05 (m, 1H), 2.76 (ABX, dd, J=4.65, 18, 103 Hz, 2H), 2.43(m, 1H), 2.18 (m, 1H), 1.33 (s, 9H).

## EXAMPLE 109

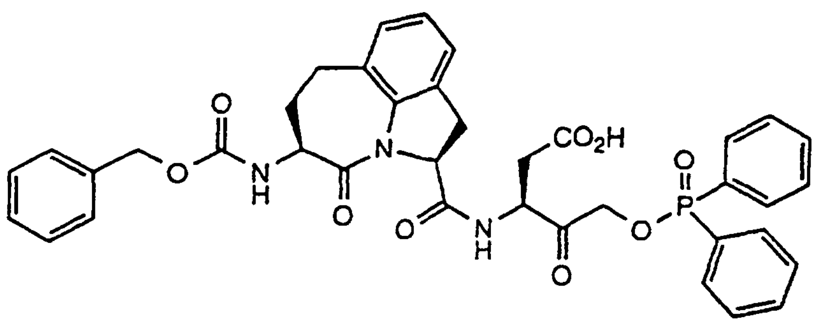

3-[(2S-cis)-[5-Benzyloxycarbonyl-amino-1,2,3,4,5,6,7-hexahydro-4-oxozepino[3,2,1-, hi]indole-2-carbonyl]amino]-5-(diphenylphosphinyl)oxy-4-oxo-pentanoic acid

To a solution of 3-[(2S-cis)-[5-benzyloxycarbonylamino-1,2,3,4,5,6,7-hexahydro-4-oxoazepino[3,2,1-hi]indole-2-carbonyl]amino]-5-(diphenylphosphinyl)oxy-4-oxo-pentanoic acid, tert-butyl ester (0.040 g, 0.054 mmol) in methylene chloride (1.0 mL) was added anisole (0.5 mL), followed by trifluoroacetic acid (1.0 mL). After stirring at room temperature under nitrogen for 30 minutes the reaction mixture was diluted with methylene chloride and evaporated, then azeotroped twice with methylene chloride. The crude residue was triturated with ethyl ether to give 0.030 g of the

titled product as a white solid; m.p. 109-111°C(dec). TLC(methylene chloride-methanol, 9:1): $R_f$=0.4. $^1$H-NMR(300 Mhz, $CDCl_3$): δ 7.87-7.66 (m, 4H), 7.60-7.28 (m, 11H), 7.05-6.95 (m, 3H), 6.84 (m, 1H), 5.12(s, 2H), 5.05 (m, 1H), 4.58 (m, 1H), 4.42-4.15 (m, 4H), 3.35-3.10 (m, 4H), 3.05 (m, 1H), 2.76 (m, 1H), 2.56 (m, 1H), 2.37(m, 1H), 2.13 (m, 1H), 1.93 (bs, 1H). Mass spectrum: m/z 710 (M+H).

EXAMPLE 110

Materials and Methods for Evaluating Effects of ICE/CED-3 Inhibitors on Granulocyte Neutrophils

Neutrophil Isolation:

Whole blood anticoagulated with Acid Citrate Dextrose (ACD) with a ratio of 1:5 ACD to blood was collected (~100 ml).

Using polypropylene plastic ware, neutrophils are isolated as follows:

> 30 ml of the whole blood is added to 50 ml polypropylene centrifuge tubes containing 15 ml of 6% Dextran (in Saline). The blood is allowed to sediment for approximately 1 hour at room temperature.

The turbid straw colored layer harvested from the top of the cylinders into 50 ml conical polypropylene tubes. The blood cells were pelleted by centrifugation at 240 xg (Sorvall centrifuge at 1200 rpm) for 12 min. at 4°C with the brake on low.

The supernatant was aspirated and the pooled pellet resuspended in 40-50 ml cold PBS (w/o Ca, Mg), and centrifuged at 240 xg (Sorvall centrifuge at 1200 rpm) for 6 min. at 4°C with the brake on high.

The supernatant was aspirated and the pellet resuspended in 12 ml of cold cell culture grade water. The suspension was titriated gently with a pipet for 30 seconds then add 4 ml of cold 0.6 M KCl. (Brought up to 50 ml with cold PBS (w/o Ca, Mg)) and then centrifuged at 300 xg (Sorvall centrifuge at 1400 rpm) for 6 min. at 4°C with the brake on high.

The above was repeated one time.

The supernatant was aspirated and the cells resuspended in 2.5 ml cold PBS (w/o Ca, Mg). The cell suspension was layered over 3 ml Ficoll-Hypaque in a 15 ml polypropylene conical tube and centrifuged at 400 xg (Sorvall centrifuge at 1900 rpm) for 30 min. at 4°C with the brake on low.

The suspension aspirated was down to the neutrophil pellet. The pellet was resuspended in cold PBS (w/o Ca, Mg) and transfered to a 50 ml conical tube and brought to 50 ml with cold PBS (w/o Ca, Mg) and centrifuged at 300 xg (Sorvall centrifuge at 1400 rpm) for 6 min. at 4°C with the brake on high.

The supernatant was aspirated and the pellet resuspended in 50 ml cold PBS (w/o Ca Mg) and centrifuged at 300 xg (Sorvall centrifuge at 1400 rpm) for 6 min. at 4°C with the brake on high.

The supernatant was aspirated and the neutrophil pellet resuspended in 4.0 ml cold PBS (w/o Ca, Mg) on ice. 10 μl of the neutrophil cell suspension was diluted with 990 μl of Trypan blue (1:100) and cells counted using a hemacytometer. The cell number and viability were determined.

Neutrophil Culture Conditions:

The culture media was as follows:

> (RPMI 1640; 10% FBS; 10 mM Hepes; 0.2 mM L-glutamine; 25 U/ml penicillin; and 25 mg/ml streptomycin)

Purified neutrophil maintenance was performed under the following conditions: ($5\times10^6$ cells/ml in above culture media; Polystyrene round-bottom 96-well plates; 250 μl/well; and 37°C, 5% $CO_2$/95% air humidified incubator) (Liles *et al.,* Blood 119 (1995) 3181-3188).

Analysis of Hypodiploid Nuclei by Flow Cytometry:

Hypotonic fluorochrome solution

(50 μg/ml propidium iodide (Sigma catalog#P4170); 0.1% Triton X-100; and 0.1% sodium citrate).

Neutrophils were pelleted at 4°C and the supernate aspirated.

Neutrophils were resuspended in hypotonic fluorochrome solution at a density of $5\times10^6$ cells/ml. Propidium iodide fluorescence of individual nuclei was evaluated in FL2 and measured on a logarithmic scale while gating on

physical parameters in forward and side scatter to exlude cell debris.

At least 10,000 events per sample were collected and the results were evaluated relative to a non-apoptotic neutrophil population. (Liles *et al.,* Blood 119 (1995) 3181-3188).

Respiratory burst in isolated neutrophils measured by

Chemiluminescence

Whole blood anticoagulated with Acid Citrate Dextrose (ACD) with a ratio of 1:5 ACD to blood was collected (150 ml).

Neutrophils were isolated as described above.

Opsonized zymosan was prepared by suspending 125 mg zymosan particles in 25 ml pooled human serum (5 mg/ml) and incubating them for 20 minutes at 37°C. Centrifuge the suspension and resuspend the particles in 7 ml of PBS (18 mg/ml) and stored on ice until use (vortex prior to pipetting).

50 ml of a 250 µM solution of Lucigenin (MW 510.5) was prepared by dissolving 6.4 mg of the solid in 50 ml of PBS-G (+ Ca, Mg). 10 µl of PBS-G (+ Ca, Mg) to the wells in a white 96 well plate.

50 µl of the 250 µM Lucigenin solution was added to the wells in a white 96 well plate.

Cell preparations were obatined from cell culture (concentration at time zero= 5.0 x $10^6$ cells/ml) with PBS-G (+ Ca, Mg).

20 µl of the neutrophil suspension was suspended to the appropriate wells and the plate was incubated the plate at 37° C for three minutes. 10 µl of the opsonized zymosan was added to the wells.

The plate was read on the luminometer (Labsystems Luminoskan, Needham Heights, MA) for 14 min. at 37° C in the kinetic mode and record results using the software DeltaSoft.

Whole Blood Assay:

The following reagents were used:

anti-CD32-FITC monoclonal antibody obtained from Pharmingen.
Lysing Solution (10X Stock: 89.9 g NH4Cl;
10.0 g KHCO;
0.37 g tetrasodium EDTA;
dissolve in 1 liter dH2O. Adjust to pH 7.3. Store at 4°C in a tightly closed bottle.
Dilute 1:10 with dH2O prior to use.)
(DPBS without calcium or magnesium obtained from Irivine Scientific.
2% fetal bovine serum in DPBS stored at 4°C.
50 µg/ml propidium iodide in DPBS sterile filtered and stored at 4°C.)

The following protocol was followed:

200 µl blood sample/2.8 ml 1X lysis solution in a 15 ml polypropylene conical tube. Cap and invert to mix. Leave at room temperature for 3-5 minutes.
Centrifuge in a table-top Sorvall at 1200 rpm for 5 minutes at 4°C.
Aspirate supernate. Resuspend pellet in 200 µl/sample 2% FBS/DPBS. Add 20 µl/sample anti-CD32-FITC. Incubate 30 minutes on ice in the dark.
Add 5 ml/sample DPBS. Centrifuge at 1000 rpm for 5 minutes at 4°C.
Aspirate supernate. Resuspend pellet in 1 ml/sample 2% FBS/DPBS.
Add 3 ml/sample ice-cold 95% EtOH dropwise while vortexing gently.
Incubate samples on ice in the dark for 30 minutes.
Centrifuge at 1000 rpm for 5 minutes at 4°C.
Resuspend each sample in 50 µl 5 mg/ml RN'ase. Transfer sample to 900 µl/sample 50 µg/ml Propidium Iodide in 12x75 mm Falcon polystyrene tubes.
Incubate on ice for 30 minutes.
Analyze samples by flow cytometry (argon laser) for forward and side scatter and fluorescence.

EXAMPLE 111

Enhancement of neutrophil/granulocyte survival by ex-vivo application of ICE/ced-3 inhibitors

The present invention provides methods to enhance the ex vivo survival of neutrophils/granulocytes. To establish the ability of compounds to preserve granulocytes in culture, compounds were tested in a number of *in vitro* assays. One common model to test for effects on granulocyte survival involves separating granulocytes from fresh whole blood, culturing the cells at 37°C and testing cells for nuclear hypodiploidy at 24 hour intervals (as described in Example 110). The presence of hypodiploid DNA is a measure of apoptosis, and is assessed using a propidium iodide stain via flow cytometry. Compounds of the present invention were incubated with the granulocytes in culture, their effects on granulocyte survival measured, and an IC50 calculated. In Figure 6, the caspase inhibitor zVADfmk prepared as described in Tetrahedron Letter, 35 9693-9696 (1994) had a weak effect on improving granulocyte survival at 48 hours, whereas example 106 from the present invention had an IC50 of <5μM and thus is a potent inhibitor of granulocyte death.

EXAMPLE 112

Enhancement of apheresis product survival by application of ICE/ced-3 inhibitors

Apheresis (leukapheresis) of blood donors can be performed to obtain a population of cells which is enriched in granulocytes. These cells are then transfused into a recipient in need of additional granulocytes. This apheresis product has a short shelf life, and current standards (American Association of Blood Banks, Standard for Blood Banks and Transfusion Services, Ed. 17, 1996) require storage at 20-24 ° C for no longer than 24 hours. Transfusion is recommended within 6 hours if possible.

Exemplary compounds as described in the present invention can be used to prolong the storage life of apheresis products. For use in the setting of apheresis, the compound can be formulated in a compatible solvent, such as dimethyl sulfoxide (DMSO). The compound can be stored in a vial, and be pre-added to the apheresis bag, or injected into the donor apheresis line during the collection process. The effective final concentration compound could range from 1-25 μM. The leukapheresis product, containing the ICE/ced-3 inhibitor, is then infused into the recipient after storage. Many storage conditions may be possible, for example, storage may be at room temperature for up to one week post-collection.

EXAMPLE 113

Bone marrow reconstitution/hematopoietic cell recovery

In order to demonstrate utility in accelerating reconstitution of hematopoietic cells, compounds are tested in several animal models. One common model to destroy hematopoietic cells involves lethal or sub-lethal irradiation of mice, in which the animals are exposed to X-rays, usually generated from a $^{137}$Cesium source. In the case of sub-lethal irradiation, various endpoints measuring hematopoiesis can be determined at different times following the irradiation, in order to monitor recovery. Compounds of the present invention are administered to animals following sub-lethal irradiation and their effects on recovery are measured. The endpoints can include: hematocrit, white blood cell count, incorporation of tritiated thymidine into bone marrow DNA, spleen weight, number of burst-forming units-erythroid or number of colony-forming units (erythroid, granulocyte/macrophage and megakaryocyte forming lineages) from spleen or bone marrow from humerus or femur. In some cases animals can be further myelosuppressed by injection of chemotherapeutic agents, such as carboplatinum. Compounds which have been efficacious in these models, although acting via different mechanisms, include thrombopoeitin, Photofrin, heme, interleukin-1 and tetrachlorodecaoxide, among others.

Models of lethal irradiation can also be used to test efficacy, although these models generally include bone marrow transplant to rescue the animals. These experiments are often performed on mice, although not exclusively. For syngeneic transplants, time to engraftment can then be measured, using the same endpoints as mentioned above. Engraftment of human progenitor cells can also be measured using SCID-hu mice as the recipients with methods similar to the above, after first establishing the human origin of the hematopoietic cells. In these cases, compounds of the present invention are administered to recipient animals at the time of bone marrow transplant. The bone marrow cells may also be treated with the ICE/CED-3 family inhibitor compounds prior to transplantation.

## Claims

1. A method for expanding or increasing survival of a human cell population comprising contacting the cells, *ex vivo,* with an inhibiting effective amount of a protease inhibitor which suppresses the activity of at least one member of the interleukin-1β-converting enzyme (ICE)/CED-3 family, inhibiting apoptosis in the cell population, thereby expanding or increasing survival of the cell population, wherein the protease inhibitor is a compound of formula 3:

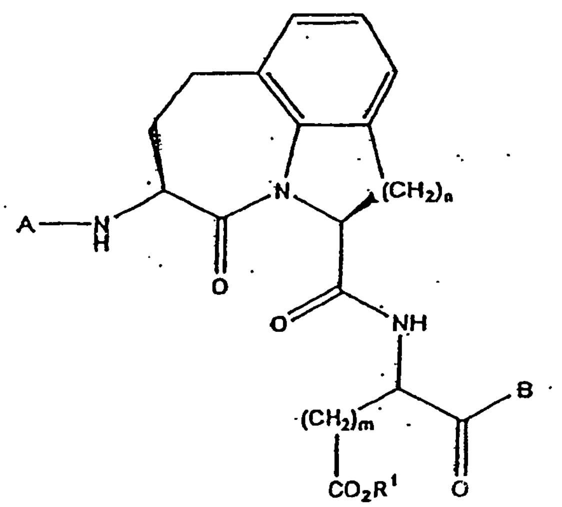

FORMULA 3

wherein:

n is 1 or 2;

m is 1 or 2;

A is $R^2CO$-, $R^3$-O-CO-, or $R^4SO_2$-;

a group of the formula:

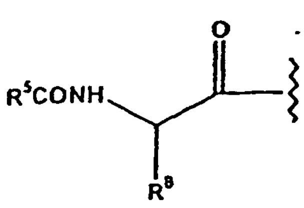 ; 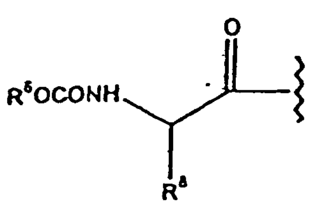 or 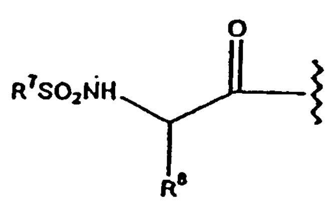 ;

further wherein:

$R^1$ is a hydrogen atom, alkyl or phenylalkyl;

$R^2$ is alkyl, cycloalkyl, (cycloalkyl)alkyl, phenyl, phenylalkyl, substituted phenyl, (substituted phenyl)alkyl, heteroaryl, or (heteroaryl)alkyl;

$R^3$ is alkyl, cycloalkyl, (cycloalkyl)alkyl, phenylalkyl, or (substituted phenyl)alkyl;

$R^4$ is alkyl, cycloalkyl, (cycloalkyl)alkyl, phenyl, phenylalkyl, substituted phenyl, (substituted phenyl)alkyl, heteroaryl, or (heteroaryl)alkyl;

$R^5$ is alkyl, cycloalkyl, (cycloalkyl)alkyl, phenyl, phenylalkyl, substituted phenyl, (substituted phenyl)alkyl, heteroaryl, or (heteroaryl)alkyl;

$R^6$ is alkyl, cycloalkyl, (cycloalkyl)alkyl, phenylalkyl, or (substituted phenyl)alkyl;

$R^7$ is alkyl, cycloalkyl, (cycloalkyl)alkyl, phenyl, phenylalkyl; substituted phenyl, (substituted phenyl)alkyl, heteroaryl, or (heteroaryl)alkyl;

$R^8$ is an amino acid side chain chosen from the group consisting of natural and unnatural amino acids;

B is a hydrogen atom, a deuterium atom, alkyl, cycloalkyl, (cycloalkyl)alkyl, phenyl, phenylalkyl, substituted phenyl, (substituted phenyl)alkyl, heteroaryl, (heteroaryl)alkyl, or halomethyl;

a group of the formula:

$$-CH_2XR^9;$$

wherein $R^9$ is phenyl, substituted phenyl, phenylalkyl, (substituted phenyl)alkyl, heteroaryl, or (heteroaryl)alkyl; and X is an oxygen or a sulfur atom;

a group of the formula:

$$-CH_2\text{-O-CO-(aryl)};$$

a group of the formula:

$$-CH_2\text{- O-CO-(heteroaryl)};$$

a group of the formula:

$$-CH_2\text{-O-PO}(R^{10})R^{11}$$

wherein $R^{10}$ and $R^{11}$ are independently selected from a group consisting of alkyl, cycloalkyl, phenyl, substituted phenyl, phenylalkyl and (substituted phenyl) alkyl; and the pharmaceutically-acceptable salts thereof.

**2.** A method for increasing bioproduction *in vitro* comprising contacting host cells that produce a product of interest with a protease inhibitor which suppresses the activity of at least one member of the ICE/CED-3 family, thereby increasing survival of the cells *in vitro,* wherein the protease inhibitor is a compound of formula 3:

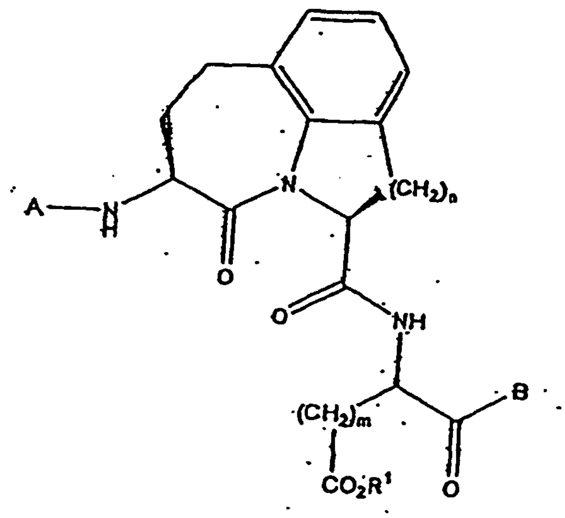

FORMULA 3

wherein:

n is 1 or 2;

m is 1 or 2;

A is $R^2CO$-, $R^3$-O-CO-, or $R^4SO_2$-;

a group of the formula:

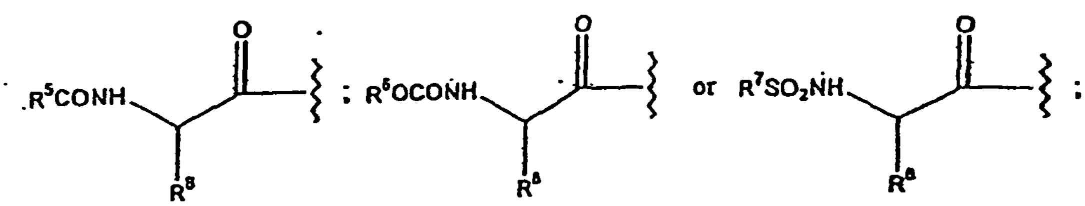

; or ;

further wherein:

$R^1$ is a hydrogen atom, alkyl or phenylalkyl;

$R^2$ is alkyl, cycloalkyl, (cycloalkyl)alkyl, phenyl, phenylalkyl, substituted phenyl, (substituted phenyl)alkyl, heteroaryl, or (heteroaryl)alkyl;

$R^3$ is alkyl, cycloalkyl, (cycloalkyl)alkyl, phenylalkyl, or (substituted phenyl)alkyl:

$R^4$ is alkyl, cycloalkyl, (cycloalkyl)alkyl, phenyl, phenylalkyl, substituted phenyl, (substituted phenyl)alkyl, heteroaryl, or (heteroaryl)alkyl;

$R^5$ is alkyl, cycloalkyl, (cycloalkyl)alkyl, phenyl, phenylalkyl, substituted phenyl, (substituted phenyl)alkyl, heteroaryl, or (heteroaryl)alkyl;

$R^6$ is alkyl, cycloalkyl, (cycloalkyl)alkyl, phenylalkyl or (substituted phenyl)alkyl;

$R^7$ is alkyl, cycloalkyl, (cycloalkyl)alkyl, phenyl, phenylalkyl; substituted phenyl, (substituted phenyl)alkyl, heteroaryl, or (heteroaryl)alkyl;

$R^8$ is an amino acid side chain chosen from the group consisting of natural and unnatural amino acids;

B is a hydrogen atom, a deuterium atom, alkyl, cycloalkyl, (cycloalkyl)alkyl, phenyl, phenylalkyl, substituted phenyl, (substituted phenyl)alkyl, heteroaryl, (heteroaryl)alkyl, or halomethyl;
a group of the formula:

$$-CH_2XR9;$$

wherein $R^9$ is phenyl, substituted phenyl, phenylalkyl, (substituted phenyl)alkyl, heteroaryl, or (heteroaryl)alkyl; and X is an oxygen or a sulfur atom;
a group of the formula:

$$-CH_2\text{-O-CO-(aryl)};$$

a group of the formula:

$$CH_2\text{-O-CO-(heteroaryl)};$$

a group of the formula:

$$-CH_2\text{-O-PO}(R^{10})R^{11}$$

wherein $R^{10}$ and $R^{11}$ are independently selected from a group consisting of alkyl, cycloalkyl, phenyl, substituted phenyl, phenylalkyl and (substituted phenyl) alkyl; and the pharmaceutically-acceptable salts thereof

**3.** A protease inhibitor which suppresses the activity of at least one member of the interleukin-1β-converting enzyme (ICE)/CED-3 family for use in a method for expanding or increasing survival of a human cell population comprising contacting the cells with an inhibiting effective amount of the protease inhibitor, inhibiting apoptosis in the cell population, thereby expanding or increasing survival of the cell population, wherein the protease inhibitor is a compound of formula 3:

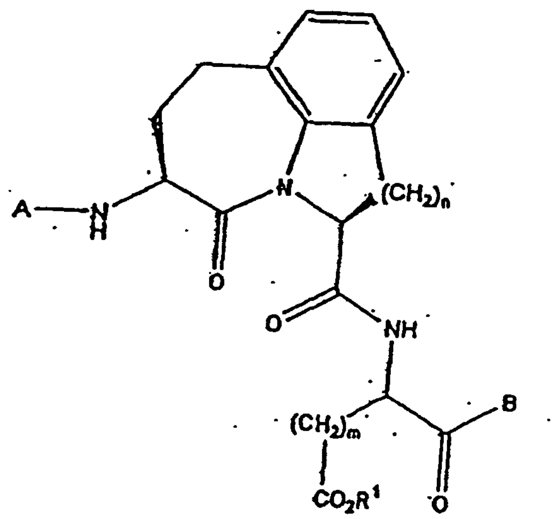

FORMULA 3

n is 1 or 2;
m is 1 or 2;
A is $R^2CO$-, $R^3$-O-CO-, or $R^4SO_2$-;
a group of the formula:

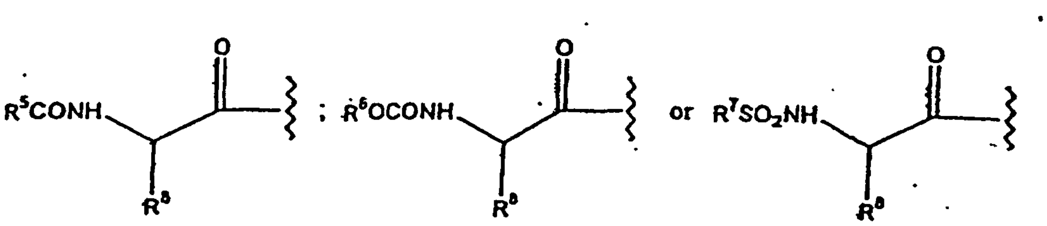

further wherein:

$R^1$ is a hydrogen atom, alkyl or phenylalkyl;

$R^2$ is alkyl, cycloalkyl, (cycloalkyl)alkyl, phenyl, phenylalkyl, substituted phenyl, (substituted phenyl)alkyl, heteroaryl, or (heteroaryl)alkyl;

$R^3$ is alkyl, cycloalkyl, (cycloalkyl)alkyl, phenylalkyl, or (substituted phenyl)alkyl;

$R^4$ is alkyl, cycloalkyl, (cycloalkyl)alkyl, phenyl, phenylalkyl, substituted phenyl, (substituted phenyl)alkyl, heteroaryl, or (heteroaryl)alkyl;

$R^5$ is alkyl, cycloalkyl, (cycloalkyl)alkyl, phenyl, phenylalkyl, substituted phenyl, (substituted phenyl)alkyl, heteroaryl, or (heteroaryl)alkyl;

$R^6$ is alkyl, cycloalkyl, (cycloalkyl)alkyl, phenylalkyl, or (substituted phenyl)alkyl;

$R^7$ is alkyl, cycloalkyl, (cycloalkyl)alkyl, phenyl, phenylalkyl, substituted phenyl, (substituted phenyl)alkyl, heteroaryl, or (heteroaryl)alkyl;

$R^8$ is an amino acid side chain chosen from the group consisting of natural and unnatural amino acids;

B is a hydrogen atom, a deuterium atom, alkyl, cycloalkyl, (cycloalkyl)alkyl, phenyl, phenylalkyl, substituted phenyl, (substituted phenyl)alkyl, heteroaryl, (heteroaryl)alkyl, or halomethyl;
a group of the formula:

$$-CH_2XR^9;$$

wherein $R^9$ is phenyl, substituted phenyl, phenylalkyl, (substituted phenyl)alkyl, heteroaryl, or (heteroaryl)alkyl; and X is an oxygen or a sulfur atom;
a group of the formula:

$$-CH_2\text{-O-CO-(aryl)};$$

a group of the formula:

$$-CH_2\text{-O-CO-(heteroaryl)};$$

a group of the formula:

$$-CH_2\text{-O-PO}(R^{10})R^{11}$$

wherein $R^{10}$ and $R^{11}$ are independently selected from a group consisting of alkyl, cycloalkyl, phenyl, substituted phenyl, phehylalkyl and (substituted phenyl) alkyl; and the pharmaceutically-acceptable salts thereof.

**4.** The method of Claim 1, or the protease inhibitor of Claim 3 wherein the cells are differentiated cells.

**5.** The method of Claim 1, or the protease inhibitor of Claim 3, wherein the cells are precursor cells.

**6.** The method of Claim 1, or the protease inhibitor of Claim 3, wherein the cells are selected from the group consisting of granulocytes, monocytes, erythrocytes, lymphocytes and platelets.

**7.** The method of Claim 1, or the protease inhibitor of Claim 3, wherein the method further comprises contacting the cells with a growth factor.

**Patentansprüche**

**1.** Verfahren zum Ausdehnen oder Erhöhen des Überlebens einer Humanzellpopulation, welches umfaßt, daß die Zellen, *ex vivo,* mit einer hemmenden wirksamen Menge eines Proteaseinhibitors in Kontakt gebracht werden, der die Aktivität wenigstens eines Mitglieds der Interleukin-1β-Converting-Enzym(ICE)/CED-3-Familie unterdrückt, was die Apoptose in der Zellpopulation hemmt, wodurch das Überleben der Zellpopulation ausgedehnt oder erhöht wird, wobei der Proteaseinhibitor eine Verbindung von Formel 3 ist:

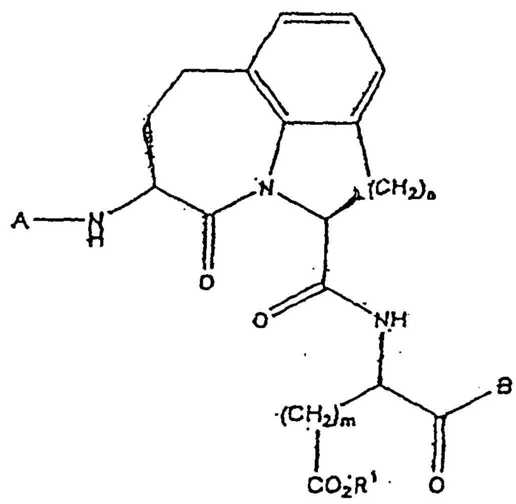

FORMEL 3

worin:

n 1 oder 2 ist;

m 1 oder 2 ist;

A $R^2CO$-, $R^3$-O-CO- oder $R^4SO_2$- ist;

eine Gruppe der Formel:

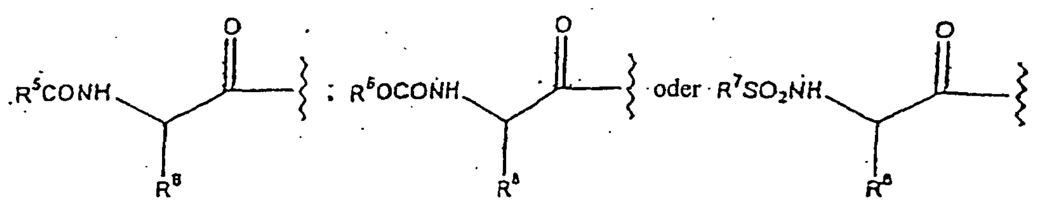

worin weiterhin:

$R^1$ ein Wasserstoffatom, Alkyl oder Phenylalkyl ist;

$R^2$ Alkyl, Cycloalkyl, (Cycloalkyl)alkyl, Phenyl, Phenylalkyl, substituiertes Phenyl, (substituiertes Phenyl)alkyl, Heteroaryl oder (Heteroaryl)alkyl ist;

$R^3$ Alkyl, Cycloalkyl, (Cycloalkyl)alkyl, Phenylalkyl oder (substituiertes Phenyl)alkyl ist;

$R^4$ Alkyl, Cycloalkyl, (Cycloalkyl)alkyl, Phenyl, Phenylalkyl, substituiertes Phenyl, (substituiertes Phenyl)alkyl, Heteroaryl oder (Heteroaryl)alkyl ist;

$R^5$ Alkyl, Cycloalkyl, (Cycloalkyl)alkyl, Phenyl, Phenylalkyl, substituiertes Phenyl, (substituiertes Phenyl)alkyl, Heteroaryl oder (Heteroaryl)alkyl ist;

$R^6$ Alkyl, Cycloalkyl, (Cycloalkyl)alkyl, Phenylalkyl oder (substituiertes Phenyl)alkyl ist;

$R^7$ Alkyl, Cycloalkyl, (Cycloalkyl)alkyl, Phenyl, Phenylalkyl, substituiertes Phenyl, (substituiertes Phenyl)alkyl, Heteroaryl oder (Heteroaryl)alkyl ist;

$R^8$ eine Aminosäureseitenkette ist, die ausgewählt ist aus der Gruppe, die aus natürlichen und unnatürlichen Aminosäuren besteht;

B ein Wasserstoffatom, ein Deuteriumatom, Alkyl, Cycloalkyl, (Cycloalkyl)alkyl, Phenyl, Phenylalkyl, substituiertes Phenyl, (substituiertes Phenyl)alkyl, Heteroaryl, (Heteroaryl)alkyl oder Halomethyl ist;

eine Gruppe der Formel:

$$-CH_2XR^9;$$

worin $R^9$ Phenyl, substituiertes Phenyl, Phenylalkyl, (substituiertes Phenyl)alkyl, Heteroaryl oder (Heteroaryl)alkyl ist; und X ein Sauerstoff- oder ein Schwefelatom ist;
eine Gruppe der Formel:

$$-CH_2\text{-O-CO-(Aryl)};$$

eine Gruppe der Formel:

$$-CH_2\text{-O-CO-(Heteroaryl)};$$

eine Gruppe der Formel:

$$-CH_2\text{-O-PO}(R^{10})R^{11},$$

worin $R^{10}$ und $R^{11}$ unabhängig ausgewählt sind aus einer Gruppe, die aus Alkyl, Cycloalkyl, Phenyl, substituiertem Phenyl, Phenylalkyl und (substituiertes Phenyl)alkyl besteht; und die pharmazeutisch annehmbaren Salze derselben.

**2.** Verfahren zum Erhöhen der Bioproduktion *in vitro,* das umfaßt, daß Wirtszellen, die ein interessierendes Produkt produzieren, mit einem Proteaseinhibitor in Kontakt gebracht werden, der die Aktivität von wenigstens einem Mitglied der ICE/CED-3-Familie unterdrückt, wodurch das Überleben der Zellen *in vitro* erhöht wird, wobei der Proteaseinhibitor eine Verbindung von Formel 3 ist:

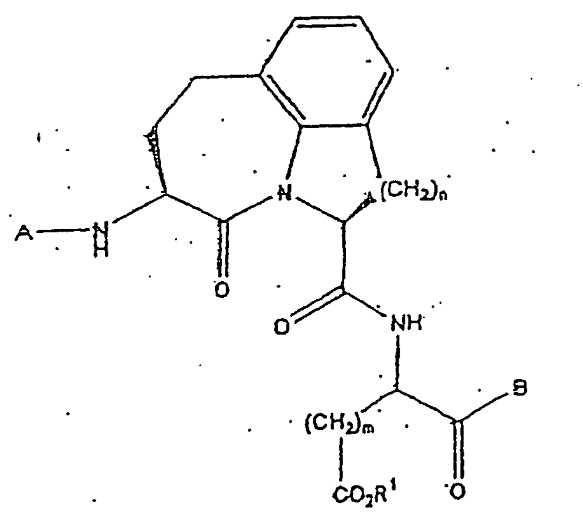

FORMEL 3

worin:

n 1 oder 2 ist;

m 1 oder 2 ist;

A $R^2CO$-, $R^3$-O-CO- oder $R^4SO_2$- ist;

eine Gruppe der Formel:

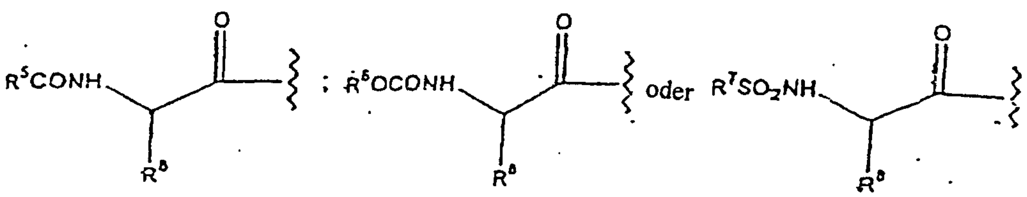

worin weiterhin:

$R^1$ ein Wasserstoffatom, Alkyl oder Phenylalkyl ist;

$R^2$ Alkyl, Cycloalkyl, (Cycloalkyl)alkyl, Phenyl, Phenylalkyl, substituiertes Phenyl, (substituiertes Phenyl)alkyl, Heteroaryl oder (Heteroaryl)alkyl ist;

$R^3$ Alkyl, Cycloalkyl, (Cycloalkyl)alkyl, Phenylalkyl oder (substituiertes Phenyl)alkyl ist;

$R^4$ Alkyl, Cycloalkyl, (Cycloalkyl)alkyl, Phenyl, Phenylalkyl, substituiertes Phenyl, (substituiertes Phenyl)alkyl, Heteroaryl oder (Heteroaryl)alkyl ist;

$R^5$ Alkyl, Cycloalkyl, (Cycloalkyl)alkyl, Phenyl, Phenylalkyl, substituiertes Phenyl, (substituiertes Phenyl)alkyl, Heteroaryl oder (Heteroaryl)alkyl ist;

$R^6$ Alkyl, Cycloalkyl, (Cycloalkyl)alkyl, Phenylalkyl oder (substituiertes Phenyl)alkyl ist;

$R^7$ Alkyl, Cycloalkyl, (Cycloalkyl)alkyl, Phenyl, Phenylalkyl, substituiertes Phenyl, (substituiertes Phenyl)alkyl, Heteroaryl oder (Heteroaryl)alkyl ist;

$R^8$ eine Aminosäureseitenkette ist, die ausgewählt ist aus der Gruppe, die aus natürlichen und unnatürlichen Aminosäuren besteht;

B ein Wasserstoffatom, ein Deuteriumatom, Alkyl, Cycloalkyl, (Cycloalkyl)alkyl, Phenyl, Phenylalkyl, substituiertes Phenyl, (substituiertes Phenyl)alkyl, Heteroaryl, (Heteroaryl)alkyl oder Halomethyl ist;

eine Gruppe der Formel:

$$-CH_2XR^9;$$

worin $R^9$ Phenyl, substituiertes Phenyl, Phenylalkyl, (substituiertes Phenyl)alkyl, Heteroaryl oder (Heteroaryl)alkyl ist; und X ein Sauerstoff- oder ein Schwefelatom ist;
eine Gruppe der Formel:

$$-CH_2\text{-O-CO-(Aryl)};$$

eine Gruppe der Formel:

$$-CH_2-O-CO-(Heteroaryl);$$

eine Gruppe der Formel:

$$-CH_2-O-PO(R^{10})R^{11},$$

worin $R^{10}$ und $R^{11}$ unabhängig ausgewählt sind aus einer Gruppe, die aus Alkyl, Cycloalkyl, Phenyl, substituiertem Phenyl, Phenylalkyl und (substituiertes Phenyl)alkyl besteht; und die pharmazeutisch annehmbaren Salze derselben.

3. Proteaseinhibitor, der die Aktivität wenigstens eines Mitglieds der Interleukin-1β-Converting-Enzym(ICE)/CED-3-Familie unterdrückt, zur Verwendung in einem Verfahren zum Ausdehnen oder Erhöhen des Überlebens einer Humanzellpopulation, welches umfaßt, daß die Zellen mit einer hemmenden wirksamen Menge des Proteaseinhibitors in Kontakt gebracht werden, was die Apoptose in der Zellpopulation hemmt, wodurch das Überleben der Zellpopulation ausgedehnt oder erhöht wird, wobei der Proteaseinhibitor eine Verbindung von Formel 3 ist:

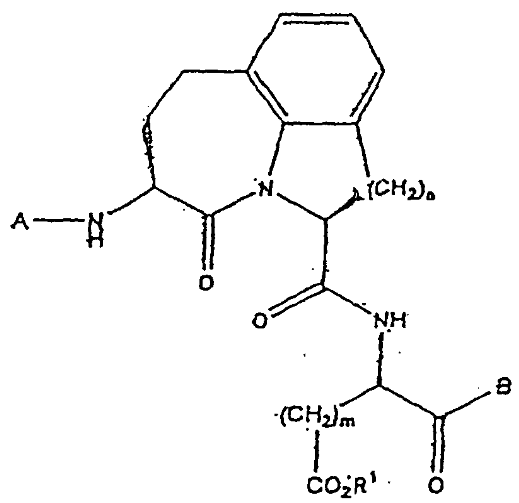

FORMEL 3

worin:

n 1 oder 2 ist;

m 1 oder 2 ist;

A $R^2CO-$, $R^3-O-CO-$ oder $R^4SO_2-$ ist;

eine Gruppe der Formel:

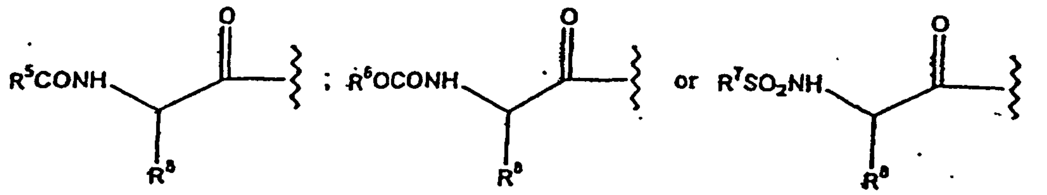

worin weiterhin:

$R^1$ ein Wasserstoffatom, Alkyl oder Phenylalkyl ist;

$R^2$ Alkyl, Cycloalkyl, (Cycloalkyl)alkyl, Phenyl, Phenylalkyl, substituiertes Phenyl, (substituiertes Phenyl)alkyl, Heteroaryl oder (Heteroaryl)alkyl ist;

$R^3$ Alkyl, Cycloalkyl, (Cycloalkyl)alkyl, Phenylalkyl oder (substituiertes Phenyl)alkyl ist;

$R^4$ Alkyl, Cycloalkyl, (Cycloalkyl)alkyl, Phenyl, Phenylalkyl, substituiertes Phenyl, (substituiertes Phenyl)alkyl, Heteroaryl oder (Heteroaryl)alkyl ist;

$R^5$ Alkyl, Cycloalkyl, (Cycloalkyl)alkyl, Phenyl, Phenylalkyl, substituiertes, Phenyl, (substituiertes Phenyl)alkyl, Heteroaryl oder (Heteroaryl)alkyl ist;

$R^6$ Alkyl, Cycloalkyl, (Cycloalkyl)alkyl, Phenylalkyl oder (substituiertes Phenyl)alkyl ist;

$R^7$ Alkyl, Cycloalkyl, (Cycloalkyl)alkyl, Phenyl, Phenylalkyl, substituiertes Phenyl, (substituiertes Phenyl)alkyl, Heteroaryl oder (Heteroaryl)alkyl ist;

$R^8$ eine Aminosäureseitenkette ist, die ausgewählt ist aus der Gruppe, die aus natürlichen und unnatürlichen Aminosäuren besteht;

B ein Wasserstoffatom, ein Deuteriumatom, Alkyl, Cycloalkyl, (Cycloalkyl)alkyl, Phenyl, Phenylalkyl, substituiertes Phenyl, (substituiertes Phenyl)alkyl, Heteroaryl, (Heteroaryl)alkyl oder Halomethyl ist;

eine Gruppe der Formel:

$$-CH_2XR^9;$$

worin $R^9$ Phenyl, substituiertes Phenyl, Phenylalkyl, (substituiertes Phenyl)alkyl, Heteroaryl oder (Heteroaryl)alkyl ist; und X ein Sauerstoff- oder ein Schwefelatom ist;
eine Gruppe der Formel:

$$-CH_2\text{-O-CO-(Aryl)};$$

eine Gruppe der Formel:

$$-CH_2\text{-O-CO-(Heteroaryl)};$$

eine Gruppe der Formel:

$$-CH_2\text{-O-PO}(R^{10})R^{11},$$

worin $R^{10}$ und $R^{11}$ unabhängig ausgewählt sind aus einer Gruppe, die aus Alkyl, Cycloalkyl, Phenyl, substituiertem Phenyl, Phenylalkyl und (substituiertes Phenyl)alkyl besteht; und die pharmazeutisch annehmbaren Salze derselben.

4. Verfahren nach Anspruch 1 oder Proteaseinhibitor nach Anspruch 3, **dadurch gekennzeichnet, daß** die Zellen differenzierte Zellen sind.

5. Verfahren nach Anspruch 1 oder Proteaseinhibitor nach Anspruch 3, **dadurch gekennzeichnet, daß** die Zellen Vorläuferzellen sind.

6. Verfahren nach Anspruch 1 oder Proteaseinhibitor nach Anspruch 3, **dadurch gekennzeichnet, daß** die Zellen ausgewählt sind aus der Gruppe, die aus Granulocyten, Monocyten, Erythrocyten, Lymphocyten und Thrombocyten besteht.

7. Verfahren nach Anspruch 1 oder Proteaseinhibitor nach Anspruch 3, **dadurch gekennzeichnet, daß** das Verfahren weiter umfaßt, daß die Zellen mit einem Wachstumsfaktor in Kontakt gebracht werden.

**Revendications**

1. Procédé pour l'expansion ou l'augmentation de la survie d'une population de cellules humaines comprenant la mise en contact des cellules, ex *vivo,* avec une quantité inhibitrice efficace d'un inhibiteur de protéase qui supprime l'activité d'au moins un membre de la famille des enzymes de conversion de l'interleukine-1β (ICE)/CED-3, inhibant l'apoptose dans la population de cellules, expansant ou augmentant ainsi la survie de la population de cellules, où l'inhibiteur de protéase est un composé de formule 3 :

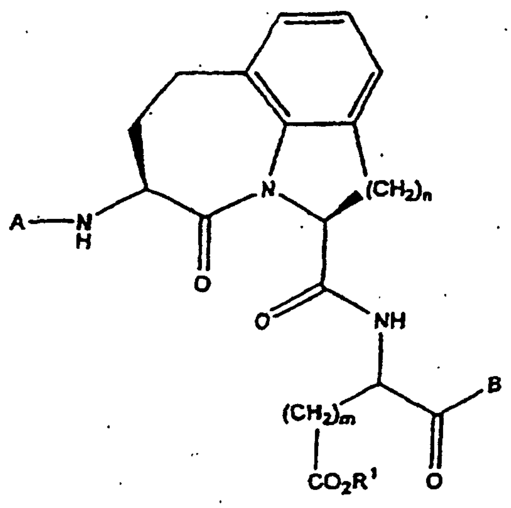

FORMULE 3

où:

n est 1 ou 2 ;
m est 1 ou 2 ;
A est $R^2CO$-, $R^3$-O-CO- ou $R^4SO_2$-;

un groupe de formule :

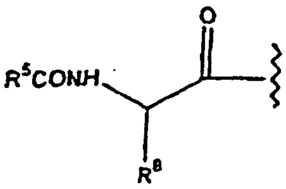 ; 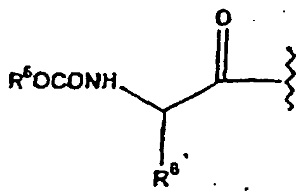 ou 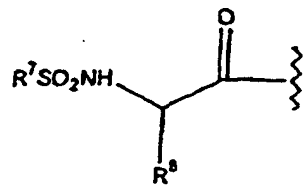 ;

où en outre:

$R^1$ est un atome d'hydrogène, alkyle ou phénylalkyle ;
$R^2$ est alkyle, cycloalkyle, (cycloalkyl)alkyle, phényle, phénylalkyle, phényle substitué, (phényle subsitué)alkyle, hétéroaryle ou (hétéroaryl)alkyle ;
$R^3$ est alkyle, cycloalkyle, (cycloalkyl)alkyle, phénylalkyle ou (phényle substitué)alkyle ;
$R^4$ est alkyle, cycloalkyle, (cycloalkyl)alkyle, phényle, phénylalkyle, phényle substitué, (phényle subsitué)alkyle, hétéroaryle ou (hétéroaryl)alkyle ;
$R^5$ est alkyle, cycloalkyle, (cycloalkyl)alkyle, phényle, phénylalkyle, phényle substitué, (phényle subsitué)alkyle, hétéroaryle ou (hétéroaryl)alkyle ;
$R^6$ est alkyle, cycloalkyle, (cycloalkyl)alkyle, phénylalkyle ou (phényle substitué)alkyle ;
$R^7$ est alkyle, cycloalkyle, (cycloalkyl)alkyle, phényle, phénylalkyle, phényle substitué, (phényle subsitué)alkyle, hétéroaryle ou (hétéroaryl)alkyle ;
$R^8$ est une chaîne latérale d'aminoacide choisie dans le groupe consistant en les aminoacides naturels et non naturels ;
B est un atome d'hydrogène, un atome de deutérium, alkyle, cycloalkyle, (cycloalkyl)alkyle, phényle, phénylalkyle, phényle substitué, (phényle substitué)alkyle, hétéroaryle, (hétéroaryl)alkyle ou halogénométhyle ;

un groupe de formule :

$$-CH_2XR^9 ;$$

où $R^9$ est phényle, phényle substitué, phénylalkyle, (phényle subsitué)alkyle, hétéroaryle ou (hétéroaryl)alkyle ; et X est un atome d'oxygène ou de soufre ;
un groupe de formule :

$$-CH_2\text{-O-CO-(aryle)} ;$$

un groupe de formule :

$$-CH_2\text{-O-CO-(hétéroaryle)} ;$$

un groupe de formule :

$$-CH_2\text{-O-PO}(R^{10})R^{11}$$

où $R^{10}$ et $R^{11}$ sont choisis indépendamment dans un groupe consistant en alkyle, cycloalkyle, phényle, phényle substitué, phénylalkyle et (phényle substitué)alkyle ; et ses sels pharmaceutiquement acceptables.

**2.** Procédé pour augmenter la bioproduction *in vitro* comprenant la mise en contact de cellules hôtes qui produisent un produit d'intérêt avec un inhibiteur de protéase qui supprime l'activité d'au moins un membre de la famille des ICE/CED-3, augmentant ainsi la survie des cellules *in vitro,* où l'inhibiteur de protéase est un composé de formule 3 :

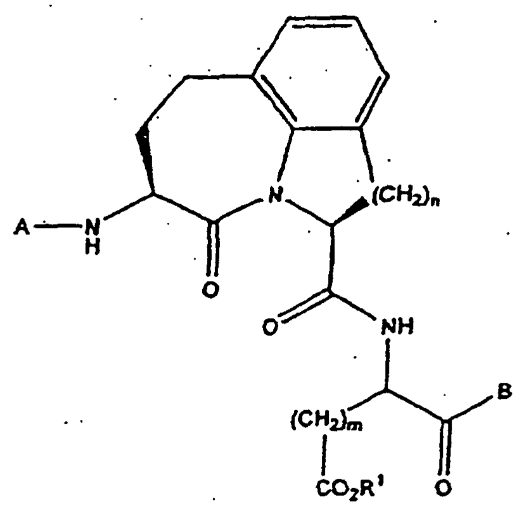

FORMULE 3

où :

n est 1 ou 2 ;
m est 1 ou 2 ;
A est $R^2CO$-, $R^3$-O-CO- ou $R^4SO_2$- ;

un groupe de formule :

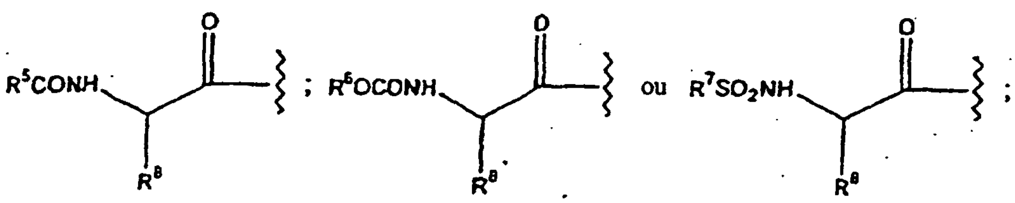

où en outre :

$R^1$ est un atome d'hydrogène, alkyle ou phénylalkyle ;
$R^2$ est alkyle, cycloalkyle, (cycloalkyl)alkyle, phényle, phénylalkyle, phényle substitué, (phényle subsitué)alkyle, hétéroaryle ou (hétéroaryl)alkyle ;
$R^3$ est alkyle, cycloalkyle, (cycloalkyl)alkyle, phénylalkyle ou (phényle substitué)alkyle ;
$R^4$ est alkyle, cycloalkyle, (cycloalkyl)alkyle, phényle, phénylalkyle, phényle substitué, (phényle subsitué)alkyle, hétéroaryle ou (hétéroaryl)alkyle ;
$R^5$ est alkyle, cycloalkyle, (cycloalkyl)alkyle, phényle, phénylalkyle, phényle substitué, (phényle subsitué)alkyle, hétéroaryle ou (hétéroaryl)alkyle ;
$R^6$ est alkyle, cycloalkyle, (cycloalkyl)alkyle, phénylalkyle ou (phényle substitué)alkyle ;
$R^7$ est alkyle, cycloalkyle, (cycloalkyl)alkyle, phényle, phénylalkyle, phényle substitué, (phényle subsitué)alkyle, hétéroaryle ou (hétéroaryl)alkyle ;
$R^8$ est une chaîne latérale d'aminoacide choisie dans le groupe consistant en les aminoacides naturels et non naturels ;
B est un atome d'hydrogène, un atome de deutérium, alkyle, cycloalkyle, (cycloalkyl)alkyle, phényle, phénylalkyle, phényle substitué, (phényle substitué)alkyle, hétéroaryle, (hétéroaryl)alkyle ou halogénométhyle ;

un groupe de formule :

$-CH_2XR^9$ ;

où $R^9$ est phényle, phényle substitué, phénylalkyle, (phényle subsitué)alkyle, hétéroaryle ou (hétéroaryl)alkyle ; et X est un atome d'oxygène ou de soufre ;
un groupe de formule :

$-CH_2$-O-CO-(aryle) ;

un groupe de formule :

$-CH_2$-O-CO-(hétéroaryle) ;

un groupe de formule :

$-CH_2-O-PO(R^{10})R^{11}$

où $R^{10}$ et $R^{11}$ sont choisis indépendamment dans un groupe consistant en alkyle, cycloalkyle, phényle, phényle substitué, phénylalkyle et (phényle substitué)alkyle ; et ses sels pharmaceutiquement acceptables.

**3.** Inhibiteur de protéase qui supprime l'activité d'au moins un membre de la famille des enzymes de conversion de l'interleukine-1β (ICE)/CED-3 destiné à être utilisé dans un procédé pour l'expansion ou l'augmentation de la survie d'une population de cellules humaines comprenant la mise en contact des cellules avec une quantité inhibitrice efficace de l'inhibiteur de protéase, inhibant l'apoptose dans la population de cellules, expansant ou augmentant ainsi la survie de la population de cellules, où l'inhibiteur de protéase est un composé de formule 3:

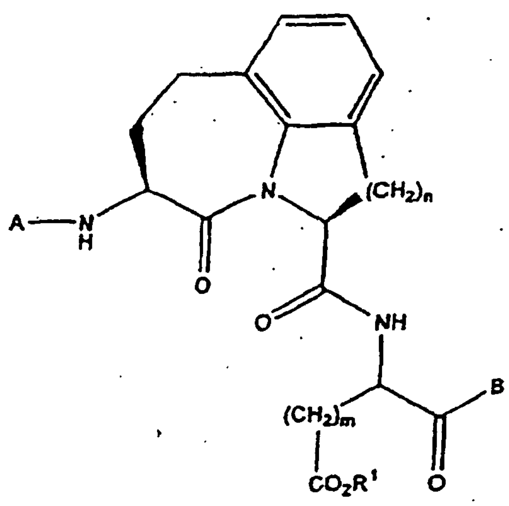

FORMULE 3

où :

n est 1 ou 2 ;
m est 1 ou 2 ;
A est $R^2CO$-, $R^3$-O-CO- ou $R^4SO_2$- ;

un groupe de formule :

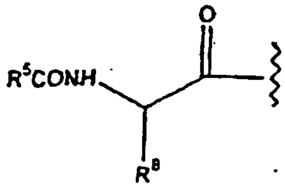 ; 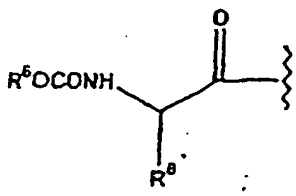 ou 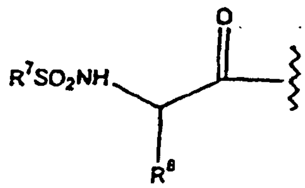 ;

où en outre :

- $R^1$ est un atome d'hydrogène, alkyle ou phénylalkyle ;
- $R^2$ est alkyle, cycloalkyle, (cycloalkyl)alkyle, phényle, phénylalkyle, phényle substitué, (phényle subsitué)alkyle, hétéroaryle ou (hétéroaryl)alkyle ;
- $R^3$ est alkyle, cycloalkyle, (cycloalkyl)alkyle, phénylalkyle ou (phényle substitué)alkyle ;
- $R^4$ est alkyle, cycloalkyle, (cycloalkyl)alkyle, phényle, phénylalkyle, phényle substitué, (phényle subsitué)alkyle, hétéroaryle ou (hétéroaryl)alkyle ;
- $R^5$ est alkyle, cycloalkyle, (cycloalkyl)alkyle, phényle, phénylalkyle, phényle substitué, (phényle subsitué)alkyle, hétéroaryle ou (hétéroaryl)alkyle ;
- $R^6$ est alkyle, cycloalkyle, (cycloalkyl)alkyle, phénylalkyle ou (phényle substitué)alkyle ;
- $R^7$ est alkyle, cycloalkyle, (cycloalkyl)alkyle, phényle, phénylalkyle, phényle substitué, (phényle subsitué)alkyle, hétéroaryle ou (hétéroaryl)alkyle ;
- $R^8$ est une chaîne latérale d'aminoacide choisie dans le groupe consistant en les aminoacides naturels et non naturels ;
- B est un atome d'hydrogène, un atome de deutérium, alkyle, cycloalkyle, (cycloalkyl)alkyle, phényle, phénylalkyle, phényle substitué, (phényle substitué)alkyle, hétéroaryle, (hétéroaryl)alkyle ou halogénométhyle ;

un groupe de formule :

$$-CH_2XR^9;$$

où $R^9$ est phényle, phényle substitué, phénylalkyle, (phényle subsitué)alkyle, hétéroaryle ou (hétéroaryl)alkyle ; et X est un atome d'oxygène ou de soufre ; un groupe de formule :

$$-CH_2\text{-O-CO-(aryle)} ;$$

un groupe de formule :

$$-CH_2\text{-O-CO-(hétéroaryle)} ;$$

un groupe de formule :

$$-CH_2\text{-O-PO}(R^{10})R^{11}$$

où $R^{10}$ et $R^{11}$ sont choisis indépendamment dans un groupe consistant en alkyle, cycloalkyle, phényle, phényle substitué, phénylalkyle et (phényle substitué)alkyle ; et ses sels pharmaceutiquement acceptables.

**4.** Procédé selon la revendication 1 ou inhibiteur de protéase selon la revendication 3 où les cellules sont des cellules differenciées.

**5.** Procédé selon la revendication 1 ou inhibiteur de protéase selon la revendication 3 où les cellules sont des cellules précurseurs.

**6.** Procédé selon la revendication 1 ou inhibiteur de protéase selon la revendication 3 où les cellules sont choisies dans le groupe consistant en les granulocytes, les monocytes, les érythrocytes, les lymphocytes et les plaquettes.

**7.** Procédé selon la revendication 1 ou inhibiteur de protéase selon la revendication 3 où le procédé comprend en outre la mise en contact des cellules avec un facteur de croissance.

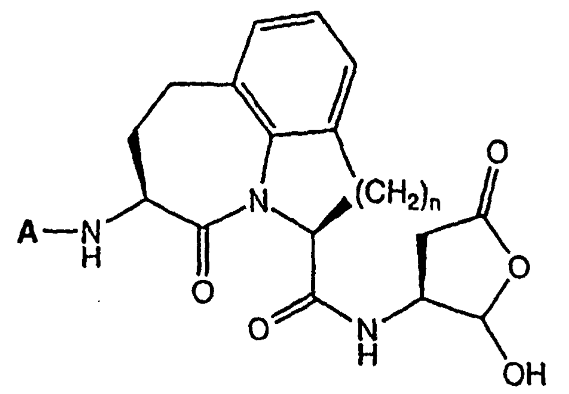

D

| TABLE 4<br>50% INHIBITORY CONCENTRATIONS $IC_{50}$<br>FOR FORMULA D | | | | | | | |
|---|---|---|---|---|---|---|---|
| Example No. | A | n | mICE $IC_{50}$ (μM) | CPP32 $IC_{50}$ (μM) | MCH-2 $IC_{50}$ (μM) | MCH-3 $IC_{50}$ (μM) | MCH-5 $IC_{50}$ (μM) |
| 78 | Cbz | 1 | 0.019 | 1.03 | 40.1 | 6.96 | >10 |
| 82 | Ac-Asp | 1 | 0.694 | 0.0014 | 6.47 | 0.145 | 2.09 |
| 85 | succinyl | 1 | 0.571 | 0.245 | 1.81 | 2.83 | 7.98 |
| 88 | Cbz-Asp | 1 | 0.096 | 0.00052 | ND | 0.084 | 1.19 |
| 91 | dihydrocinnamoyl | 1 | 0.045 | 0.780 | >10 | 32.6 | 18.7 |
| 94 | Ac | 1 | 3.07 | 3.87 | >10 | >50 | >50 |
| 100 | Benzoyl | 1 | 0.159 | 8.77 | >50 | >50 | 4.63 |
| 97 | 1-Naphthoyl | 1 | 0.010 | 2.91 | >50 | 12.3 | 1.09 |
| 103 | Cbz | 2 | 0.026 | 0.437 | 32.0 | 1.11 | 2.06 |
| reference | - | - | 0.064 | 47.0 | >10 | >10 | 2.96 |

FIGURE 4

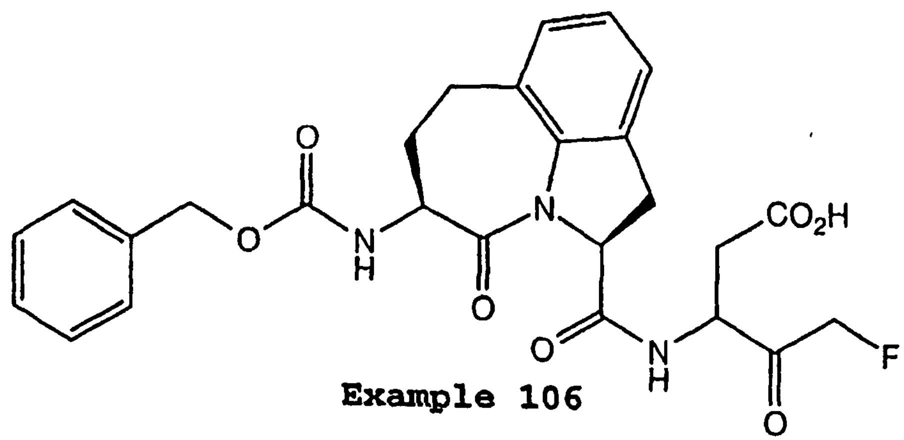

Example 106

| TABLE 5<br>DISSOCIATION CONSTANT Ki AND INACTIVATION RATE<br>$k_3$/Ki FOR EXAMPLE 106 | | | | |
|---|---|---|---|---|
| | Example 106 | | Reference | |
| Enzyme | Ki (μM) | $k_3$/Ki ($M^{-1}s^{-1}$) | Ki (μM) | $k_3$/Ki ($M^{-1}s^{-1}$) |
| mICE | 0.0005 | 12,000,000 | 0.015 | 214,000 |
| CPP32 | 0.012 | 960,000 | 0.820 | 12,200 |
| MCH-2 | 0.033 | 25,000 | 0.594 | 2,950 |
| MCH-5 | 0.022 | 98,000 | 0.018 | 83,300 |

FIGURE 5

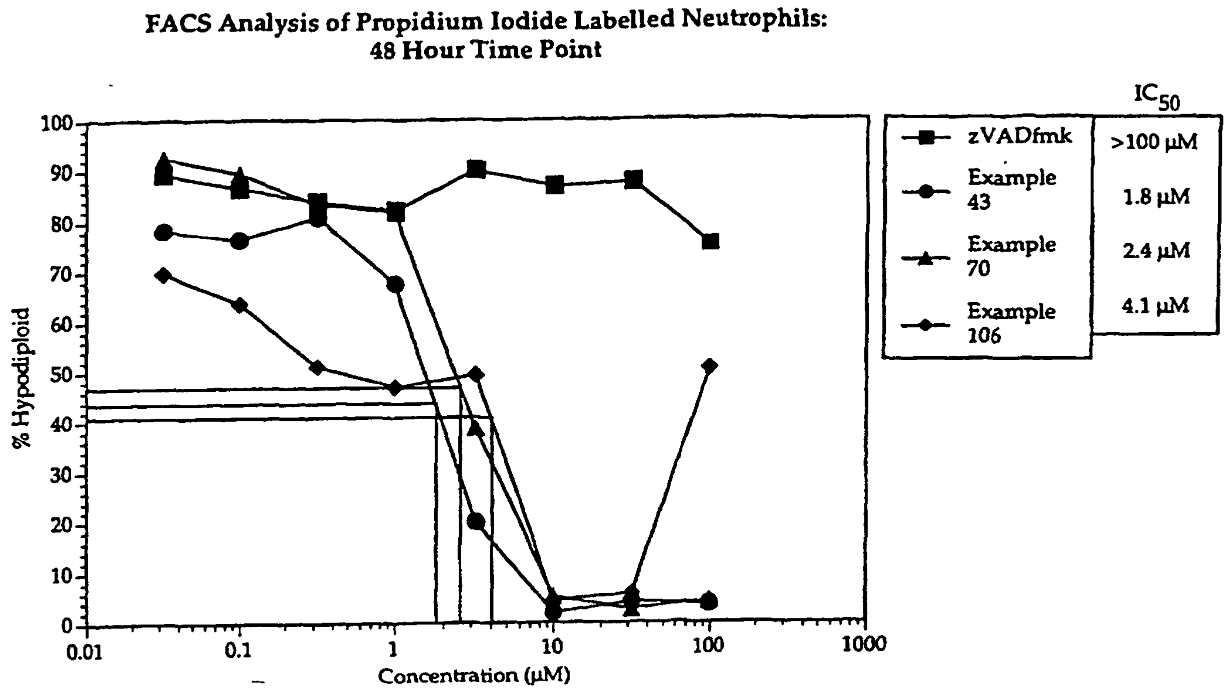
FACS Analysis of Propidium Iodide Labelled Neutrophils:
48 Hour Time Point
% Hypodiploid
100
90
80
70
60
50
40
30
20
10
0
0.01
0.1
1
10
100
1000
Concentration (μM)
IC50
zVADfmk
>100 μM
Example 43
1.8 μM
Example 70
2.4 μM
Example 106
4.1 μM

Figure 6